# EUROPEAN PATENT APPLICATION

(11) **EP 4 552 498 A1**
(43) Date of publication of application: **14.05.2025**
(21) Application number: 23835536.6
(22) Date of filing: 04.07.2023
(51) Int. Cl.: A01N 37/44, A01M 1/20, A01N 37/46, A01N 41/02, A01N 41/04, A01N 41/06, A01N 41/12, A01N 43/36, A01N 43/50, A01N 43/90, A01N 47/12, A01N 47/44, A01N 57/12, A01N 57/14, A01P 7/04, C07K 5/06, C12N 15/09

(54) **METHOD FOR PREVENTING INSECT DAMAGE TO PLANTS**

(30) Priority: 06.07.2022 JP 2022109057
(71) Applicant: AJINOMOTO CO., INC., Chuo-ku Tokyo 104-8315 (JP); National University Corporation Saitama University, Saitama City, Saitama 338-8570 (JP)
(72) Inventor: TOYOTA, Masatsugu, Saitama-shi, Saitama 338-8570 (JP); SUDA, Hiraku, Saitama-shi, Saitama 338-8570 (JP); KAKUDA, Kyohei, Saitama-shi, Saitama 338-8570 (JP); TAKEDA, Taito, Kawasaki-shi, Kanagawa 210-8681 (JP); SATO, Seiichi, Kawasaki-shi, Kanagawa 210-8681 (JP); TAHARA, Yuki, Kawasaki-shi, Kanagawa 210-8681 (JP); KUWAHARA, Kayano, Kawasaki-shi, Kanagawa 210-8681 (JP)
(74) Representative: Strehl & Partner mbB
(86) International application number: PCT/JP2023/024818
(87) International publication number: WO 2024/010005

(57) **Abstract**

A technique useful in the field of agri-horticulture such as a technique for preventing pest damage to plants is provided. Pest damage to plants is prevented by using various glutamic acid derivatives.

## Description

### TECHNICAL FIELD

The present invention relates to techniques useful in the field of agri-horticulture (agriculture and horticulture), such as a technique for preventing pest damage to plants.

### BACKGROUND ART

According to a report by the United Nations, the world population is projected to increase by 2 billion people over the next 30 years, reaching 9.7 billion by 2050. The Food and Agriculture Organization of the United Nations states that this population growth will require agricultural production (e.g., food, feed, and biofuel production amounts) in 2050 to be at least 50% higher than in 2012 (Non-patent document 1).

On the other hand, pest damage and other damages have been reported in plants; for example, 26 to 40% of grain is lost due to pests, pathogens, weeds, viruses, etc.

Pest damage prevention techniques largely rely on pest control utilizing pesticidal synthetic agricultural chemicals, and the emergence of drug-resistant pests has been a recurring problem. Therefore, there is a need to develop pest control technique that does not allow emergence of drug-resistant pests.

It has been shown that when plants are fed by pests, glutamic acid leaked from injured cells activates the glutamate receptor GLR3.3/3.6, a Ca²⁺ permeable ion channel, the generated Ca²⁺ signals rapidly propagate through vascular bundles, so that jasmonic acid, a resistance plant hormone, is synthesized within several minutes even in distant organs not directly fed, and thus systemic defense mechanisms are activated (Non-patent document 2).

### PRIOR ART DOCUMENTS

### Non-patent documents

Non-patent document 1: FAO, The future of food and agriculture - Trends and challenges, 2017.
Non-patent document 2: Toyota, M et al. Glutamate triggers long-distance, calcium-based plant defense signaling. Science. 2018 Sep 14;361(6407):1112-1115.

### SUMMARY OF THE INVENTION

### PROBLEMS TO BE ACHIEVED BY THE INVENTION

An object of the present invention is to provide a technique useful in the field of agri-horticulture, such as a technique for preventing pest damage to plants.

### MEANS FOR SOLVING THE PROBLEMS

The inventors of the present invention conducted various researches in order to achieve the aforementioned object. As a result, they found that various glutamic acid derivatives can induce resistance to pest damage in plants, and thereby pest damage to plants can be prevented, and thus accomplished the present invention.

Accordingly, the present invention can be illustrated as follows.
[1] A composition for inducing pest damage resistance in a plant or preventing pest damage to a plant, comprising the following ingredient (A):
   (A) a compound represented by the general formula (I) described below,
      wherein:
      n represents an integer of from 1 to 3 (i.e., 1, 2, or 3),
      X represents an interatomic bond, oxygen atom (O), nitrogen atom bonded with hydrogen atom (NH), nitrogen atom bonded with methyl group (NMe), sulfur atom (S), or 4-phenoxy group,
      R¹ represents hydrogen atom (H), amidino group, or an acyl group,
      R² represents OR⁴ or NHR⁴,
      R³ represents C(=O)-R⁵, sulfo group (SO₃H), phosphate group (PO₃H₂), or SO₂R⁶,
      R⁴ represents hydrogen atom (H), or an optionally substituted alkyl group having 1 to 8 carbon atoms,
      R⁵ represents an optionally substituted alkyl group having 1 to 8 carbon atoms, an amino acid, a dipeptide, OR⁷, or NR⁸R⁹,
      R⁶ represents methyl group, or an optionally substituted phenyl group,
      R⁷ represents hydrogen atom (H), or an alkyl group having 1 to 8 carbon atoms, and
      R⁸ and R⁹ independently represent hydrogen atom (H), an optionally substituted alkyl group having 1 to 8 carbon atoms, or an optionally substituted phenyl group.
[2] The composition mentioned above, wherein n is 2.
[3] The composition mentioned above, wherein X is an interatomic bond.
[4] The composition mentioned above, wherein the composition satisfies any of the following conditions:
   (1) n is 2, X is an interatomic bond, R² is hydroxyl group, and R³ is C(=O)-R⁵;
   (2) n is 2, X is an interatomic bond, R¹ is hydrogen atom, R² is hydroxyl group, R³ is C(=O)-R⁵, and R⁵ is other than hydroxyl group;
   (3) n is 2, X is an interatomic bond, R¹ is other than hydrogen atom, R² is hydroxyl group, R³ is C(=O)-R⁵, and R⁵ is hydroxyl group; and
   (4) n is 2, X is an interatomic bond, R¹ is hydrogen atom, R² is other than hydroxyl group, and R³ is C(=O)-OH.
[5] The composition mentioned above, wherein the ingredient (A) is selected from the compounds represented by the formulas of Compound 1-58 and Compound 67-75 described below.
[6] The composition mentioned above, wherein the pest damage is pest damage caused by an organism classified in the order *Lepidoptera, Hemiptera, Coleoptera, Diptera, Orthoptera, Thysanoptera, Tylenchida, Collembola, Acarina,* or *Stylommatophora.*
[7] The composition mentioned above, wherein the pest damage is pest damage caused by an organism classified in the family *Plutellidae, Noctuidae, Pyralidae, Tortricidae, Lyonetiidae, Carposinidae, Gelechiidae, Crambidae, Arctiidae (Erebidae), Lymantriidae (Erebidae), Cicadellidae, Delphacidae, Psyllidae, Aphididae, Aleyrodidae, Coccoidea, Miridae, Tingidae, Pentatomidae, Lygaeidae, Scarabaeidae, Elateridae, Coccinellidae, Cerambycidae, Chrysomelidae, Curculionidae, Muscidae, Calliphoridae, Sarcophagidae, Anthomyiidae, Tephritidae, Opomyzoidea* (superfamily), *Chloropoidea* (superfamily), *Acrididae, Catantopidae, Pyrgomorphidae, Thripidae, Aeolothripidae, Merothripidae, Aphelenchoididae, Neotylenchidae, Onychiuridae, Isotomidae, Tetranychidae, Dermanyssidae, Acaridae, Sarcoptidae, Philomycidae,* or *Bradybaenidae.*
[8] The composition mentioned above, wherein the plant is a *Poaceae* plant, *Solanaceae* plant, *Cucurbitaceae* plant, *Fabaceae* plant, *Brassicaceae* plant, *Rosaceae* plant, *Moraceae* plant, *Malvaceae* plant, *Apiaceae* plant, *Liliaceae* plant, *Asteraceae* plant, *Amaranthaceae* plant, *Ericaceae* plant, *Vitaceae* plant, *Rutaceae* plant, *Rubiaceae* plant, *Oleaceae* plant, *Lauraceae* plant, *Anacardiaceae* plant, *Sapindaceae* plant, *or Lamiaceae* plant.
[9] A method for inducing pest damage resistance in a plant, comprising applying the following ingredient (A) to the plant:
   (A) a compound represented by the general formula (I) described below, wherein:
      n represents an integer of from 1 to 3 (i.e., 1, 2, or 3),
      X represents an interatomic bond, oxygen atom (O), nitrogen atom bonded with hydrogen atom (NH), nitrogen atom bonded with methyl group (NMe), sulfur atom (S), or 4-phenoxy group,
      R¹ represents hydrogen atom (H), amidino group, or an acyl group,
      R² represents OR⁴ or NHR⁴,
      R³ represents C(=O)-R⁵, sulfo group (SO₃H), phosphate group (PO₃H₂), or SO₂R⁶,
      R⁴ represents hydrogen atom (H), or an optionally substituted alkyl group having 1 to 8 carbon atoms,
      R⁵ represents an optionally substituted alkyl group having 1 to 8 carbon atoms, an amino acid, a dipeptide, OR⁷, or NR⁸R⁹,
      R⁶ represents methyl group, or an optionally substituted phenyl group,
      R⁷ represents hydrogen atom (H), or an alkyl group having 1 to 8 carbon atoms, and
      R⁸ and R⁹ independently represent hydrogen atom (H), an optionally substituted alkyl group having 1 to 8 carbon atoms, or an optionally substituted phenyl group.
[10] A method for preventing pest damage to a plant,
   comprising applying the following ingredient (A) to the plant:
   (A) a compound represented by the general formula (I) described below, wherein:
      n represents an integer of from 1 to 3 (i.e., 1, 2, or 3),
      X represents an interatomic bond, oxygen atom (O), nitrogen atom bonded with hydrogen atom (NH), nitrogen atom bonded with methyl group (NMe), sulfur atom (S), or 4-phenoxy group,
      R¹ represents hydrogen atom (H), amidino group, or an acyl group,
      R² represents OR⁴ or NHR⁴,
      R³ represents C(=O)-R⁵, sulfo group (SO₃H), phosphate group (PO₃H₂), or SO₂R⁶,
      R⁴ represents hydrogen atom (H), or an optionally substituted alkyl group having 1 to 8 carbon atoms,
      R⁵ represents an optionally substituted alkyl group having 1 to 8 carbon atoms, an amino acid, a dipeptide, OR⁷, or NR⁸R⁹,
      R⁶ represents methyl group, or an optionally substituted phenyl group,
      R⁷ represents hydrogen atom (H), or an alkyl group having 1 to 8 carbon atoms, and
      R⁸ and R⁹ independently represent hydrogen atom (H), an optionally substituted alkyl group having 1 to 8 carbon atoms, or an optionally substituted phenyl group.
[11] A method for producing a plant body,
   comprising cultivating a plant with applying the following ingredient (A) to the plant:
   (A) a compound represented by the general formula (I) described below, wherein:
      n represents an integer of from 1 to 3 (i.e., 1, 2, or 3),
      X represents an interatomic bond, oxygen atom (O), nitrogen atom bonded with hydrogen atom (NH), nitrogen atom bonded with methyl group (NMe), sulfur atom (S), or 4-phenoxy group,
      R¹ represents hydrogen atom (H), amidino group, or an acyl group,
      R² represents OR⁴ or NHR⁴,
      R³ represents C(=O)-R⁵, sulfo group (SO₃H), phosphate group (PO₃H₂), or SO₂R⁶,
      R⁴ represents hydrogen atom (H), or an optionally substituted alkyl group having 1 to 8 carbon atoms,
      R⁵ represents an optionally substituted alkyl group having 1 to 8 carbon atoms, an amino acid, a dipeptide, OR⁷, or NR⁸R⁹,
      R⁶ represents methyl group, or an optionally substituted phenyl group,
      R⁷ represents hydrogen atom (H), or an alkyl group having 1 to 8 carbon atoms, and
      R⁸ and R⁹ independently represent hydrogen atom (H), an optionally substituted alkyl group having 1 to 8 carbon atoms, or an optionally substituted phenyl group.
[12] The method mentioned above, wherein n is 2.
[13] The method mentioned above, wherein X is an interatomic bond.
[14] The method mentioned above, wherein the method satisfies any of the following conditions:
   (1) n is 2, X is an interatomic bond, R² is hydroxyl group, and R³ is C(=O)-R⁵ ;
   (2) n is 2, X is an interatomic bond, R¹ is hydrogen atom, R² is hydroxyl group, R³ is C(=O)-R⁵, and R⁵ is other than hydroxyl group;
   (3) n is 2, X is an interatomic bond, R¹ is other than hydrogen atom, R² is hydroxyl group, R³ is C(=O)-R⁵, and R⁵ is hydroxyl group; and
   (4) n is 2, X is an interatomic bond, R¹ is hydrogen atom, R² is other than hydroxyl group, and R³ is C(=O)-OH.
[15] The method mentioned above, wherein the ingredient (A) is selected from the compounds represented by the formulas of Compound 1-58 and Compound 67-75 described below.
[16] The method mentioned above, wherein the ingredient (A) is used in the form of a liquid containing the ingredient (A) at a concentration of 0.1 to 1,000 mM.
[17] The method mentioned above, wherein the pest damage is pest damage caused by an organism classified in the order *Lepidoptera, Hemiptera, Coleoptera, Diptera, Orthoptera, Thysanoptera, Tylenchida, Collembola, Acarina,* or *Stylommatophora.*
[18] The method mentioned above, wherein the pest damage is pest damage caused by an organism classified in the family *Plutellidae, Noctuidae, Pyralidae, Tortricidae, Lyonetiidae, Carposinidae, Gelechiidae, Crambidae, Arctiidae (Erebidae), Lymantriidae (Erebidae), Cicadellidae, Delphacidae, Psyllidae, Aphididae, Aleyrodidae, Coccoidea, Miridae, Tingidae, Pentatomoidea, Lygaeidae, Scarabaeidae, Elateridae, Coccinellidae, Cerambycidae, Chrysomelidae, Curculionidae, Muscidae, Calliphoridae, Sarcophagidae, Anthomyiidae, Tephritidae, Opomyzoidea* (superfamily), *Chloropoidea* (superfamily), *Acrididae, Catantopidae, Pyrgomorphidae, Thripidae, Aeolothripidae, Merothripidae, Aphelenchoididae, Neotylenchidae, Onychiuridae, Isotomidae, Tetranychidae, Dermanyssidae, Acaridae, Sarcoptidae, Philomycidae,* or *Bradybaenidae.*
[19] The method mentioned above, wherein the plant is a *Poaceae* plant, *Solanaceae* plant, *Cucurbitaceae* plant, *Fabaceae* plant, *Brassicaceae* plant, *Rosaceae* plant, *Moraceae* plant, *Malvaceae* plant, *Apiaceae* plant, *Liliaceae* plant, *Asteraceae* plant, *Amaranthaceae* plant, *Ericaceae* plant, *Vitaceae* plant, *Rutaceae* plant, *Rubiaceae* plant, *Oleaceae* plant, *Lauraceae* plant, *Anacardiaceae* plant, *Sapindaceae* plant, *or Lamiaceae* plant.

### BRIEF DESCRIPTION OF THE DRAWINGS

Figure 1 shows a graph showing pest damage resistance gene expression-inducing effect of glutamic acid derivatives in *Arabidopsis thaliana.* "Sor" stands for sorbitol, "Glu" for glutamic acid, "#1" for Compound 1, and "#34" for Compound 34. "n.s." means not significant. Time (0 minute or 30 minutes) means the time from compound treatment to sample collection. Statistical analysis was performed by the Steel's test.
Figure 2 shows a graph showing feeding damage-suppressing effect of glutamic acid derivatives in *Arabidopsis thaliana.* The vertical axis indicates weight of damaged plants. "#1" stands for Compound 1. "n.s." means not significant. Statistical analysis was performed by the Steel's test.
Figure 3 shows a graph showing feeding damage-suppressing effect of a glutamic acid derivative in *Arabidopsis thaliana.* The vertical axis indicates weight gain rate of the pests. "#1" stands for Compound 1. "n.s." means not significant. Statistical analysis was performed by the Steel's test.
Figure 4 shows a graph showing feeding damage-suppressing effect of a glutamic acid derivative in *Arabidopsis thaliana.* The vertical axis indicates weight gain rate of the pests. "#1" stands for Compound 1. "n.s." means not significant. Statistical analysis was performed by the Steel's test.
Figure 5 shows a graph showing feeding damage-suppressing effect of a glutamic acid derivative in komatsuna (*Brassica rapa* var. *perviridis*)*.* The vertical axis indicates ratio of the area damaged by pests (feeding damage area).

### MODE FOR CARRYING OUT THE INVENTION

Hereafter, the present invention will be explained in detail.

### 1. Active ingredient

In the present invention, the following ingredient (A) is utilized:
(A) a compound represented by the following general formula (I).

The ingredient (A) is also referred to as the "active ingredient".

The ingredient (A) is a compound represented by the following general formula (I). For the ingredient (A), "Rⁿ" and "Rn" (n is a positive integer) may be used interchangeably.

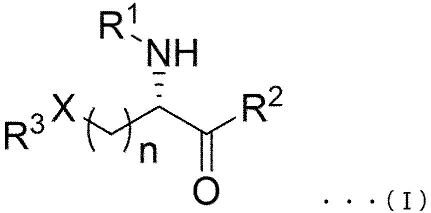

In the formula (I), n represents an integer of from 1 to 3 (i.e., 1, 2, or 3).

In the formula (I), X represents an interatomic bond, oxygen atom (O), nitrogen atom bonded with hydrogen atom (NH), nitrogen atom bonded with methyl group (NMe), sulfur atom (S), or 4-phenoxy group. The term "4-phenoxy group" as X means phenoxy group in which the carbon at position 1 binds to (-CH₂-)ₙ and O binding to the carbon at position 4 binds to R³.

In the formula (I), R¹ represents hydrogen atom (H), amidino group, or an acyl group.

In the formula (I), R² represents OR⁴ or NHR⁴.

In the formula (I), R³ represents C(=O)-R⁵, sulfo group (SO₃H), phosphate group (PO₃H₂), or SO₂R⁶.

R⁴ represents hydrogen atom (H), or an optionally substituted alkyl group having 1 to 8 carbon atoms.

R⁵ represents an optionally substituted alkyl group having 1 to 8 carbon atoms, an amino acid, a dipeptide, OR⁷, or NR⁸R⁹.

R⁶ represents methyl group, or an optionally substituted phenyl group.

R⁷ represents hydrogen atom (H), or an alkyl group having 1 to 8 carbon atoms.

R⁸ and R⁹ independently represent hydrogen atom (H), an optionally substituted alkyl group having 1 to 8 carbon atoms, or an optionally substituted phenyl group.

In one embodiment, n may be 2.

In one embodiment, X may be an interatomic bond.

In one embodiment, n may be 2, X may be an interatomic bond, R² may be hydroxyl group, and R³ may be C(=O)-R⁵.

In one embodiment, n may be 2, X may be an interatomic bond, R¹ may be hydrogen atom, R² may be hydroxyl group, R³ may be C(=O)-R⁵, and R⁵ may be other than hydroxyl group.

In one embodiment, n may be 2, X may be an interatomic bond, R¹ may be other than hydrogen atom, R² may be hydroxyl group, R³ may be C(=O)-R⁵, and R⁵ may be hydroxyl group.

In one embodiment, n may be 2, X may be an interatomic bond, R¹ may be hydrogen atom, R² may be other than hydroxyl group, and R³ may be C(=O)-OH.

Unless especially noted, X, n, and R¹ to R⁹ can be independently selected for the ingredient (A).

The term "optionally substituted functional group" is a generic term for substituted and unsubstituted functional groups. That is, for example, "optionally substituted alkyl group" is a generic term for substituted and unsubstituted alkyl groups. A functional group that is substituted is also referred to as a "substituted functional group" and a functional group that is not substituted as an "unsubstituted functional group". That is, for example, an alkyl group that is substituted is also referred to as a "substituted alkyl group" and an alkyl group that is not substituted as an "unsubstituted alkyl group". The term "functional group" without reference to substitution means an unsubstituted functional group, unless especially noted. That is, for example, "alkyl group" without reference to substitution means an unsubstituted alkyl group, unless especially noted. The "number of carbon atoms" of an optionally substituted functional group means the number of carbon atoms of the functional group in its unsubstituted state (i.e., the number of carbon atoms not including those of substituent), regardless of whether it is substituted or not, unless especially noted. Descriptions for unsubstituted functional groups can also be applied to the part other than substituents of substituted functional groups. That is, for example, description for an unsubstituted alkyl group can be applied to the part other than substituent of a substituted alkyl group (i.e., the alkyl group moiety).

The expression that "a functional group is substituted" means that one or more of the hydrogen atoms constituting the functional group are replaced with one or more substituents. The expression that "a functional group is substituted" is also expressed as "the functional group has a substituent". The number of hydrogen atoms replaced with substituents may be, for example, 1 to 5, 1 to 4, 1 to 3, or 1 or 2. The number of hydrogen atoms replaced with substituents may specifically be, for example, 1, 2, 3, 4, or 5. The number of hydrogen atoms replaced with substituents may be read as the number of substituents that the functional group has. When two or more hydrogen atoms are replaced with substituents, the substituents are independently selected for each hydrogen atom. Examples of hydrogen atom replaced with a substituent include hydrogen atom bonding to carbon atom, hydrogen atom bonding to nitrogen atom, and hydrogen atom bonding to oxygen atom. Hydrogen atom bonding to carbon atom may or may not be, for example, hydrogen atom bonding to terminal carbon atom. The term "terminal carbon atom" means carbon atom at the terminus of a carbon chain. If the carbon chain is branched, the terminus may be terminus of any branch.

Examples of the acyl group include an optionally substituted alkylcarbonyl group, an optionally substituted alkenylcarbonyl group, an optionally substituted arylcarbonyl group, and an optionally substituted alkoxycarbonyl group.

Examples of substituent of the functional group constituting the acyl group as R¹ (e.g., a substituted alkyl group, a substituted alkenyl group, a substituted aryl group, or a substituted alkoxy group) include a halogen atom, hydroxyl group, amino group, carboxyl group, aminocarbonyl group, mercapto group, an optionally substituted aryl group, an optionally substituted heteroaryl group, an optionally substituted alkoxy group, an optionally substituted alkylamino group, an optionally substituted alkoxycarbonylamino group, an optionally substituted alkoxycarbonyl group, and an optionally substituted alkylthio group.

Examples of substituent of the substituted alkyl group as R⁴, R⁵, R⁸, or R⁹ include a halogen atom, hydroxyl group, amino group, guanidino group, carboxyl group, aminocarbonyl group, mercapto group, an optionally substituted aryl group, an optionally substituted heteroaryl group, an optionally substituted alkoxy group, an optionally substituted alkylamino group, an optionally substituted alkoxycarbonylamino group, an optionally substituted alkoxycarbonyl group, and an optionally substituted alkylthio group.

Examples of substituent of the substituted phenyl group as R⁶, R⁸, or R⁹ include a halogen atom, hydroxyl group, amino group, carboxyl group, aminocarbonyl group, mercapto group, nitro group, an optionally substituted aryl group, an optionally substituted heteroaryl group, an optionally substituted alkoxy group, an optionally substituted alkylamino group, an optionally substituted alkoxycarbonylamino group, an optionally substituted alkoxycarbonyl group, and an optionally substituted alkylthio group.

Examples of substituent of a substituted aryl group in the functional group constituting the acyl group as R¹ include a halogen atom, hydroxyl group, amino group, carboxyl group, and an alkyl group.

Examples of substituent of a substituted aryl group in the substituted alkyl group as R⁴, R⁵, R⁸, or R⁹ include a halogen atom, hydroxyl group, amino group, carboxyl group, and an alkyl group.

Examples of substituent of a substituted aryl group in the substituted phenyl group as R⁶, R⁸, or R⁹ include a halogen atom, hydroxyl group, amino group, carboxyl group, and an alkyl group.

Examples of substituent of a substituted heteroaryl group in a functional group constituting the acyl group as R¹ include a halogen atom, hydroxyl group, amino group, carboxyl group, and an alkyl group.

Examples of substituent of a substituted heteroaryl group in the substituted alkyl group as R⁴, R⁵, R⁸, or R⁹ include a halogen atom, hydroxyl group, amino group, carboxyl group, and an alkyl group.

Examples of substituent of a substituted heteroaryl group in the substituted phenyl group as R⁶, R⁸, or R⁹ include a halogen atom, hydroxyl group, amino group, carboxyl group, and an alkyl group.

Examples of substituent of a substituted alkoxy group in a functional group constituting the acyl group as R¹ include a halogen atom, hydroxyl group, amino group, carboxyl group, aminocarbonyl group, an aryl group, and a heteroaryl group.

Examples of substituent of a substituted alkoxy group in the substituted alkyl group as R⁴, R⁵, R⁸, or R⁹ include a halogen atom, hydroxyl group, amino group, carboxyl group, aminocarbonyl group, an aryl group, and a heteroaryl group.

Examples of substituent of a substituted alkoxy group in the substituted phenyl group as R⁶, R⁸, or R⁹ include a halogen atom, hydroxyl group, amino group, carboxyl group, aminocarbonyl group, an aryl group, and a heteroaryl group.

Examples of substituent of a substituted alkoxycarbonyl group in a functional group constituting the acyl group as R¹ include a halogen atom, hydroxyl group, amino group, carboxyl group, aminocarbonyl group, an aryl group, and a heteroaryl group.

Examples of substituent of a substituted alkoxycarbonyl group in the substituted alkyl group as R⁴, R⁵, R⁸, or R⁹ include a halogen atom, hydroxyl group, amino group, carboxyl group, aminocarbonyl group, an aryl group, and a heteroaryl group.

Examples of substituent of a substituted alkoxycarbonyl group in the substituted phenyl group as R⁶, R⁸, or R⁹ include a halogen atom, hydroxyl group, amino group, carboxyl group, aminocarbonyl group, an aryl group, and a heteroaryl group.

Examples of substituent of a substituted alkylamino group in a functional group constituting the acyl group as R¹ include a halogen atom, hydroxyl group, amino group, carboxyl group, aminocarbonyl group, an N-alkyl group, an aryl group and a heteroaryl group.

Examples of substituent of a substituted alkylamino group in the substituted alkyl group as R⁴, R⁵, R⁸, or R⁹ include a halogen atom, hydroxyl group, amino group, carboxyl group, aminocarbonyl group, an N-alkyl group, an aryl group, and a heteroaryl group.

Examples of substituent of a substituted alkylamino group in the substituted phenyl group as R⁶, R⁸, or R⁹ include a halogen atom, hydroxyl group, amino group, carboxyl group, aminocarbonyl group, an N-alkyl group, an aryl group, and a heteroaryl group.

Examples of substituent of a substituted alkoxycarbonylamino group in a functional group constituting the acyl group as R¹ include a halogen atom, hydroxyl group, amino group, carboxyl group, aminocarbonyl group, an N-alkyl group, an aryl group, and a heteroaryl group.

Examples of substituent of a substituted alkoxycarbonylamino group in the substituted alkyl group as R⁴, R⁵, R⁸, or R⁹ include a halogen atom, hydroxyl group, amino group, carboxyl group, aminocarbonyl group, an N-alkyl group, an aryl group, and a heteroaryl group.

Examples of substituent of a substituted alkoxycarbonylamino group in the substituted phenyl group as R⁶, R⁸, or R⁹ include a halogen atom, hydroxyl group, amino group, carboxyl group, aminocarbonyl group, an N-alkyl group, an aryl group, and a heteroaryl group.

The term "N-alkyl group" means an alkyl group that substitutes for hydrogen atom of amino group.

Examples of the halogen atom include fluorine atom, chlorine atom, bromine atom, and iodine atom.

Examples of the alkyl group include an alkyl group having 1 to 12 carbon atoms. Examples of the alkyl group include, in particular, an alkyl group having 1 to 8 carbon atoms. Examples of the alkyl group include, in more particular, an alkyl group having 1 to 6 carbon atoms. Examples of the alkyl group include, in more particular, an alkyl group having 1 to 4 carbon atoms. The alkyl group may be linear, branched, or cyclic. Examples of the alkyl group having 1 to 8 carbon atoms include methyl group, ethyl group, propyl group, isopropyl group, butyl group, isobutyl group, sec-butyl group, tert-butyl group, pentyl group, isopentyl group, 2,3-dimethylpropyl group, hexyl group, heptyl group, octyl group, cyclopropyl group, cyclobutyl group, cyclopentyl group, cyclohexyl group, cycloheptyl group, and cyclooctyl group. Examples of the alkyl group having 1 to 6 carbon atoms include methyl group, ethyl group, propyl group, isopropyl group, butyl group, isobutyl group, sec-butyl group, tert-butyl group, pentyl group, isopentyl group, 2,3-dimethylpropyl group, hexyl group, cyclopropyl group, cyclobutyl group, cyclopentyl group, and cyclohexyl group. Examples of the alkyl group having 1 to 4 carbon atoms include methyl group, ethyl group, propyl group, isopropyl group, butyl group, isobutyl group, sec-butyl group, tert-butyl group, cyclopropyl group, and cyclobutyl groups. The descriptions for the alkyl group can be applied to the N-alkyl group.

Examples of the alkenyl group include an alkenyl group having 1 to 6 carbon atoms. The alkenyl group may be linear or branched. Specific examples of the alkenyl group include vinyl group, allyl group, propenyl group, butenyl group, butadienyl group, pentenyl group, pentadienyl group, hexenyl group, and hexadienyl group.

Examples of the aryl group include an aryl group having 6 to 14 carbon atoms. Examples of the aryl group include, in particular, an aryl group having 6 to 10 carbon atoms. Specific examples of the aryl group include phenyl group, naphthyl group, and fluorenyl group.

The term "heteroaryl group" means an aryl group having at least one kind of heteroatom selected from the group consisting of nitrogen atom, oxygen atom, and sulfur atom as an atom constituting the ring. Examples of the heteroaryl group include a heteroaryl group having 3 to 14 carbon atoms. Examples of the heteroaryl group include, in particular, a heteroaryl group having 4 to 10 carbon atoms. Examples of the heteroaryl group include, in more particular, a heteroaryl group having 4 to 9 carbon atoms. Specific examples of the heteroaryl group include furanyl group, pyrrolyl group, oxazolyl group, imidazolyl group, pyrazolyl group, pyranyl group, indenyl group, thiophenyl group, pyridinyl group, indolyl group and quinolinyl group. Examples of the heteroaryl group include, in particular, imidazolyl group and indolyl group.

Examples of the alkoxy group include an alkoxy group having 1 to 6 carbon atoms. Examples of the alkoxy group include, in particular, an alkoxy group having 1 to 4 carbon atoms. The alkoxy group may be linear or branched. Specific examples of the alkoxy group include methoxy group, ethoxy group, 1-propoxy group, 2-propoxy group, n-butoxy group, i-butoxy group, sec-butoxy group, tert-butoxy group, 1-pentyloxy group, 2-pentyloxy group, 3-pentyloxy group, 2-methyl-1-butyloxy group, 3-methyl-1-butyloxy group, 2-methyl-2-butyloxy group, 3-methyl-2-butyloxy group, 2,2-dimethyl-1-propyloxy group, 1-hexyloxy group, 2-hexyloxy group, and 3-hexyloxy group. Examples of the alkoxy group include, in particular, tert-butoxy group.

The optionally substituted alkyl group is an unsubstituted alkyl group or a substituted alkyl group. The alkyl group is as described above. To the alkyl group constituting the substituted alkyl group, the above descriptions for alkyl group can be applied. The substituent constituting the substituted alkyl group is as described above. For example, examples of the optionally substituted alkyl group having 1 to 4 carbon atoms include, in particular, methyl group, ethyl group, isopropyl group, isobutyl group, tert-butyl group, cyclopropyl group, cyclobutyl group, benzyl group, hydroxyethyl group, and hydroxypropyl group (2-hydroxypropyl group etc.).

The optionally substituted phenyl group is unsubstituted phenyl group or a substituted phenyl group. The substituent constituting the substituted phenyl group is as described above. Specific examples of the optionally substituted phenyl group include phenyl group, a hydroxyphenyl group (2-, 3-, or 4-hydroxyphenyl group, etc.), a chlorophenyl group (2-, 3-, or 4-chlorophenyl group, etc.), a dichlorophenyl group (2,5-dichlorophenyl group, etc.), a fluorophenyl group (2-, 3-, or 4-fluorophenyl group, etc.), and a nitrophenyl group (2-, 3-, or 4-nitrophenyl group, etc.).

The optionally substituted aryl group is an aryl unsubstituted group or a substituted aryl group. The aryl group is as described above. To the aryl group constituting the substituted aryl group, the above descriptions for the aryl group can be applied. The substituent constituting the substituted aryl group is as described above.

Specific examples of the substituted aryl group include an optionally substituted alkylaryl group (i.e., an unsubstituted alkylaryl group and a substituted alkylaryl group). The above descriptions for the alkyl group, aryl group, and substituent thereof can be applied to the alkyl group, aryl group, and substituent thereof constituting the unsubstituted alkylaryl group or substituted alkylaryl group, respectively. Specific examples of the optionally substituted alkylaryl group include a methylphenyl group (such as 2-, 3-, or 4-methylphenyl group).

Specific examples of the substituted aryl group also include a hydroxyphenyl group (2-, 3-, or 4-hydroxyphenyl group, etc.), a chlorophenyl group (2-, 3-, or 4-chlorophenyl group, etc.), a dichlorophenyl group (2,5-dichlorophenyl group, etc.), a fluorophenyl group (2-, 3-, or 4-fluorophenyl group, etc.), and 2,3-dihydroxyindenyl group.

The optionally substituted heteroaryl group is an unsubstituted heteroaryl group or a substituted heteroaryl group. The heteroaryl group is as described above. To the heteroaryl group constituting the substituted heteroaryl group, the above description for the heteroaryl group can be applied. The substituent constituting the substituted heteroaryl group is as described above.

The optionally substituted alkoxy group is an unsubstituted alkoxy group or a substituted alkoxy group. The alkoxy group is as described above. To the alkoxy group constituting the substituted alkoxy group, the above description for the alkoxy group can be applied. The substituent constituting the substituted alkoxy group is as described above.

Specific examples of the substituted alkoxy group include an optionally substituted arylalkoxy group (i.e., an unsubstituted arylalkoxy group and substituted arylalkoxy group). To the aryl group, alkoxy group, and substituent constituting the unsubstituted arylalkoxy group or substituted arylalkoxy group, the above descriptions for the aryl group, alkoxy group, and substituent thereof can be applied, respectively. Specific examples of the optionally substituted arylalkoxy group include phenylmethoxy group.

Specific examples of the optionally substituted alkoxy group also include a hydroxyalkoxy group (such as hydroxyethoxy group) and trifluoromethoxy group.

The optionally substituted alkylamino group is an unsubstituted alkylamino group or a substituted alkylamino group. To the alkyl group constituting the alkylamino group, the above description for the alkyl group can be applied. To the alkyl group and substituent constituting the substituted alkylamino group, the above descriptions for the alkyl group and substituent thereof can be applied. For example, examples of the number of carbon atoms of the alkyl group (e.g., 1 to 12, 1 to 6, or the like) described above may also be examples of the number of carbon atoms of the optionally substituted alkylamino group (not including the number of carbon atoms of the substituent). Specific examples of the substituted alkylamino group include an optionally substituted carboxyalkylamino group (i.e., an unsubstituted carboxyalkylamino group and a substituted carboxyalkylamino group). The number of carbon atoms of the optionally substituted carboxyalkylamino group (not including the number of carbon atoms of substituent) may be, for example, 2 to 13 or 2 to 7. Specific examples of the carboxyalkylamino group include carboxymethylamino group and 1-methylcarboxymethylamino group. Specific examples of the substituted carboxyalkylamino group include a carboxyalkylamino group substituted with hydroxyl group, amino group, carboxyl group, phenyl groups, indolyl group, or N-methyl group. More specific examples of the substituted carboxyalkylamino group include 1-(aminobutyl)carboxymethylamino group, 1-methylcarboxymethylamino group, 1-(indolylmethyl)carboxymethylamino group, 1-(carboxymethyl)carboxymethylamino group, 1-(1-hydroxyethyl)carboxymethylamino group, 1-(hydroxymethyl)carboxymethylamino group, 1-isobutylcarboxymethylamino group, 1-benzylcarboxymethylamino group, and N-methylcarboxymethylamino group.

The optionally substituted alkylcarbonyl group is an unsubstituted alkylcarbonyl group or a substituted alkylcarbonyl group. To the alkyl group constituting the alkylcarbonyl group, the above description for the alkyl group can be applied. To the alkyl group and substituent constituting the substituted alkylcarbonyl group, the above descriptions for the alkyl group and substituent thereof can be applied.

The optionally substituted alkenylcarbonyl group is an unsubstituted alkenylcarbonyl group or a substituted alkenylcarbonyl group. To the alkenyl group constituting the alkenylcarbonyl group, the above description for the alkenyl group can be applied. To the alkenyl group and substituent constituting the substituted alkenylcarbonyl group, the above descriptions for the alkenyl group and substituent thereof can be applied.

The optionally substituted arylcarbonyl group is an unsubstituted arylcarbonyl group or a substituted arylcarbonyl group. To the aryl group constituting the arylcarbonyl group, the above description for the aryl group can be applied. To the aryl group and substituent constituting the substituted arylcarbonyl group, the above descriptions for the aryl group and substituent thereof can be applied.

The optionally substituted alkoxycarbonyl group is an unsubstituted alkoxycarbonyl group or a substituted alkoxycarbonyl group. To the alkoxy group constituting the alkoxycarbonyl group, the above description for the alkoxy group can be applied. To the alkoxy group and substituent constituting the substituted alkoxycarbonyl group, the above descriptions for the alkoxy group and substituent thereof can be applied. Specific examples of the optionally substituted alkoxycarbonyl group include indolylacetylmethoxycarbonyl group, methoxycarbonyl group, ethoxycarbonyl group, propoxycarbonyl group, isopropoxycarbonyl group, butoxycarbonyl group, isobutoxycarbonyl group, sec-butoxycarbonyl group, tert-butoxycarbonyl group (also referred to as Boc), phenylmethoxycarbonyl group, 9-fluorenylmethyloxycarbonyl group (also referred to as Fmoc), 3-methylphenyloxyacetyl group, 3-chlorophenylmethoxycarbonyl group, trifluoromethoxycarbonyl group, 2,5-dichlorobenzoyl group, and benzyloxycarbonyl group (also referred to as Cbz). Examples of the optionally substituted alkoxycarbonyl group include, in particular, tert-butoxycarbonyl group, 9-fluorenylmethyloxycarbonyl group, and benzyloxycarbonyl group.

The optionally substituted alkoxycarbonylamino group is an unsubstituted alkoxycarbonylamino group or a substituted alkoxycarbonylamino group. To the alkoxy group (or alkoxycarbonyl group) constituting the alkoxycarbonylamino group, the above description for the alkoxy group (or alkoxycarbonyl group) can be applied. To the alkoxy group (or alkoxycarbonyl group) and substituent constituting the substituted alkoxycarbonylamino group, the above descriptions for the alkoxy group (or alkoxycarbonyl group) and substituent thereof can be applied. Specific examples of the optionally substituted alkoxycarbonylamino group include tert-butoxycarbonylamino group, 9-fluorenylmethyloxycarbonylamino group, and benzyloxycarbonylamino group.

The optionally substituted alkylthio group is an unsubstituted alkylthio group or a substituted alkylthio group. To the alkyl group constituting the alkylthio group, the above descriptions for the alkyl group and substituent can be applied. To the alkyl group and substituent constituting the substituted alkylthio group, the above descriptions for the alkyl group and substituent thereof can be applied. Specific examples of the optionally substituted alkylthio group include methylthio group.

Examples of the acyl group include, in particular, an optionally substituted alkylcarbonyl group, and an optionally substituted alkoxycarbonyl group. Specific examples of the acyl group include acetyl group, propionyl group, butyryl group (also referred to as butanoyl group), isobutyryl group, valeryl group (also referred to as pentanoyl group), isovaleryl group, pivaloyl group, hexanoyl group, acryloyl group, methacryloyl group, crotonoyl group, isocrotonoyl group, cyclopropanoyl group, cyclobutanoyl group, cyclopentanoyl group, cyclohexanoyl group, benzoyl group, pteroyl group, aminoacetyl group, 2-oxo-3-methylbutanoyl group, and the examples of the optionally substituted alkoxycarbonyl group mentioned above. Examples of the acyl group include, in particular, acetyl group, tert-butoxycarbonyl group, 9-fluorenylmethyloxycarbonyl group, and benzyloxycarbonyl group. Specific examples of the acyl group also include an acyl group corresponding to an amino acid. The term "acyl group corresponding to an amino acid" means a group formed by eliminating OH of the carboxyl group of the amino acid. For example, the acyl group corresponding to glycine is aminoacetyl group. Amino acids are discussed below.

The "amino acid" as R⁵ means an amino acid bonding to C(=O) of R³ via an appropriate bond.

Examples of the amino acid as R⁵ include an amino acid bonding through amino group, amino acid bonding through carboxyl group, amino acid bonding through hydroxyl group, amino acid bonding through thiol group, and amino acid bonding through amide group. Examples of the amino acid as R⁵ include, among others, an amino acid bonding through amino group.

The term "amino acid bonding through amino group" means an amino acid bonding to C(=O) of R³ at the nitrogen atom of the amino group. When the amino acid has two or more amino groups, any of those amino groups may be used for bonding to C(=O) of R³. For example, an α-amino group may be used for bonding with C(=O) of R³, or an ε-amino group may be used for bonding with C(=O) of R³.

The term "amino acid bonding through carboxyl group" means an amino acid bonding to C(=O) of R³ at the oxygen atom of OH of the carboxyl group. When the amino acid has two or more carboxyl groups, any of those carboxyl groups may be used for bonding to C(=O) of R³ . For example, an α-carboxyl group may be used for bonding to C(=O) of R³, or another carboxyl group may be used for bonding to C(=O) of R³.

The term "amino acid bonding through hydroxyl group" means an amino acid bonding to C(=O) of R³ at the oxygen atom of the hydroxyl group.

The term "amino acid bonding through thiol group" means an amino acid bonding to C(=O) of R³ at the sulfur atom of the thiol group.

The term "amino acid bonding through amide group" means an amino acid bonding to C(=O) of R³ at the nitrogen atom of the amide group.

Examples of the amino acid include basic amino acids such as lysine, ornithine, arginine, histidine, citrulline, diaminobutanoic acid, and diaminopropanoic acid, aliphatic amino acids such as isoleucine, alanine, valine, leucine, and glycine, amino acids that are hydroxymonoaminocarboxylic acids such as threonine and serine, cyclic amino acids such as proline (also referred to as "imino acids"), aromatic amino acids such as phenylalanine, tyrosine, and tryptophan, sulfur-containing amino acids such as cysteine, cystine, and methionine, acidic amino acids such as glutamic acid and aspartic acid, and amino acids having amide group on the side chain such as glutamine and asparagine. Examples of the amino acid also include norvaline, norleucine, α-aminobutyric acid, γ-aminobutyric acid, hydroxyproline, tert-leucine, and sarcosine. The amino acid may or may not have a substituent on the amino group and/or carboxyl group thereof. That is, the "amino acid" includes those having a substituent on the amino group and/or carboxyl group thereof, unless especially noted. That is, examples of the amino acid include such amino acids as described above further having a substituent on the amino group and/or carboxyl group thereof. Examples of the substituent of the amino group in the amino acid include an optionally substituted alkoxycarbonyl group, and an optionally substituted alkyl group. That is, the amino group in the amino acid may constitute, for example, an optionally substituted alkoxycarbonylamino group, or an optionally substituted alkylamino group. Examples of the substituent of the amino group in the amino acid include, in particular, tert-butoxycarbonyl group, 9-fluorenylmethyloxycarbonyl group, benzyloxycarbonyl group, and acetyl group. Examples of the substituent of the carboxyl group in the amino acid include an optionally substituted alkyl group, and amino group. That is, the carboxyl group of the amino acid may constitute, for example, an optionally substituted alkoxycarbonyl group, or aminocarbonyl group. Examples of the substituent of the carboxyl group in the amino acid include, in particular, methyl group, ethyl group, tert-butyl group, and benzyl group.

The amino acid may be a D-amino acid, L-amino acid, or a combination thereof, unless especially noted. The ratio of D- and L-amino acids in the combination is not particularly restricted. The ratio of D- or L-amino acid in the combination may be, for example, 20 to 80%, 30 to 70%, 40 to 60%, or 45 to 55% in molar ratio. The amino acid may be, in particular, an L-amino acid. When a D- or L-amino acid is selected, it is sufficient to use that D- or L-amino acid, but the corresponding L- or D-amino acid may further be used in combination.

The "dipeptide" as R⁵ means a dipeptide bonding to C(=O) of R³ via an appropriate bond.

Examples of the dipeptide as R⁵ include a dipeptide bonding through amino group, dipeptide bonding through carboxyl group, dipeptide bonding through hydroxyl group, dipeptide bonding through thiol group, and dipeptide bonding through amide group. Examples of the dipeptide as R⁵ include, in particular, a dipeptide bonding through amino group.

The term "dipeptide bonding through amino group" means a dipeptide bonding to C(=O) of R³ at the nitrogen atom of the amino group. If the dipeptide has two or more amino groups, any of those amino groups may be used for bonding to C(=O) of R³. For example, α-amino group may be used for bonding to C(=O) of R³, or ε-amino group may be used for bonding to C(=O) of R³ .

The term "dipeptide bonding through carboxyl group" means a dipeptide bonding to C(=O) of R³ at the oxygen atom of OH of the carboxyl group. If the dipeptide has two or more carboxyl groups, any of those carboxyl groups may be used for bonding to C(=O) of R³. For example, α-carboxyl group may be used for bonding to C(=O) of R³, or another carboxyl group may be used for bonding to C(=O) of R³.

The term "dipeptide bonding through hydroxyl group" means a dipeptide bonding to C(=O) of R³ at the oxygen atom of the hydroxyl group.

The term "dipeptide bonding through thiol group" means a dipeptide bonding to C(=O) of R³ at the sulfur atom of the thiol group.

The term "dipeptide bonding through amide group" means a dipeptide bonding to C(=O) of R³ at the nitrogen atom of the amide group.

To the amino acids constituting the dipeptide, the above description for the amino acid can be applied. Two amino acids constituting the dipeptide may be the same amino acids or may be different amino acids. Specific examples of the dipeptide include Val-Val, Glu-Val, Cys-Gly, Glu-Glu, Glu-Gly, Gly-Gly, Glu-Phe, and Glu-Pro. The dipeptide may or may not have a substituent on the amino group and/or carboxyl group thereof. That is, examples of the "dipeptide" include those having a substituent on the amino group and/or carboxyl group thereof, unless especially noted. To the substituent of the amino group and/or carboxyl group in the dipeptide, the above description for the substituent of the amino and/or carboxyl group in the amino acid can be applied.

Specific examples of X, n, and R¹ to R⁹ include X, n, and R¹ to R⁹ of the compounds represented by the formulas of Compound 1-66 and the compounds represented by the formulas of Compound 67-75 described below. That is, specifically, X, n, and R¹ to R⁹ may be selected from, for example, X, n, and R¹ to R⁹ of the compounds represented by the formulas of Compound 1-66 and the compounds represented by the formulas of Compound 67-75 described below.

Specific examples of the ingredient (A) include compounds represented by the formulas of Compound 1-58 and compounds represented by the formulas of Compound 67-75 described below. Examples of the ingredient (A) include, in particular, the compounds represented by the formulas of Compound 1-58 described below. Specific examples of the ingredient (A) also include the compounds represented by the formulas of Compound 59-66 mentioned below.

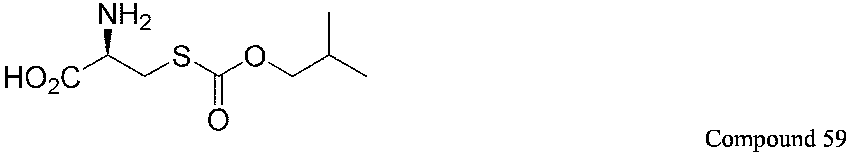

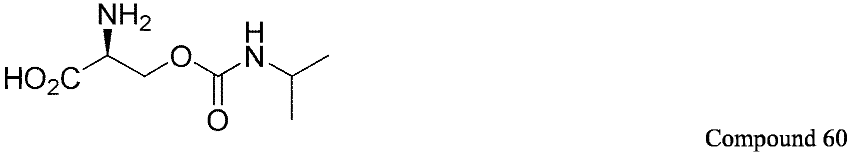

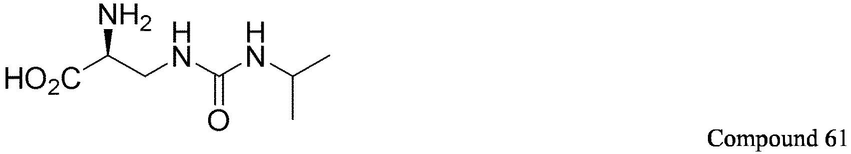

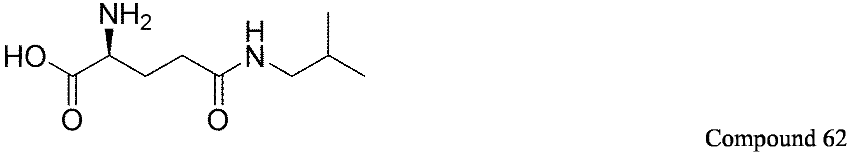

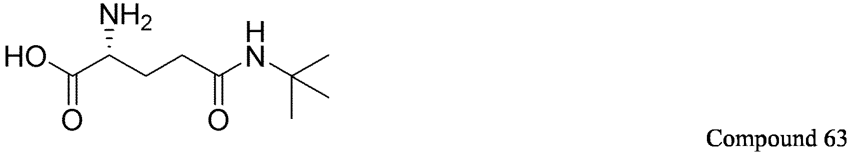

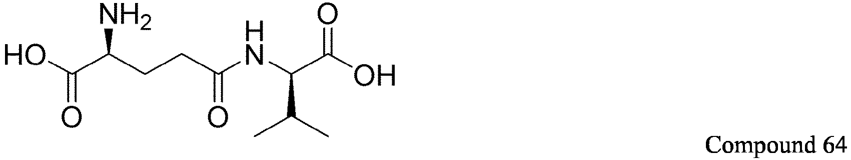

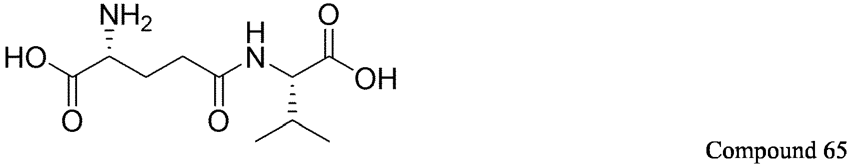

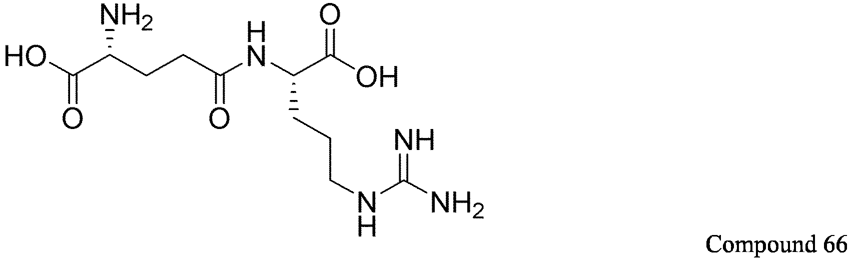

When the ingredient (A) can form a salt, the ingredient (A) may be used as a free compound, a salt, or a combination thereof. That is, the term "ingredient (A)" may mean the ingredient (A) in the form of a free compound, a salt thereof, or a combination thereof, unless especially noted. The term "free compound" means a compound that does not form a salt. The salt is not particularly limited as long as it does not impair the effects of the present invention. For example, examples of the salt as a salt for acidic group such as carboxyl group include ammonium salts, salts with alkali metals such as sodium and potassium, salts with alkaline earth metals such as calcium and magnesium, aluminum salts, zinc salts, salts with organic amines such as triethylamine, ethanolamine, morpholine, pyrrolidine, piperidine, piperazine, and dicyclohexylamine, and salts with basic amino acids such as arginine and lysine. Examples of the salt as a salt for basic group such as amino group include salts with inorganic acids such as hydrochloric acid, sulfuric acid, phosphoric acid, nitric acid, and hydrobromic acid, salts with organic carboxylic acids such as acetic acid, citric acid, benzoic acid, maleic acid, fumaric acid, tartaric acid, succinic acid, tannic acid, butyric acid, hibenzic acid, pamoic acid, enanthic acid, decanoic acid, theoclic acid, salicylic acid, lactic acid, oxalic acid, mandelic acid, and malic acid, and salts with organic sulfonic acids such as methanesulfonic acid, benzenesulfonic acid, and p-toluenesulfonic acid. As the salt, one kind of salt may be used, or a combination of two or more kinds of salts may be used.

When the ingredient (A) can form a hydrate, the ingredient (A) may be used as non-hydrate, hydrate, or a combination thereof. That is, the term "ingredient (A)" (e.g., "ingredient (A) as a free compound" or "salt of ingredient (A)") may encompass both non-hydrate and hydrate, unless especially noted.

The ingredient (A) may be in any form, such as the form of ion, at the time of use.

As the ingredient (A), one kind of ingredient may be used, or a combination of two or more kinds of ingredients may be used. When two or more kinds of ingredients are selected as the ingredient (A), "amount" or "concentration" of the ingredient (A) may mean the total amount or total concentration of the selected ingredients, unless especially noted.

As the ingredient (A), a commercial product may be used, or one obtained by appropriately producing it may be used. The method for producing the ingredient (A) is not particularly limited. The ingredient (A) can be produced by, for example, known methods. The ingredient (A) can be specifically produced by, for example, chemical synthesis, enzymatic reaction, fermentation, extraction, or a combination thereof. The ingredient (A) may or may not be purified to a desired degree. That is, as the ingredient (A), a purified product or a material containing the ingredient (A) may be used. Specific examples of the material containing the ingredient (A) include, for example, fermentation products obtained by culturing a microorganism capable of producing the ingredient (A) such as culture medium, bacterial cells, and culture supernatant, agricultural, marine, and livestock products containing the ingredient (A), and processed products thereof. Examples of the processed products include those obtained by subjecting the above-mentioned materials such as the fermentation products to such a treatment as concentration, dilution, drying, fractionation, extraction, and purification. For example, a material containing 1% (w/w) or more, 5% (w/w) or more, 10% (w/w) or more, 30% (w/w) or more, 50% (w/w) or more, 70% (w/w) or more, 90% (w/w) or more, or 95% (w/w) or more of the ingredient (A) may be used as the ingredient (A).

By utilizing the active ingredient, specifically, by applying the active ingredient to a plant, for example, pest damage resistance may be induced in the plant, i.e., an effect of inducing pest damage resistance in the plant may be obtained. This effect is also referred to as "pest damage resistance-inducing effect". Therefore, the active ingredient may be used, for example, to induce pest damage resistance in a plant. The term "pest damage resistance" may mean resistance to pest damage, specifically, a property of reducing degree of pest damage. The expression "to induce pest damage resistance in a plant" may be used interchangeably with "to impart pest damage resistance to a plant" or "to improve pest damage resistance of a plant".

By utilizing the active ingredient, specifically, by applying the active ingredient to a plant, for example, pest damage to the plant may be prevented, i.e., an effect of preventing pest damage to the plant may be obtained. This effect is also referred to as "pest damage-preventing effect". Therefore, the active ingredient may be used, for example, to prevent pest damage to a plant. In particular, the pest damage-preventing effect may be obtained by inducing pest damage resistance in a plant. Therefore, the pest damage-preventing effect may be an example of the pest damage resistance-inducing effect. The term "prevention of pest damage" may be used interchangeably with "reduction of pest damage", "amelioration of pest damage", or "control of pest damage".

By utilizing the active ingredient, specifically, by applying the active ingredient to a plant, for example, cultivation performance of the plant may be improved, i.e., an effect of improving cultivation performance of a plant may be obtained. This effect is also referred to as the "cultivation performance-improving effect". The cultivation performance-improving effect may be obtained by, in particular, inducing pest damage resistance in a plant and/or preventing pest damage to a plant. Therefore, the cultivation performance-improving effect may be an example of the pest damage resistance-inducing effect or the pest damage-preventing effect. Examples of improvement of cultivation performance of a plant include improvement of yield of a plant, improvement of growth of a plant, and improvement of viability of a plant. Examples of improvement of cultivation performance of a plant include, in particular, improvement of yield of a plant.

The type of the plant is not particularly limited as long as it may be damaged by pests.

The plant may be, for example, a woody plant or herbaceous plant. Examples of the plant include *Poaceae* plants (rice *(Oryza sativa*)*,* barley (*Hordeum vulgare*)*,* wheat (*Triticum* spp.), maize (*Zea mays*)*,* sorghum (*Sorghum bicolor*)*,* millet (*Panicum miliaceum*)*,* sugarcane (*Saccharum officinarum*), oat (*Avena sativa*)*,* lawngrass (*Zoysia* spp.), pearl millet (*Pennisetum glaucum*)*,* finger millet (*Eleusine coracana*)*,* fonio (*Digitaria exilis*)*,* etc.), *Solanaceae* plants (tomato (*Solanum lycopersicum*)*,* bell pepper (*Capsicum annuum* var. grossum), eggplant (*Solanum melongena*)*,* potato (*Solanum tuberosum*)*,* tobacco (*Nicotiana tabacum*)*,* etc.), *Cucurbitaceae* plants (cucumber (*Cucumis sativus*)*,* melon (*Cucumis melo*)*,* pumpkin (*Cucurbita* spp.), etc.), *Fabaceae* plants (pea (*Pisum sativum*)*,* soybean (*Glycine max*)*,* common bean (*Phaseolus vulgaris*)*,* alfalfa (*Medicago sativa*)*,* peanut (*Arachis hypogaea*)*,* broad bean (faba bean, *Vicia faba*)*,* cowpea (*Vigna unguiculata*)*,* lentil (*Lens culinaris*)*,* chickpea (*Cicer arietinum*)*,* clover (*Trifolium* spp.), peanut (*Arachis hypogaea*)*,* bambara bean (bambara groundnut, *Vigna subterranea*)*,* etc.), *Brassicaceae* plants (radish (*Raphanus sativus*)*,* Chinese cabbage (*Brassica rapa* subsp. *pekinensis*)*,* cabbage (*Brassica oleracea* var. *capitata*)*,* komatsuna (Japanese mustard spinach, *Brassica rapa* var. *perviridis*)*,* nanohana (rapeseed, *Brassica* spp.), bok choy (*Brassica rapa* subsp. *chinensis*)*,* thale cress (*Arabidopsis thaliana*)*,* etc.), *Rosaceae* plants (strawberry (*Fragaria* × *ananassa*)*,* apple (*Malus domestica*)*,* pear (*Pyrus spp*.)*,* peach (*Prunus persica*)*,* etc.), *Moraceae* plants (mulberry (*Morus* spp,), etc.), *Malvaceae* plants (cotton (*Gossypium* spp.), etc.), *Apiaceae* plants (carrot (*Daucus carota* subsp. *sativus*)*,* parsley (*Petroselinum crispum*)*,* celery (*Apium graveolens*)*,* etc.), *Liliaceae* plants (Welsh onion (*Allium fistulosum*)*,* onion (*Allium cepa*)*,* asparagus (*Asparagus officinalis*)*,* etc.), *Asteraceae* plants (burdock (*Arctium lappa*)*,* sunflower (*Helianthus annuus*)*,* chrysanthemum (*Chrysanthemum* spp.), garland chrysanthemum (*Glebionis coronaria*)*,* safflower (*Carthamus tinctorius*)*,* lettuce (*Lactuca sativa*)*,* etc.), *Amaranthaceae* plants (sugar beet (*Beta vulgaris* subsp. *vulgaris* var. *altissima*)*,* etc.), *Ericaceae* plants (blueberry (*Vaccinium* sect. *Cyanococcus*)*,* cranberry (*Vaccinium* subgenus *Oxycoccus* spp.), etc.), *Vitaceae* plants (grape (*Vitis* spp.), etc.), *Rutaceae* plants (satsuma mandarin (*Citrus unshiu*)*,* lemon (*Citrus limon*)*,* yuzu (*Citrus junos*)*,* etc.), *Rubiaceae* plants (coffee tree (*Coffea* spp.), etc.), *Oleaceae* plants (olive (*Olea europaea*)*,* etc.), *Lauraceae* plants (avocado (*Persea americana*)*,* etc.), *Anacardiaceae* plants (mango (*Mangifera indica*)*,* cashew (*Anacardium occidentale*)*,* etc.), *Sapindaceae* plants (lychee (*Litchi chinensis*)*,* etc.), and *Lamiaceae* plants (coleus (*Coleus scutellarioides*)*,* etc.).

As for the plant, one species of plant, or two or more species of plants may be the subject.

The term "pest damage" may refer to damage caused by a pest. Examples of the pest damage include feeding damage (i.e., feeding of plants by pests).

As for the type of the pest, the pest is not particularly limited as long as it can cause pest damage.

Examples of the pest include agricultural pests.

Specific examples of the pests include organisms classified into the order *Lepidoptera,* (families *Plutellidae, Noctuidae, Pyralidae, Tortricidae, Lyonetiidae, Carposinidae, Gelechiidae, Crambidae, Arctiidae (Erebidae), Lymantriidae* (*Erebidae*)*,* etc.), *Hemiptera* (families *Cicadellidae, Delphacidae, Psyllidae, Aphididae, Aleyrodidae, Coccoidea, Miridae, Tingidae, Pentatomoidea, Lygaeidae,* etc.), *Coleoptera* (families *Scarabaeidae, Elateridae, Coccinellidae, Cerambycidae, Chrysomelidae, Curculionidae,* etc.), *Diptera* (families *Muscidae, Calliphoridae, Sarcophagidae, Anthomyiidae, Tephritidae,* superfamilies *Opomyzoidea, Chloropoidea,* etc.), *Orthoptera* (families *Acrididae, Catantopidae, Pyrgomorphidae,* etc.), *Thysanoptera* (families *Thripidae, Aeolothripidae, Merothripidae,* etc.), *Tylenchida* (families *Aphelenchoididae, Neotylenchidae,* etc.), *Collembola* (families *Onychiuridae, Isotomidae,* etc.), *Acarina* (families *Tetranychidae, Dermanyssidae, Acaridae, Sarcoptidae,* etc.), or *Stylommatophora* (families *Philomycidae, Bradybaenidae,* etc.).

Specific examples of pests of the order *Lepidoptera* include *Chilo suppressalis, Cnaphalocrocis medinalis, Parnara guttata, Sesamia inferens, Mythimna separate, Naranga aenescens, Spodoptera litura, Etiella zinckenella, Etiella behrii, Matsumuraeses falcana, Leguminivora glycinivorella, Pleuroptya ruralis, Agrotis segetum, Agrotis ipsilon, Helcystogramma triannulella, Xestia c-nigrum, Helicoverpa assulta, Helicoverpa armigera, Mamestra brassicae, Spodoptera exigua, Plutella xylostella, Pieris rapae, Pieris brassicae, Hellula undalis,* and *Autographa nigrisigna.* Specific examples of pests of the order *Hemiptera* include *Nilaparvata lugens, Sogatella furcifera, Laodelphax striatellus, Nephotettix cincticeps, Recilia dorsalis, Stenotus rubrovittatus, Trigonotylus caelestialium, Leptocorisa chinensis, Nezara antennata, Nezara viridula, Lagynotomus elongatus, Scotinophara lurida, Eysarcoris aeneus, Eysarcoris lewisi, Eysarcoris ventralis, Togo hemipterus, Cletus punctiger, Piezodorus hybneri, Halyomorpha halys, Dolycoris baccarum, Neotoxoptera formosana, Rhopalosiphum padi, Rhopalosiphum maidis,* and *Aphis glycines.* Specific examples of pests of the order *Coleoptera* include *Hypothenemus hampei, Lissorhoptrus oryzophilus, Oulema oryzae, Echinocnemus squamous, Melanotus legatus, Melanotus foltnumi*, *Anomala cuprea, Anomala cuprea, Popillia japonica, Maladera castanea, Epilachna varivestis, Medythia nigrobilineata, Epilachna vigintioctomaculata, Henosepilachna vigintioctopunctata, Harmonia axyridis, Anomala rufocuprea, Mimela testaceipes, Aulacophora femoralis,* and *Phyllotreta striolata.* Specific examples of pests of the order *Diptera* include *Chlorops oryzae, Hydrellia griseola, Sitodiplosis mosellana, Delia platura, Asphondylia yushimai, Melanagromyza sojae, Liriomyza trifolii, Liriomyza sativae, Liriomyza huidobrensis,* and *Liriomyza bryoniae.* Specific examples of pests of the order *Orthoptera* include *Oxya yezoensis* and *Oxya japonica.* Specific examples of pests of the order *Thysanoptera* include *Stenchaetothrips biformis* and *Thrips palmi.* Specific examples of pests of the order *Tylenchida* include *Meloidogyne* spp. (*Meloidogyne* nematodes), *Pratylenchus* spp. (*Pratylenchus* nematodes), and *Globodera* spp. (*Globodera* nematodes). Specific examples of pests of the order *Collembola* include *Onychiurus pseudarmatus* and *Onychiurus matsumotoi.* Specific examples of pests of the order *Acarina* include *Penthaleus major, Tetranychus urticae, Tetranychus kanzawai, Tyrophagus putrescentiae,* and *Tarsonemus bilobatus.* Specific examples of pests of the order *Stylommatophora* include snails and slugs.

The target pest may consist of one species of pest, or two or more species of pests.

The pest damage resistance-inducing effect can be confirmed by, for example, using Ca²⁺ signal transduction as an indicator. That is, if Ca²⁺ signal transduction is increased when the active ingredient is used compared with the same observed when the active ingredient is not used, the pest damage resistance-inducing effect may be provided by the active ingredient. The Ca²⁺ signal transduction can be confirmed by, for example, calcium imaging using a calcium indicator or calcium-sensitive fluorescent protein. Specifically, Ca²⁺ signal transduction can be confirmed by calcium imaging, for example, in a recombinant plant modified to express a calcium-sensitive protein. Examples of the calcium-sensitive fluorescent protein include Cameleon, TN-XL, GCaMP3, and G-GECO. Detection of signals (e.g., fluorescence) in calcium imaging can be performed by using a fluorescence detector or the like appropriate for the type of signal. Calcium imaging can be specifically performed by, for example, the procedure described in the section of Examples.

The pest damage resistance-inducing effect can be confirmed by using, for example, expression of a pest damage resistance gene as an indicator. That is, if the expression of the pest damage resistance gene increases when the active ingredient is used compared with the same observed when the active ingredient is not used, the pest damage resistance-inducing effect might be provided by the active ingredient. Examples of the pest damage resistance gene include the AtOPR3 gene, which encodes 12-oxophytodienoic acid reductase involved in the jasmonic acid biosynthesis. Increased expression of a gene can be confirmed by, for example, measuring transcription amount of the gene (e.g., amount of mRNA), translation amount of the gene (e.g., amount of protein encoded by the gene), or activity of the protein encoded by the gene. Examples of the method for measuring amount of mRNA include Northern hybridization and qPCR. Examples of the method for measuring amount of protein include Western blotting. The method for measuring protein activity can be appropriately selected according to various conditions, such as the type of objective protein.

The pest damage resistance-inducing effect can be confirmed by using, for example, the pest damage-preventing effect as an indicator. That is, when the pest damage-preventing effect is obtained with the active ingredient, it can be judged that the active ingredient has provided the pest damage resistance-inducing effect.

The pest damage-preventing effect can be confirmed by using, for example, degree of pest damage (e.g., amount of plants fed by pests) as an indicator. That is, when the degree of pest damage (e.g., amount of plants fed by pests) is reduced when the active ingredient is used compared with that observed when the active ingredient is not used, it can be judged that the active ingredient has provided the pest damage-preventing effect.

The pest damage resistance-inducing effect or pest damage-preventing effect can be confirmed by, for example, using cultivation performance-improving effect as an indicator. That is, when the active ingredient provided a cultivation performance-improving effect, it can be judged that the active ingredient has provided the pest damage resistance-inducing effect or pest damage-preventing effect.

The cultivation performance-improving effect can be confirmed by using, for example, cultivation performance (e.g., yield, growth, or viability) of a plant as an indicator. That is, if cultivation performance (e.g., yield, growth, or viability) of a plant is improved when the active ingredient is used compared with the same observed when the active ingredient is not used, it can be judged that the active ingredient has provided the cultivation performance-improving effect.

### 2. Composition of the present invention

The composition of the present invention is a composition containing the active ingredient (i.e., the ingredient (A) mentioned above).

The composition of the present invention can be used by applying it to a plant. The manner of use of the composition of the present invention will be described in detail in the section of "Method of the present invention". The composition of the present invention can be used, for example, to achieve the effects exemplified above.

By utilizing the composition of the present invention, specifically, by applying the composition of the present invention to a plant, for example, pest damage resistance may be induced in the plant, i.e., the pest damage resistance-inducing effect may be obtained. Therefore, the composition of the present invention may be, for example, a composition for inducing pest damage resistance in a plant. A composition for inducing pest damage resistance in a plant is also referred to as a "pest damage resistance inducer for plants".

By utilizing the composition of the present invention, specifically, by applying the composition of the present invention to a plant, for example, pest damage to the plant may be prevented, i.e., the pest damage-preventing effect may be obtained. Therefore, the composition of the present invention may be, for example, a composition for preventing pest damage to a plant. A composition for preventing pest damage to a plant is also referred to as a "pest damage-preventing agent for plants". Such a composition for preventing pest damage to a plant may be an example of the composition for inducing pest damage resistance in a plant.

By utilizing the composition of the present invention, specifically, by applying the composition of the present invention to a plant, for example, cultivation performance of the plant may be improved, i.e., the cultivation performance-improving effect may be obtained. Therefore, the composition of the present invention may be, for example, a composition for improving cultivation performance of a plant. A composition for improving cultivation performance of a plant is also referred to as a "plant cultivation performance-improving agent". A composition for improving cultivation performance of a plant may be an example of the composition for inducing pest damage resistance in a plant or composition for preventing pest damage to a plant.

Because of such uses of the composition of the present invention as exemplified above, the composition of the present invention may be useful, for example, in the agro-horticultural field. Therefore, the composition of the present invention may be used, for example, in the agro-horticultural field. That is, the composition of the present invention may be, for example, a composition for agro-horticulture. The composition of the present invention may be specifically, for example, a composition for agro-horticulture used for such uses of the composition of the present invention as exemplified above.

The composition of the present invention may be provided as, for example, a pesticide. The composition of the present invention may also be provided as, for example, a fertilizer.

The physicochemical composition of the composition of the present invention is not particularly limited as long as the composition of the present invention contains the active ingredient and provides the desired effect. Types and amounts of ingredients contained in the composition of the present invention may be appropriately selected according to various conditions such as the type of the object, cultivation method of the object, growth stage of the object, purpose of use of the composition of the present invention, and manner of use of the composition of the present invention.

The composition of the present invention may consist of the active ingredient or may also contain an ingredient other than the active ingredient. Examples of the ingredient other than the active ingredient include, for example, ingredients normally used for pesticides, fertilizers, pharmaceuticals, and so forth. Specific examples of such ingredients include, for example, excipient, binder, disintegrant, lubricant, stabilizer, diluent, surfactant, spreading agent, pH adjuster, water, alcohol, vitamins, minerals, and other additives. Specific examples of the spreading agent include, for example, Approach BI^{™} (Kao Corporation), Mix Power^{™} (Syngenta Japan), and Squash^{™} (Maruwa Biochemical). Other than the active ingredient, one kind of ingredient may be used, or two or more kinds of ingredients may be used. The composition of the present invention may be formed in the form of a preparation as appropriate. The form of the composition of the present invention is not particularly limited. The form of the composition of the present invention can be appropriately selected according to various conditions, such as the manner of use of the composition of the present invention. Examples of the form include liquid, suspension, powder, tablet, pill, capsule, and paste.

The content of the active ingredient in the composition of the present invention is more than 0% (w/w) and not more than 100% (w/w). The content of the active ingredient in the composition of the present invention may be, for example, 20 ppm (w/w) or higher, 50 ppm (w/w) or higher, 100 ppm (w/w) or higher, 200 ppm (w/w) or higher, 500 ppm (w/w) or higher, 1,000 ppm (w/w) or higher, 2,000 ppm (w/w) or higher, 5,000 ppm (w/w) or higher, 1% (w/w) or higher, 2% (w/w) or higher, 5% (w/w) or higher, 10% (w/w) or higher, 20% (w/w) or higher, 30% (w/w) or higher, 50% (w/w) or higher, or 70% (w/w) or higher, and may be 100% (w/w) or lower, lower than 100% (w/w), 99.9% (w/w) or lower, 90% (w/w) or lower, 70% (w/w) or lower, 50% (w/w) or lower, 30% (w/w) or lower, 20% (w/w) or lower, 10% (w/w) or lower, 5% (w/w) or lower, 2% (w/w) or lower, 1% (w/w) or lower, 5,000 ppm (w/w) or lower, 2,000 ppm (w/w) or lower, 1,000 ppm (w/w) or lower, or 500 ppm (w/w) or lower, or in a range defined by any non-contradictory combination of these exemplary maximum and minimum contents. The content of the active ingredient in the composition of the present invention may specifically be, for example, 20 ppm (w/w) to 50 ppm (w/w), 50 ppm (w/w) to 100 ppm (w/w), 100 ppm (w/w) to 1,000 ppm (w/w), 1,000 ppm (w/w) to 1% (w/w), 1% (w/w) to 10% (w/w), 10% (w/w) to 30% (w/w), 30% (w/w) to 50% (w/w), 50% (w/w) to 70% (w/w), or 70% (w/w) to 99.9% (w/w). The content of the active ingredient in the composition of the present invention may specifically be, for example, 20 ppm (w/w) to 99.9% (w/w), 100 ppm (w/w) to 99.9% (w/w), 1,000 ppm (w/w) to 99.9% (w/w), 1% (w/w) to 99.9% (w/w), 10% (w/w) to 99.9% (w/w), 100 ppm (w/w) to 50% (w/w), 100 ppm (w/w) to 10% (w/w), 100 ppm (w/w) to 1% (w/w), 1,000 ppm (w/w) to 50% (w/w), 1,000 ppm (w/w) to 10% (w/w), 1,000 ppm (w/w) to 1% (w/w), 1% (w/w) to 50% (w/w), or 1% (w/w) to 10% (w/w).

The content of the active ingredient in the composition of the present invention may be, for example, 0.1 mM or higher, 0.2 mM or higher, 0.5 mM or higher, 1 mM or higher, 2 mM or higher, 5 mM or higher, 10 mM or higher, 20 mM or higher, 50 mM or higher, 100 mM or higher, 200 mM or higher, or 500 mM or higher, and may be 1,000 mM or lower, 500 mM or lower, 200 mM or lower, 100 mM or lower, 50 mM or lower, 20 mM or lower, 10 mM or lower, 5 mM or lower, 2 mM or lower, or 1 mM or lower, or in a range defined by any non-contradictory combination of these exemplary maximum and minimum contents. The content of the active ingredient in the composition of the present invention may be, for example, 0.1 to 0.2 mM, 0.2 to 0.5 mM, 0.5 to 1 mM, 1 to 2 mM, 2 to 5 mM, 5 to 10 mM, 10 to 20 mM, 20 to 50 mM, 50 to 100 mM, 100 to 200 mM, 200 to 500 mM, or 500 to 1,000 mM. The content of the active ingredient in the composition of the present invention may specifically be, for example, 0.1 to 1,000 mM, 0.5 to 500 mM, or 2 to 200 mM.

The content of the active ingredient in the composition of the present invention can be set, for example, so that the concentration of the active ingredient at the time of use of the composition of the present invention is within a predetermined range. The concentration of the active ingredient at the time of use of the composition of the present invention is also referred to as the "concentration of the active ingredient for use" or "application concentration of the active ingredient". The concentration of the active ingredient for use may be, in particular, the concentration of the composition of the present invention when it is used in the form of liquid.

The concentration of the active ingredient for use may be, for example, 0.1 mM or higher, 0.2 mM or higher, 0.5 mM or higher, 1 mM or higher, 2 mM or higher, 5 mM or higher, 10 mM or higher, 20 mM or higher, 50 mM or higher, 100 mM or higher, 200 mM or higher, or 500 mM or higher, and may be 1,000 mM or lower, 500 mM or lower, 200 mM or lower, 100 mM or lower, 50 mM or lower, 20 mM or lower, 10 mM or lower, 5 mM or lower, 2 mM or lower, or 1 mM or lower, or in a range defined by any non-contradictory combination of these exemplary maximum and minimum concentrations. The concentration of the active ingredient for use may be, for example, 0.1 to 0.2 mM, 0.2 to 0.5 mM, 0.5 to 1 mM, 1 to 2 mM, 2 to 5 mM, 5 to 10 mM, 10 to 20 mM, 20 to 50 mM, 50 to 100 mM, 100 to 200 mM, 200 to 500 mM, or 500 to 1,000 mM. The concentration of the active ingredient for use may specifically be, for example, 0.1 to 1,000 mM, 0.5 to 500 mM, or 2 to 200 mM.

When a material containing the active ingredient is used, the amount of the active ingredient (e.g. content (concentration) or amount for use) shall be calculated on the basis of the amount of the active ingredient itself in the material. When the active ingredient forms a salt or hydrate, the amount of the active ingredient (e.g., content (concentration) or amount for use) shall be calculated on the basis of a value obtained by converting the mass of the salt or hydrate to the mass of the corresponding free compound in an equimolar amount.

The active ingredient and other ingredients may be contained in the composition of the present invention as a mixture thereof, or they may be contained in the composition of the present invention each separately, or separately in any combinations thereof.

### 3. Method of the present invention

The method of the present invention is a method comprising applying the active ingredient (i.e., the ingredient (A) mentioned above) to a plant. The method of the present invention can be implemented, for example, in order to obtain the effects exemplified above.

By implementing the method of the present invention, specifically, by applying the active ingredient to a plant, for example, pest damage resistance may be induced in the plant, i.e., the pest damage resistance-inducing effect may be obtained. Therefore, the method of the present invention may be, for example, a method for inducing pest damage resistance in a plant.

By implementing the method of the present invention, specifically, by applying the active ingredient to a plant, for example, pest damage to the plant may be prevented, i.e., the pest damage-preventing effect may be obtained. Therefore, the method of the present invention may be, for example, a method for preventing pest damage to a plant. The method for preventing pest damage to a plant may be an example of the method for inducing pest damage resistance in a plant.

By implementing the method of the present invention, specifically, by applying the active ingredient to a plant, for example, cultivation performance of the plant may be improved, i.e., the cultivation performance-improving effect may be obtained. Therefore, the method of the present invention may be, for example, a method for improving cultivation performance of a plant. The method for improving cultivation performance of a plant may be an example of the method for inducing pest damage resistance in a plant or method for preventing pest damage to a plant.

The active ingredient can be applied to a plant, for example, by utilizing the composition of the present invention (i.e., by applying the composition of the present invention). That is, one embodiment of the method of the present invention may be, for example, a method comprising applying the composition of the present invention to a plant. The "applying the active ingredient to a plant" includes applying the composition of the present invention to a plant. For example, the composition of the present invention can be applied to a plant as it is, or diluted, dispersed, or dissolved in a liquid such as water, physiological saline, buffer, alcohol, and DMSO as appropriate, and applied to a plant. That is, the composition of the present invention can be applied to a plant, for example, after the concentration of the active ingredient thereof is appropriately adjusted so that such a concentration of the active ingredient for use as exemplified above is obtained. The composition of the present invention can be applied to a plant, in particular, in the form of liquid. The composition of the present invention may be used alone or in combination with another ingredient. To the other ingredient, the descriptions for the ingredients other than the active ingredient in the explanation of the composition of the present invention can be applied. That is, the composition of the present invention may be used, for example, in combination with an additive such as spreading agent.

The method for applying the composition of the present invention is not particularly limited as long as the desired effect is obtained. The method for applying the composition of the present invention can be selected depending on various conditions, such as the type of pest, type of plant, cultivation method of the plant, growth stage of the plant, and the purpose of use of the composition of the present invention. The composition of the present invention can be applied to a plant, for example, in a usual manner for applying pesticides or fertilizers to plants. The composition of the present invention may be applied to, for example, a plant itself, a medium in which the plant is cultivated, or a combination thereof. The "application of the active ingredient to a plant" is not limited to application of the composition of the present invention to the plant itself, but also includes application of the composition of the present invention to the medium in which the plant is cultivated. The medium in which the plant is cultivated is also referred to as "growth medium" or "growth system". The growth medium can be appropriately selected according to various conditions, such as the type of plant and cultivation method of the plant. The cultivation method of the plant is not particularly limited. Cultivation of the plant can be carried out by, for example, the same method as usual methods for cultivating plants, except that the composition of the present invention is applied. Examples of the cultivation method of a plant include soil cultivation, nutrient solution cultivation, and nutrient solution soil cultivation. Examples of the nutrient solution cultivation include hydroponic cultivation and solid medium cultivation. Examples of the method of the hydroponic cultivation include nutrient film technique (NFT) and deep flow technique (DFT). That is, examples of the medium (growth medium) in which plant cultivation is carried out include soil, hydroponic culture liquid, and solid medium. Examples of the application to a plant itself include spraying or applying the composition to the plant, and immersion of the plant in the composition. The composition of the present invention may be applied to the whole plant or to a part of the plant. For example, if the plant has a plant body, the composition of the present invention may be applied to the entire plant body or to a part of the plant body. For example, the composition of the present invention may be applied to the entire aboveground portion of the plant body. Examples of the part of the plant body include leaf, stem, trunk, root, fruit, and seed. Examples of the part of the plant body include, in particular, leaf. When the composition of the present invention is applied to a leaf, the composition may be applied to only one of the surface and back face of the leaf, or both. Specific examples of the application to a plant body include, for example, foliar spraying and root dipping. Examples of the application to the growth medium include spraying, irrigating, or mixing the composition of the present invention to or with the growth medium. Specifically, the composition of the present invention may be applied (e.g., sprayed) to the growth medium, for example, through an irrigation tube. The application to the growth medium may be carried out so that the active ingredient reaches a position where it can act on the plant. For example, the application to a medium in which a plant is cultivated may be carried out so that the active ingredient reaches the root zone of the plant.

The time of application of the composition of the present invention is not particularly limited as long as the desired effect is obtained. The time of application of the composition of the present invention may be selected according to various conditions, such as the type of pest, type of plant, method of cultivating the plant, growth stage of the plant, and purpose of use of the composition of the present invention. When the composition of the present invention is applied to a growth medium, the plant may or may not be already present in the growth medium. By applying the composition of the present invention to a plant itself, or by applying the composition of the present invention to a growth medium in which a plant is already present, the desired effect may be obtained in the plant. By applying the composition of the present invention to a growth medium in which no plant is present, the desired effect may be obtained in a plant that may be present in the growth medium in the future. Examples of the plant that may be present in the growth medium in the future include a plant that will be transferred from outside to the growth medium in the future and a plant that will be produced in the growth medium in the future. The composition of the present invention may be applied only once, or may be applied two or more times. The composition of the present invention may be applied intermittently or continuously.

The amount of the composition of the present invention to be applied is not particularly limited as long as the desired effect is obtained. The amount of the composition of the present invention to be applied may be appropriately selected according to various conditions such as the type of pest, type of the plant, method for cultivating the plant, growth stage of the plant, purpose of use of the composition of the present invention, and method and time of the application of the composition of the present invention.

The application amount of the composition of the present invention is, for example, in terms of the application amount of the composition of the present invention in the form of liquid (e.g., the composition of the present invention in the form of liquid containing the active ingredient at such a concentration for use as exemplified above), 100 L/hectare or more, 200 L/hectare or more, 500 L/hectare or more, 1,000 L/hectare or more, 1,500 L/ha or more, 2,000 L/ha or more, 3,000 L/ha or more, 4,000 L/ha or more, 5,000 L/ha or more, 7,000 L/ha or more, 10,000 L/ha or more, 30,000 L/ha or more, 50,000 L/ha or more, 70,000 L/ha or more, 100,000 L/hectare or more, 150,000 L/hectare or more, 200,000 L/hectare or more, 300,000 L/hectare or more, 500,000 L/hectare or more, or 750,000 L/hectare or more, and may be 1,000,000 L/hectare or less, 750,000 L/hectare or less, 500,000 L/hectare or less, 300,000 L/hectare or less, 200,000 L/hectare or less, 150,000 L/hectare or less, 100,000 L/hectare or less, 70,000 L/hectare or less, 50,000 L/hectare or less, 30,000 L/hectare or less, 10,000 L/hectare or less, 9,000 L/hectare or less, 8,000 L/hectare or less, 7,000 L/hectare or less, 6,000 L/hectare or less, 5,000 L/hectare or less, 4,000 L/hectare or less, 3,000 L/hectare or less, 2,000 L/hectare or less, or 1500 L/hectare or less, or in a range defined by any non-contradictory combination of these exemplary maximum and minimum amounts. Specifically, the application amount of the composition of the present invention, for example, in terms of the application amount of the composition of the present invention in the form of liquid (e.g., the composition of the present invention in the form of liquid containing the active ingredients at such a concentration for use as exemplified above) may be, for example, 100 to 1,500 L/hectare, 1,500 to 5,000 L/hectare, 5,000 to 10,000 L/hectare, 10,000 to 30,000 L/hectare, 30,000 to 50,000 L/hectare, 50,000 to 100,000 L/hectare, 100,000 to 150,000 L/hectare, 150,000 to 200,000 L/hectare, 200,000 to 500,000 L/hectare, or 500,000 to 10,000,000 L/hectare. Specifically, the application amount of the composition of the present invention, for example, in terms of the application amount of the composition of the present invention in the form of liquid (e.g., the composition of the present invention in the form of liquid containing the active ingredients at such a concentration for use as exemplified above) may be, for example, 100 to 1,000,000 L/hectare, 200 to 1,000,000 L/hectare, 500 to 1,000,000 L/hectare, 1,000 to 750,000 L/hectare, 10,000 to 750,000 L/hectare, or 100,000 to 750,000 L/hectare.

The application amount of the composition of the present invention can be set considering not only the application area (two-dimensional factor), but also a three-dimensional factor. That is, the application amount of the composition of the present invention can be set, for example, according to height of plants to which the composition of the present invention is applied (e.g., sprayed). For plants having a height of from the ground surface to knee height, the application amount of the composition of the present invention in terms of the application amount of the composition of the present invention in the form of liquid (e.g., the composition of the present invention in the form of liquid containing the active ingredient at such a concentration for use as exemplified above) may specifically be, for example, 1,000 to 750,000 L/hectare, 1,000 to 30,000 L/hectare, 1,000 to 5,000 L/hectare, or 1,000 to 1,500 L/hectare. For plants having a height of from knee height to human height, the application amount of the composition of the present invention in terms of the application amount of the composition of the present invention in the form of liquid (e.g., the composition of the present invention in the form of liquid containing the active ingredient at such a concentration for use as exemplified above) may specifically be, for example, 1,500 to 750,000 L/hectare, 1,500 to 70,000 L/hectare, 1,500 to 10,000 L/hectare, or 1,500 to 3,000 L/hectare,. For plants having a height of from human height to 2 meters, the application amount of the composition of the present invention in terms of the application amount of the composition of the present invention in the form of liquid (e.g., the composition of the present invention in the form of liquid containing the active ingredient at such a concentration for use as exemplified above) may specifically be, for example, 3,000 to 750,000 L/hectare, 3,000 to 100,000 L/hectare, 3,000 to 30,000L/hectare, or 3,000 to 5,000 L/hectare. For plants having a height over 2 meters, the application amount of the composition of the present invention in terms of the application amount of the composition of the present invention in the form of liquid (e.g., the composition of the present invention in the form of liquid containing the active ingredient at such a concentration for use as exemplified above) may specifically be, for example, 5,000 to 750,000 L/hectare, 5,000 to 150,000 L/hectare, 5,000 to 30,000 L/hectare, or 5,000 to 7,000 L/hectare.

When the composition of the present invention is applied to a growth medium (e.g., irrigated to the ground surface), the application amount of the composition of the present invention may specifically be, for example, 250,000 to 750,000 L/hectare, in terms of the application amount of the composition of the present invention in the form of liquid (e.g., the composition of the present invention in the form of liquid containing the active ingredient at such a concentration for use as exemplified above).

The composition of the present invention may be applied only once or multiple times as divided portions. For example, the composition of the present invention may be applied two or more times, three or more times, five or more times, or ten or more times, as divided portions. When the composition of the present invention is applied multiple times as divided portions, the "application amount of the composition of the present invention" means the total application amount of the composition of the present invention applied in multiple times of applications.

The application amount of the composition of the present invention can be set, for example, so that the application amount of the active ingredient is within a predetermined range.

The application amount of the active ingredient may be, for example, 1 mmol/hectare or more, 2 mmol/hectare or more, 5 mmol/hectare or more, 10 mmol/hectare or more, 20 mmol/hectare or more, 50 mmol/hectare or more, 100 mmol/hectare or more, 200 mmol/hectare or more, 500 mmol/hectare or more, 1 mol/hectare or more, 2 mol/hectare or more, 5 mol/hectare or more, 10 mol/hectare or more, 20 mol/hectare or more, 50 mol/hectare or more, 100 mol/hectare or more, 200 mol/hectare or more, 500 mol/ha or more, 1,000 mol/ha or more, 2,000 mol/ha or more, 5,000 mol/ha or more, 10,000 mol/ha or more, 20,000 mol/ha or more, 50,000 mol/ha or more, 100,000 mol/ha or more, 150,000 mol/hectare or more, or 200,000mol/hectare or more, and may be 250,000 mol/hectare or less, 200,000 mol/hectare or less, 150,000 mol/hectare or less, 100,000 mol/hectare or less, 50,000 mol/hectare or less, 20,000 mol/hectare or less, 10,000 mol/hectare or less, 5,000 mol/hectare or less, 2,000 mol/hectare or less, 1,000 mol/hectare or less, 500 mol/hectare or less, 200 mol/hectare or less, 100 mol/hectare or less, 50 mol/hectare or less, 20 mol/hectare or less, 10 mol/hectare or less, 5 mol/hectare or less, 2 mol/hectare or less, 1 mol/hectare or less, 500 mmol/hectare or less, 200 mmol/hectare or less, 100 mmol/hectare or less, 50 mmol/hectare or less, 20 mmol/hectare or less, 10 mmol/hectare or less, 5 mmol/hectare or less, or 2 mmol/hectare or less, or in a range defined by any non-contradictory combination of these exemplary maximum and minimum application amounts. The application amount of the active ingredient may specifically be, for example, 1 to 2 mmol/hectare, 2 to 5 mmol/hectare, 5 to 10 mmol/hectare, 10 to 20 mmol/hectare, 20 to 50 mmol/hectare, 50 to 100 mmol/hectare, 100 to 200 mmol/hectare, 200 to 500 mmol/hectare, 500 mmol/hectare to 1 mol/hectare, 1 to 2 mol/hectare, 2 to 5 mol/hectare, 5 to 10 mol/hectare, 10 to 20 mol/hectare, 20 to 50 mol/hectare, 50 to 100 mol/hectare, 100 to 200 mol/hectare, 200 to 500 mol/hectare, 500 to 1,000 mol/hectare, 1,000 to 2,000 mol/hectare, 2,000 to 5,000 mol/hectare, 5,000 to 10,000 mol/hectare, 10,000 to 20,000 mol/hectare, 20,000 to 50,000 mol/hectare, 50,000 to 100,000 mol/hectare, 100,000 to 150,000 mol/hectare, 150,000 to 200,000 mol/hectare, or 200,000 to 250,000 mol/hectare. When the composition of the present invention is applied multiple times as divided portions, the "application amount of the active ingredient" means the total amount of the active ingredient applied by multiple times of applications.

The description concerning the manner of application of the composition of the present invention as mentioned above can be applied to any other cases where the active ingredient is applied to plants. That is, the active ingredient may be applied to plants, for example, at such concentrations for use as exemplified above. The active ingredient may be applied to plants, for example, at such application amounts of the active ingredient as exemplified above. The active ingredient may also be prepared as a composition, such as, for example, a liquid composition containing the active ingredient, and applied to plants. To the composition containing the active ingredient, the descriptions for the composition of the present invention can be applied. The active ingredient can be applied to plants, in particular, in the form of liquid. That is, specifically, the active ingredient may be prepared, for example, as a liquid composition containing the active ingredient at such a concentration for use as exemplified above, and applied to plants. The active ingredient may also be used in combination with another ingredient, such as spreading agent.

By cultivating a plant by using the method of the present invention, a plant (specifically, a plant body) is obtained. Therefore, one embodiment of the method of the present invention may be a method for producing a plant (specifically, a plant body). One embodiment of the method of the present invention may be, more specifically, a method for producing a plant (specifically, a plant body), comprising cultivating a plant with applying the active ingredient (i.e., the ingredient (A) mentioned above) to the plant. Cultivation of a plant can be carried out by, for example, the same method as the usual cultivation methods for plants, except that the active ingredient is applied to the plant. The cultivation method for plants is as described above. The application of the active ingredient and the cultivation of the plant may be carried out, for example, so that the desired effect of the application of the active ingredient can be obtained. For example, the application of the active ingredient may be carried out during the cultivation of the plant, or the cultivation of the plant may be carried out after the application of the active ingredient. The application of the active ingredient may be carried out, in particular, during the cultivation of the plant. Specifically, for example, by cultivating a plant with applying the active ingredient to the plant, the pest damage resistance-inducing effect, pest damage-preventing effect, and/or cultivation performance-improving effect can be obtained, and thus the plant (specifically, plant body) may be efficiently produced. The plant (specifically, plant body) can be harvested as appropriate. That is, the method of the present invention may further comprise harvesting the plant (specifically, plant body). The term "harvest" may be interchangeably used with the term "collect". The expression "harvesting or collecting a plant" may be interchangeably used with the expression "harvesting or collecting a plant body". The plant to be harvested (specifically, plant body) may be the entire plant body or a part of the plant body. Examples of the part of the plant body include leaf, stem, trunk, root, fruit, and seed.

### 4. Use of active ingredient

The present invention also discloses use of the active ingredient for the usages exemplified above. That is, the present invention discloses, for example, use of the active ingredient for inducing pest damage resistance in a plant, preventing pest damage to a plant, and/or improving cultivation performance of a plant, and use of the active ingredient in production of a composition for inducing pest damage resistance in a plant, preventing pest damage to a plant, and/or improving cultivation performance of a plant.

The present invention also discloses the active ingredient for use in the usages exemplified above. That is, the present invention discloses the active ingredient for use in inducing pest damage resistance in a plant, preventing pest damage to a plant, and/or improving cultivation performance of a plant, or the active ingredient for use in production of a composition for inducing pest damage resistance in a plant, preventing pest damage to a plant, and/or improving cultivation performance of a plant.

### EXAMPLES

Hereafter, the present invention will be more specifically explained with reference to the following non-limiting examples.

### Synthesis Examples

Compounds 1-58 and 67-75 were obtained by the following methods. The structures of Compounds 1-58 and 67-75 are shown by the following formulas of Compounds 1-58 and Compounds 67-75, respectively.

### Synthesis Example 1. Synthesis of Compound 1

### Step 1. Synthesis of intermediate of Compound 1

To a solution of N-Cbz-glutamic acid α-benzyl ester (361 mg, 0.97 mmol) in acetonitrile (5 mL), HOAt (135 mg, 0.99 mmol) and WSC-HCl (191 mg, 1.00 mmol) were added, and the mixture was stirred at room temperature for 1 hour. To the reaction mixture, isopropanol (0.5 mL) and triethylamine (0.05 mL, 0.36 mmol) were added, and the mixture was stirred at room temperature for further 18 hours. Ethyl acetate was added to the reaction mixture, and the organic layer was washed by partitioning successively with a 10% aqueous citric acid solution and a saturated aqueous sodium hydrogencarbonate solution. The organic layer was dehydrated, dried over anhydrous sodium sulfate, and then concentrated to dryness under reduced pressure to obtain an intermediate of Compound 1.
Yield 80%; ESI MS m/z 414.1 (M+H)⁺; ¹H NMR (400 MHz, CD₃OD) d 7.38-7.32 (10H, m), 5.21 (1H, d, J=12.4 Hz), 5.16 (1H, d, J=12.4 Hz), 5.10 (2H, s), 4.96 (1H, m), 4.30 (1H, dd, J=4.8, 9.2 Hz), 2.40-2.36 (2H, m), 2.17 (1H, m), 1.95 (6H, m), 1.22 (6H, d, J=6.4 Hz).

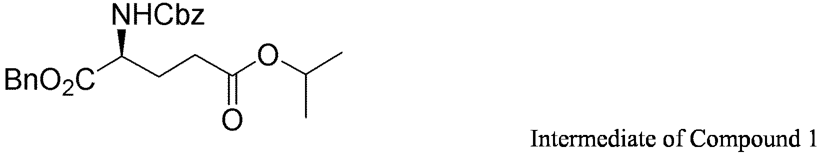

### Step 2. Synthesis of Compound 1

To the intermediate of Compound 1 (319 mg, 0.77 mmol), ethyl acetate (10 mL), water (10 mL) and acetic acid (0.1 mL) were added, then 5% palladium/carbon (43 mg) was added, and the mixture was stirred at room temperature for 20 hours under a hydrogen atmosphere. The reaction mixture was filtered, and the aqueous layer was concentrated to dryness under reduced pressure to obtain Compound 1. Yield 96%; ESI MS m/z 190.0 (M+H)⁺; ¹H NMR (400 MHz, D₂O) d 4.92 (1H, m), 3.69 (1H, t, J=6.4 Hz), 2.47-2.42 (2H, m), 2.10-2.03 (2H, m), 1.16 (6H, t, J=6.4 Hz).

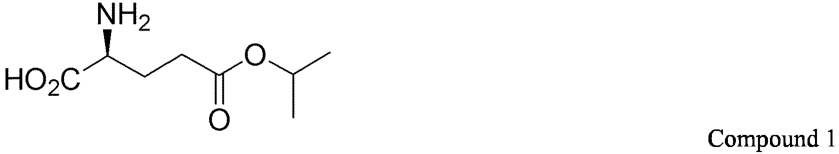

### Synthesis Example 2. Synthesis of Compound 2

### Step 1. Synthesis of intermediate of Compound 2

An intermediate of Compound 2 was obtained in the same manner as that used for the production of the intermediate of Compound 1 by using N-Cbz-glutamic acid α-benzyl ester and isobutanol as the starting materials.
Yield 89%; ESI MS m/z 428.2 (M+H)⁺; ¹H NMR (400 MHz, CD₃OD) d 7.38-7.30 (10H, m), 5.21 (1H, d, J=12.0 Hz), 5.16 (1H, d, J=12.0 Hz), 5.12 (1H, d, J=12.4 Hz), 5.09 (1H, d, J=12.4 Hz), 4.31 (1H, dd, J=5.2, 9.6 Hz), 3.86 (2H, d, J=6.8 Hz), 2.46-2.42 (2H, m), 2.20 (1H, m), 1.96-1.89 (2H, m), 0.93 (6H, d, J=6.8 Hz).

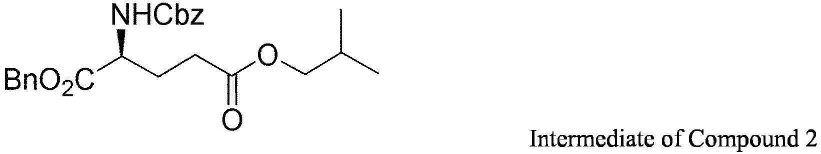

### Step 2. Synthesis of Compound 2

Compound 2 was obtained in the same manner as that used for the production of Compound 1 by using the intermediate of Compound 2 as the starting material. Yield 85%; ESI MS m/z 204.1 (M+H)⁺; ¹H NMR (400 MHz, D₂O) d 3.85 (2H, d, J=6.4 Hz), 3.71 (1H, t, J=6.4 Hz), 2.53-2.49 (2H, m), 2.12-2.06 (2H, m), 1.87 (1H, m), 0.84 (6H, d, J=6.8 Hz).

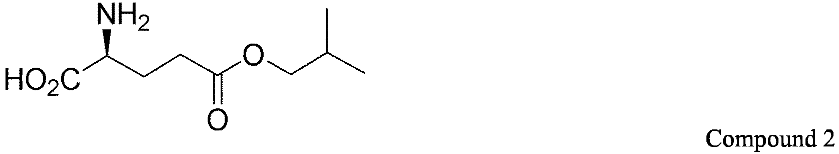

### Synthesis Example 3. Synthesis of Compound 3

### Step 1. Synthesis of intermediate of Compound 3

An intermediate of Compound 3 was obtained in the same manner as that used for the production of the intermediate of Compound 1 by using N-Cbz-glutamic acid α-benzyl ester and cyclopropyl alcohol as the starting materials.
Yield 78%; ESI MS m/z 412.1 (M+H)⁺; ¹H NMR (400 MHz, CD₃OD) d 7.38-7.30 (10H, m), 5.21 (1H, d, J=12.8 Hz), 5.16 (1H, d, J=12.8 Hz), 5.11 (2H, s), 4.29 (1H, dd, J=4.8, 9.2 Hz), 4.08 (1H, m), 2.41-2.37 (2H, m), 2.17 (1H, m), 1.94 (1H, m), 0.70-0.64 (4H, m).

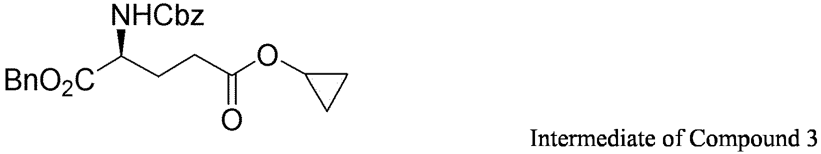

### Step 2. Synthesis of Compound 3

Compound 3 was obtained in the same manner as that used for the production of Compound 1 by using the intermediate of Compound 3 as the starting material. Yield 56%; ESI MS m/z 188.1 (M+H)⁺; ¹H NMR (400 MHz, D₂O) d 3.98 (1H, m), 3.67 (1H, t, J=6.4 Hz), 2.50-2.41 (2H, m), 2.08-2.02 (2H, m), 0.66-0.65 (4H, m).

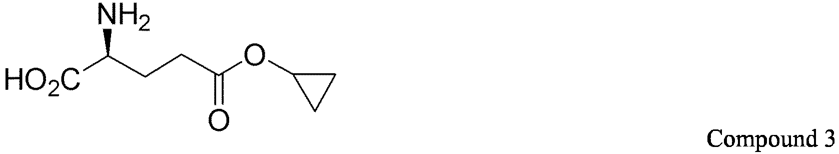

### Synthesis Example 4. Synthesis of Compound 4

### Step 1. Synthesis of intermediate of Compound 4

An intermediate of Compound 4 was obtained in the same manner as that used for the production of the intermediate of Compound 1 by using N-Cbz-glutamic acid α-benzyl ester and 3-pentanol as the starting materials.
Yield 95%; ESI MS m/z 442.2 (M+H)⁺; ¹H NMR (400 MHz, CD₃OD) d 7.36-7.30 (10H, m), 5.21-5.09 (4H, m), 4.74 (1H, m), 4.33 (1H, dd, J=5.2, 9.2 Hz), 2.44-2.40 (2H, m), 2.18 (1H, m), 1.95 (1H, m), 1.58-1.50 (4H, m), 0.86 (6H, t, J=7.6 Hz).

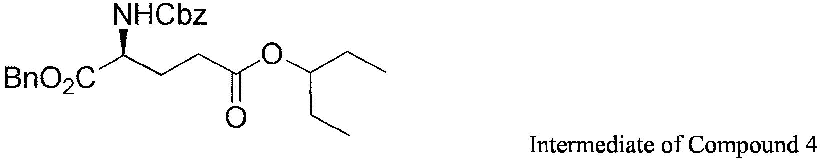

### Step 2. Synthesis of Compound 4

Compound 4 was obtained in the same manner as that used for the production of Compound 1 by using the intermediate of Compound 4 as the starting material. Yield 89%; ESI MS m/z 218.1 (M+H)⁺; ¹H NMR (400 MHz, D₂O) d 3.69 (1H, t, J=6.4 Hz), 2.53-2.48 (2H, m), 2.11-2.04 (2H, m), 1.58-1.44 (4H, m), 0.77 (6H, t, J=7.6 Hz).

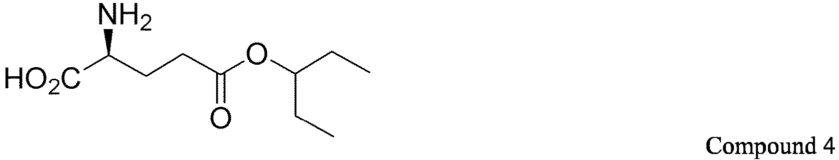

### Synthesis Example 5. Synthesis of Compound 5

### Step 1. Synthesis of intermediate of Compound 5

An intermediate of Compound 5 was obtained in the same manner as that used for the production of the intermediate of Compound 1 by using N-Cbz-glutamic acid α-benzyl ester and cyclobutanol as the starting materials.
Yield 95%; ESI MS m/z 426.2 (M+H)⁺; ¹H NMR (400 MHz, CD₃OD) d 7.37-7.30 (10H, m), 5.19 (1H, d, J=12.4 Hz), 5.15 (1H, d, J=12.4 Hz), 5.10 (2H, s), 4.30 (1H, dd, J=5.2, 9.2 Hz), 2.40-2.36 (2H, m), 2.33-2.27 (2H, m), 2.16 (1H, m), 2.09-1.88 (3H, m), 1.77 (1H, m), 1.63 (1H, m).

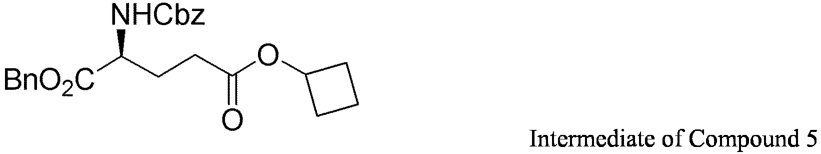

### Step 2. Synthesis of Compound 5

Compound 5 was obtained in the same manner as that used for the production of Compound 1 by using the intermediate Compound 5 as the starting material. Yield 55%; ESI MS m/z 202.1 (M+H)⁺; ¹H NMR (400 MHz, D₂O) d 4.87 (1H, m), 3.67 (1H, t, J=6.4 Hz), 2.47-2.43 (2H, m), 2.24-2.20 (2H, m), 2.08-1.98 (4H, m), 1.71 (1H, m), 1.54 (1H, m).

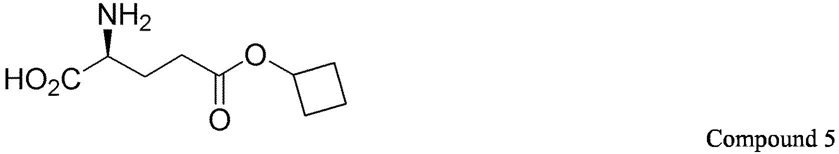

### Synthesis Example 6. Synthesis of Compound 6

### Step 1. Synthesis of intermediate of Compound 6

An intermediate of Compound 6 was obtained in the same manner as that used for the production of the intermediate of Compound 1 by using N-Boc-glutamic acid α-t-butyl ester and isopropylamine as the starting materials.
Yield 88%; ESI MS m/z 345.2 (M+H)⁺; ¹H NMR (400 MHz, CD₃OD) d 3.88-3.82 (2H, m), 2.13 (2H, t, J=7.6 Hz), 1.94 (1H, m), 1.76 (1H, m), 1.37 (9H, s), 1.34 (9H, s), 1.03 (3H, d, J=6.4 Hz), 1.02 (3H, d, J=6.4 Hz).

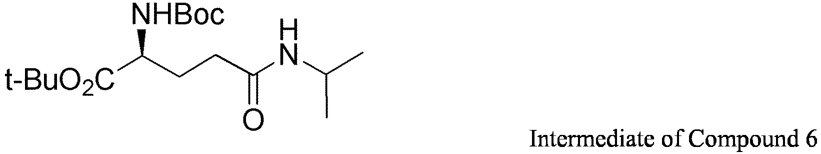

### Step 2. Synthesis of Compound 6

The intermediate of Compound 6 (94 mg, 0.27 mmol) was dissolved in a 4 N hydrochloric acid solution in dioxane (2 mL), and the solution was stirred at room temperature for 18 hours. The reaction solution was concentrated to dryness under reduced pressure to obtain hydrochloride of Compound 6.
Yield 99%; ESI MS m/z 189.1 (M+H)⁺; ¹H NMR (400 MHz, D₂O) d 3.99 (1H, t, J=6.4 Hz), 3.82 (1H, dd, J=6.8, 9.2 Hz), 2.73-2.32 (2H, m), 2.15-2.09 (2H, m), 1.03 (6H, d, J=6.8 Hz).

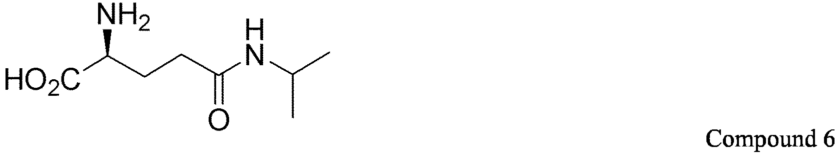

### Synthesis Example 7. Synthesis of Compound 7

### Step 1. Synthesis of intermediate of Compound 7

An intermediate of Compound 7 was obtained in the same manner as that used for the production of the intermediate of Compound 1 by using N-Boc-glutamic acid α-t-butyl ester and cyclopropylamine as the starting materials.
Yield 72%; ESI MS m/z 343.2 (M+H)⁺; ¹H NMR (400 MHz, CD₃OD) d 3.85 (1H, m), 2.53 (1H, m), 2.12 (2H, t, J=7.6 Hz), 1.96 (1H, m), 1.73 (1H, m), 1.37 (9H, s), 1.34 (9H, s), 0.61-0.59 (2H, m), 0.38-0.36 (2H, m).

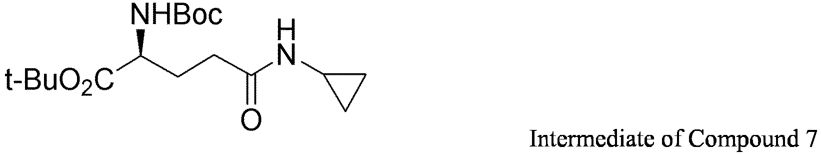

### Step 2. Synthesis of Compound 7

Hydrochloride of Compound 7 was obtained in the same manner as that used for the production of Compound 6 by using the intermediate of Compound 7 as the starting material.
Yield 99%; ESI MS m/z 187.0 (M+H)⁺; ¹H NMR (400 MHz, D₂O) d 3.80 (1H, t, J=6.4 Hz), 2.27 (1H, m), 2.16-2.11 (2H, m), 1.94-1.87 (2H, m), 0.44-0.41 (2H, m), 0.23-0.20 (2H, m).

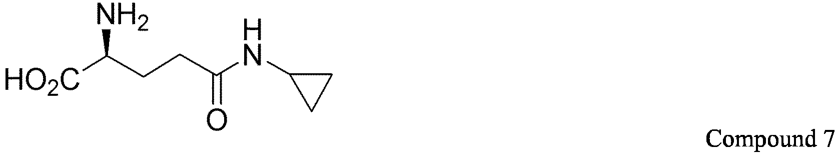

### Synthesis Example 8. Synthesis of Compound 8

### Step 1. Synthesis of intermediate of Compound 8

An intermediate of Compound 8 was obtained in the same manner as that used for the production of the intermediate of Compound 1 by using N-Boc-glutamic acid α-t-butyl ester and dimethylamine hydrochloride as the starting materials.
Yield 92%; ESI MS m/z 331.2 (M+H)⁺; ¹H NMR (400 MHz, CD₃OD) d 4.02 (1H, m), 3.07 (3H, s), 2.95 (3H, s), 2.51-2.45 (2H, m), 2.10 (1H, m), 1.89 (1H, m), 1.49 (9H, s), 1.46 (9H, s).

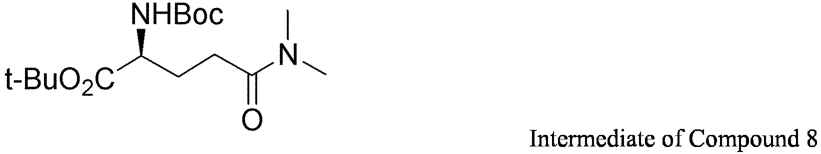

### Step 2. Synthesis of Compound 8 (known)

Hydrochloride of Compound 8 was obtained in the same manner as that used for the production of Compound 6 by using the intermediate of Compound 8 as the starting material.
Yield 99%; ESI MS m/z 175.1 (M+H)⁺; ¹H NMR (400 MHz, D₂O) d 4.02 (1H, t, J=6.4 Hz), 2.93 (3H, s), 2.80 (3H, s), 2.56-2.52 (2H, m), 2.14-2.05 (2H, m).

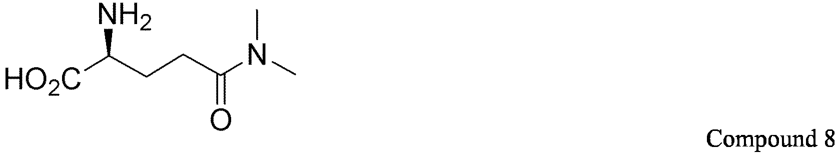

### Synthesis Example 9. Synthesis of Compound 9

### Step 1. Synthesis of intermediate of Compound 9

An intermediate of Compound 9 was obtained in the same manner as that used for the production of the intermediate of Compound 1 by using N-Cbz-glutamic acid α-benzyl ester and n-propanol as the starting materials.
Yield 93%; ESI MS m/z 414.1 (M+H)⁺; ¹H NMR (400 MHz, CD₃OD) d 7.38-7.30 (10H, m), 5.20 (1H, d, J=12.4 Hz), 5.16 (1H, d, J=12.4 Hz), 5.10 (2H, s), 4.31 (1H, dd, J=5.2, 9.2 Hz), 4.02 (2H, t, J=6.8 Hz), 2.44-2.40 (2H, m), 2.18 (1H, m), 1.96 (1H, m), 1.68-1.59 (2H, m), 0.94 (3H, t, J=7.2 Hz).

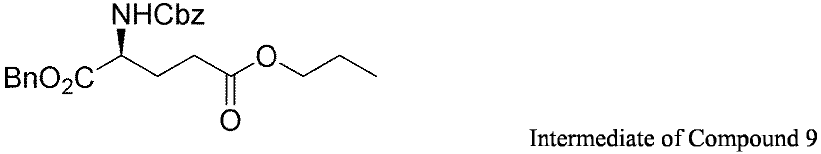

### Step 2. Synthesis of Compound 9

Compound 9 was obtained in the same manner as that used for the production of Compound 1 by using the intermediate of Compound 9 as the starting material. Yield 88%; ESI MS m/z 190.1 (M+H)⁺; ¹H NMR (400 MHz, D₂O) d 3.99 (2H, t, J=6.8 Hz), 3.66 (1H, t, J=6.4 Hz), 2.50-2.45 (2H, m), 2.09-2.02 (2H, m), 1.59-1.54 (2H, m), 0.82 (3H, t, J=7.2 Hz).

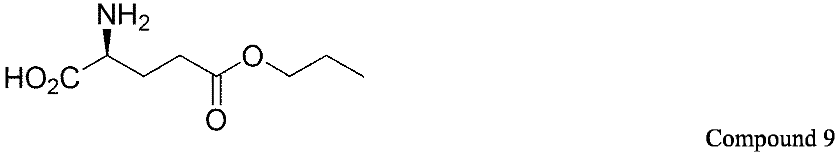

### Synthesis Example 10. Synthesis of Compound 10

### Step 1. Synthesis of intermediate of Compound 10

An intermediate of Compound 10 was obtained in the same manner as that used for the production of the intermediate of Compound 1 by using N-Cbz-glutamic acid α-benzyl ester and ethanolamine as the starting materials.
Yield 92%; ESI MS m/z 415.1 (M+H)⁺; ¹H NMR (400 MHz, CD₃OD) d 7.37-7.30 (10H, m), 5.20-5.06 (4H, m), 4.27 (1H, dd, J=4.8, 9.6 Hz), 3.58 (2H, t, J=5.6 Hz), 3.30-3.25 (2H, m), 2.32 (2H, t, J=7.6 Hz), 2.20 (1H, m), 1.95 (1H, m).

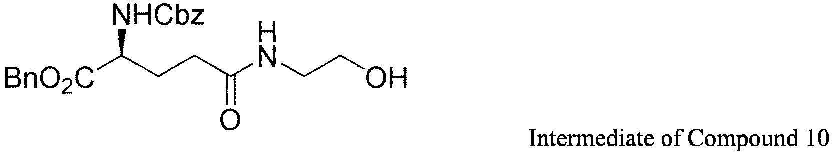

### Step 2. Synthesis of Compound 10

Compound 10 was obtained in the same manner as that used for the production of Compound 1 by using the intermediate of Compound 10 as the starting material.
Yield 83%; ESI MS m/z 191.0 (M+H)⁺; ¹H NMR (400 MHz, D₂O) d 3.67 (1H, t, J=6.4 Hz), 3.56 (2H, t, J=5.6 Hz), 3.24 (2H, t, J=5.6 Hz), 2.37-2.30 (2H, m), 2.07-2.02 (2H, m).

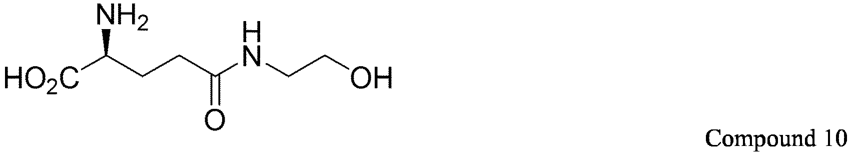

### Synthesis Example 11. Synthesis of Compound 11

### Step 1. Synthesis of intermediate of Compound 11

To a solution of N^{α}-Boc-2,4-diaminobutyric acid t-butyl ester hydrochloride (311 mg, 1.00 mmol) in acetonitrile (4 mL), triethylamine (0.4 mL, 2.87 mmol) was added under ice cooling, and the mixture was stirred for 5 minutes. Methanesulfonyl chloride (0.075 mL, 0.97 mmol) was added to the reaction mixture, and the resulting mixture was further stirred at the same temperature for 1 hour. Ethyl acetate was added to the reaction mixture, and the organic layer was washed by partitioning with 0.1 N hydrochloric acid. The organic layer was dehydrated, dried over anhydrous sodium sulfate, and then concentrated to dryness under reduced pressure to obtain an intermediate of Compound 11.
Yield 90%; ESI MS m/z 375.1 (M+Na)⁺; ¹H NMR (400 MHz, CD₃OD) d 4.10 (1H, dd, J=4.8, 9.2 Hz), 3.21-3.09 (2H, m), 2.95 (3H, s), 2.03 (1H, m), 1.84 (1H, m), 1.49 (9H, s), 1.47 (9H, s).

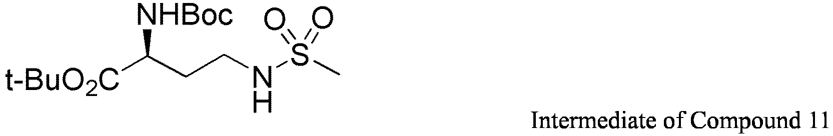

### Step 2. Synthesis of Compound 11

The intermediate of Compound 11 (310 mg, 0.88 mmol) was dissolved in a 4 N hydrochloric acid solution in dioxane (2 mL), and the solution was stirred at room temperature for 18 hours. The reaction solution was concentrated to dryness under reduced pressure to obtain hydrochloride of Compound 11.
Yield 99%; ESI MS m/z 197.0 (M+H)⁺; ¹H NMR (400 MHz, D₂O) d 4.09 (1H, t, J=6.4 Hz), 3.22 (2H, t, J=6.8 Hz), 2.97 (3H, s), 2.14 (1H, m), 2.04 (1H, m).

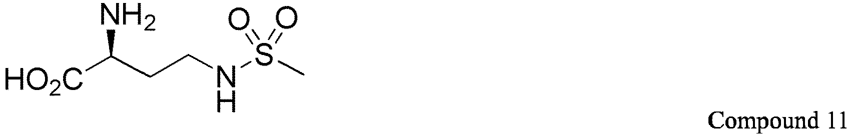

### Synthesis Example 12. Synthesis of Compound 12

### Step 1. Synthesis of intermediate of Compound 12

An intermediate of Compound 12 was obtained in the same manner as that used for the production of intermediate of Compound 11 by using N^{α}-Boc-ornithine t-butyl ester and methanesulfonyl chloride as the starting materials.
Yield 89%; ESI MS m/z 389.1 (M+Na)⁺; ¹H NMR (400 MHz, CD₃OD) d 3.99 (1H, dd, J=4.8, 8.0 Hz), 3.09 (2H, t, J=6.4 Hz), 2.95 (3H, s), 1.85 (1H, m), 1.70-1.61 (3H, m), 1.49 (9H, s), 1.47 (9H, s).

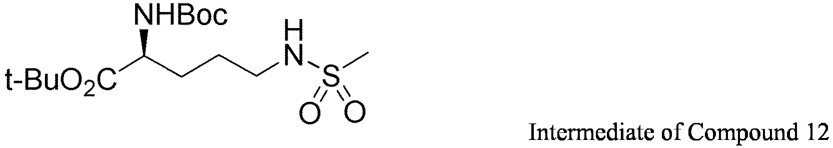

### Step 2. Synthesis of Compound 12

Hydrochloride of Compound 12 was obtained in the same manner as that used for the production of Compound 11 by using the intermediate of Compound 12 as the starting material.
Yield 99%; ESI MS m/z 211.0 (M+H)⁺; ¹H NMR (400 MHz, D₂O) d 4.02 (1H, t, J=6.4 Hz), 3.05 (2H, t, J=6.8 Hz), 2.96 (3H, s), 1.96-1.85 (2H, m), 1.66-1.52 (2H, m).

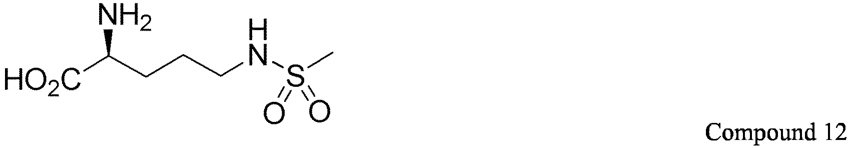

### Synthesis Example 13. Synthesis of Compound 13

### Step 1. Synthesis of intermediate of Compound 13

An intermediate of Compound 13 was obtained in the same manner as that used for the production of the intermediate of Compound 1 by using N-Cbz-serine t-butyl ester and isovaleric acid as the starting materials.
Yield 55%; ESI MS m/z 402.2 (M+Na)⁺; ¹H NMR (400 MHz, CD₃OD) d 7.40-7.31 (5H, m), 5.14 (1H, d, J=12.8 Hz), 5.11 (1H, d, J=12.8 Hz), 4.42 (1H, t, J=4.8 Hz), 4.36 (2H, d, J=4.8 Hz), 2.21 (2H, d, J=7.2 Hz), 2.05 (1H, m), 1.48 (6H, s), 1.43 (3H, brs), 0.95 (6H, d, J=6.8 Hz).

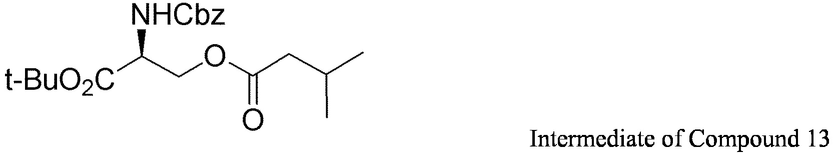

### Step 2. Synthesis of Compound 13

The intermediate of Compound 13 (205 mg, 0.54 mmol) was dissolved in a 4 N hydrochloric acid solution in dioxane (3 mL), and the solution was stirred at room temperature for 18 hours. The reaction solution was concentrated under reduced pressure to dryness, and the resulting residue was dissolved in ethyl acetate/water (1:1) (20 mL), 5% palladium carbon (32 mg) was added, and the mixture was stirred at room temperature for 18 hours under a hydrogen atmosphere. The reaction mixture was filtered, and then the aqueous layer of the filtrate was concentrated to dryness under reduced pressure to obtain Compound 13.
Yield 46%; ESI MS m/z 190.1 (M+H)⁺; ¹H NMR (400 MHz, D₂O) d 4.42 (1H, dd, J=4.0, 5.2 Hz), 3.87 (1H, dd, J=5.2, 12.0 Hz), 3.80 (1H, dd, J=4.0, 12.0 Hz), 2.12 (2H, d, J=6.8 Hz), 1.94 (1H, m), 0.86 (3H, d, J=6.8 Hz), 0.85 (3H, d, J=6.8 Hz).

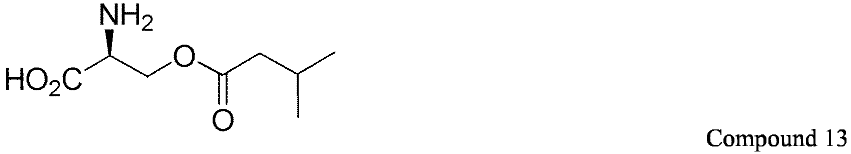

### Synthesis Example 14. Synthesis of Compound 14

### Step 1. Synthesis of intermediate of Compound 14

An intermediate of Compound 14 was obtained in the same manner as that used for the production of the intermediate of Compound 1 by using N-Cbz-serine t-butyl ester and 3-hydroxybutyric acid as the starting materials.
Yield 40%; ESI MS m/z 404.1 (M+Na)⁺; ¹H NMR (400 MHz, CD₃OD) d 7.40-7.31 (5H, m), 5.13 (2H, s), 4.44-4.35 (2H, m), 4.16 (1H, dd, J=6.4, 12.8 Hz), 2.46-2.44 (2H, m), 1.48 (6H, s), 1.43 (3H, brs), 1.22-1.20 (3H, m).

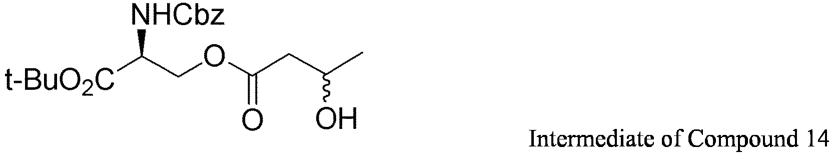

### Step 2. Synthesis of Compound 14

Compound 14 was obtained in the same manner as that used for the production of Compound 13 by using the intermediate of Compound 14 as the starting material.
Yield 57%; ESI MS m/z 192.0 (M+H)⁺; ¹H NMR (400 MHz, D₂O) d 4.52-4.43 (2H, m), 4.17 (1H, m), 4.05 (1H, dd, J=3.2, 4.4 Hz), 2.59-2.43 (2H, m), 1.15 (6H, d, J=6.4 Hz).

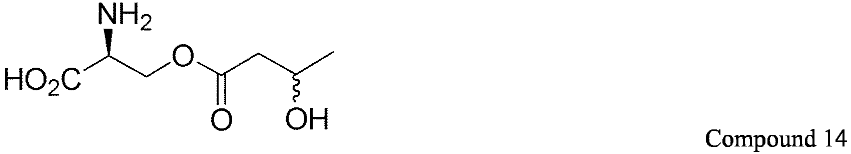

### Synthesis Example 15. Synthesis of Compound 15

### Step 1. Synthesis of intermediate of Compound 15

To a solution of N-Cbz-serine benzyl ester (331 mg, 1.01 mmol) in THF (2 mL), triethylamine (0.2 mL, 1.43 mmol), and then isopropyl chloroformate (0.15 mL, 1.31 mmol) were added under ice cooling, and the mixture was stirred at the same temperature for 0.5 hour. Ethyl acetate was added to the reaction mixture, and the organic layer was washed by partitioning with 0.1 N hydrochloric acid. The organic layer was dehydrated, dried over anhydrous sodium sulfate, and then concentrated to dryness under reduced pressure. The residue was purified by reverse phase HPLC (water-acetonitrile) to obtain an intermediate of Compound 15.
Yield 53%; ESI MS m/z 416.1 (M+H)⁺; ¹H NMR (400 MHz, CD₃OD) d 7.38-7.30 (10H, m), 5.23 (1H, d, J=12.4 Hz), 5.18 (1H, d, J=12.4 Hz), 5.12 (2H, s), 4.81 (1H, m), 4.62 (1H, t, J=5.2 Hz), 4.44 (2H, d, J=5.2 Hz), 1.26 (3H, d, J=6.4 Hz), 1.25 (3H, d, J=6.0 Hz).

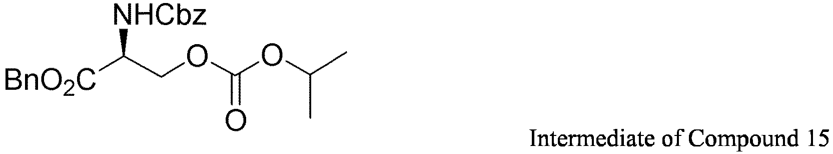

### Step 2. Synthesis of Compound 15

Compound 15 was obtained in the same manner as that used for the production of Compound 2 by using the intermediate of Compound 15 as the starting material.
Yield 50%; ESI MS m/z 192.0 (M+H)⁺; ¹H NMR (400 MHz, D₂O) d 4.81 (1H, m), 4.49-4.43 (2H, m), 3.98 (1H, dd, J=4.0, 4.8 Hz), 1.21 (6H, d, J=6.4 Hz).

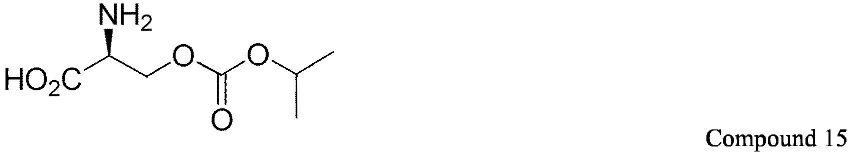

### Synthesis Example 16. Synthesis of Compound 67

### Step 1. Synthesis of Intermediate 1 of Compound 67

Intermediate 1 of Compound 67 was synthesized in the same manner as that used for the production of the intermediate of Compound 1 by using Cbz-glutamic acid α-t-butyl ester and valine t-butyl ester as the starting materials.
Yield 98%; ESI MS m/z 493.3 (M+H)⁺; ¹H NMR (400 MHz, CD₃OD) δ 7.40-7.31 (5H, m), 5.13 (1H, d, J=12.4 Hz), 5.09 (1H, d, J=12.4 Hz), 4.21 (1H, d, J=5.6 Hz), 4.09 (1H, dd, J=4.4, 10.0 Hz), 2.40 (2H, t, J=7.6 Hz), 2.18-2.10 (2H, m), 1.90 (1H, m), 1.48 (9H, s), 1.47 (9H, s), 0.87 (3H, d, J=6.8 Hz), 0.86 (3H, d, J=6.8 Hz).

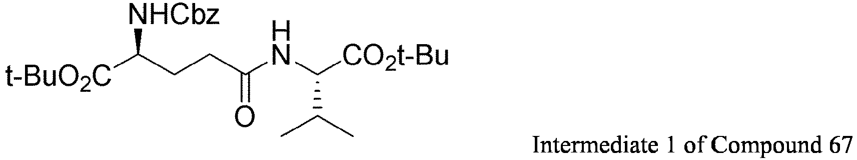

### Step 2. Synthesis of Intermediate 2 of Compound 67

Intermediate 2 of Compound 67 was obtained in the same manner as that used for the production of Compound 1 by using Intermediate 1 of Compound 67 as the starting material.
Yield 98%; ESI MS m/z 359.2 M+H)⁺; ¹H NMR (400 MHz, CD₃OD) δ 4.21 (1H, d, J=5.6 Hz), 3.37 (1H, dd, J=5.2, 7.2 Hz), 2.41-2.37 (2H, m), 2.14 (1H, m), 2.03 (1H, m), 1.85 (1H, m), 1.50 (9H, s), 1.49 (9H, s), 0.98 (6H, d, J=6.8 Hz).

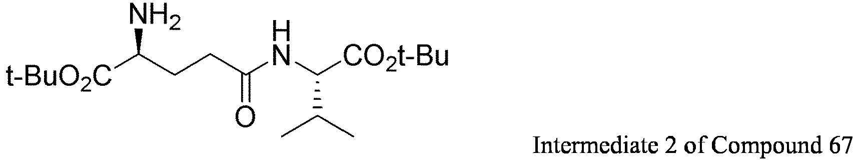

### Step 3. Synthesis of Intermediate 3 of Compound 67

Intermediate 3 of Compound 67 was obtained in the same manner as that used for the production of intermediate of Compound 1 by using Boc-glutamic acid α-t-butyl ester and Intermediate 2 of Compound 67 as the starting materials.
Yield 90%; ESI MS m/z 644.5 (M+H)⁺; ¹H NMR (400 MHz, CD₃OD) δ 4.30-4.20 (2H, m), 4.01 (1H, m), 2.40-2.35 (4H, m), 2.21-2.12 (4H, m), 1.86 (1H, m), 1.488 (18H, s), 1.485 (18H, s), 0.99 (3H, d, J=6.8 Hz), 0.98 (3H, d, J=6.8 Hz).

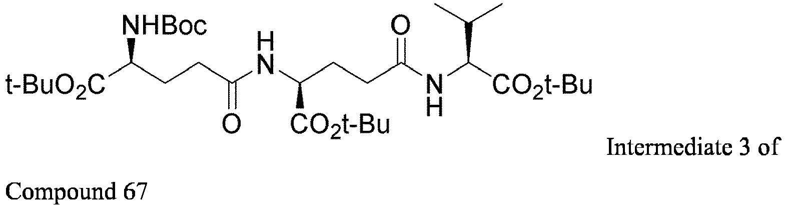

### Step 4. Synthesis of Compound 67

Compound 67 was obtained in the same manner as that used for the production of Compound 11 by using Intermediate 3 of Compound 67.
Yield 99%; ESI MS m/z 376.1 (M+H)⁺; ¹H NMR (400 MHz, D₂O) δ 4.25 (1H, dd, J=4.8, 9.2 Hz), 4.16 (1H, d, J=6.0 Hz), 4.01 (1H, t, J=6.4 Hz), 2.50-2.46 (2H, m), 2.39-2.35 (2H, m), 2.17-2.06 (4H, m), 1.92 (1H, m), 0.86 (3H, d, J=6.8 Hz), 0.85 (3H, d, J=6.8 Hz).

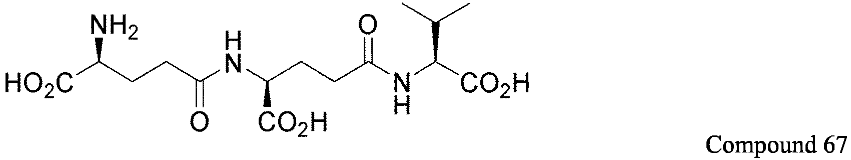

### Synthesis Example 17. Synthesis of Compound 68

### Step 1. Synthesis of Intermediate 1 of Compound 68

Intermediate 1 of Compound 68 was obtained in the same manner as that used for the production of the intermediate of Compound 1 by using Cbz-valine and valine t-butyl ester as the starting materials.
Yield 96%; ESI MS m/z 429.2 (M+Na)⁺; ¹H NMR (400 MHz, CD₃OD) δ 7.38-7.29 (5H, m), 5.11 (2H, s), 4.24 (1H, d, J=6.0 Hz), 4.05 (1H, d, J=7.2 Hz), 2.16-2.03 (2H, m), 1.00-0.96 (12H, m).

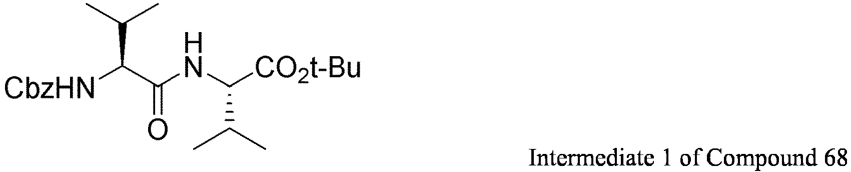

### Step 2. Synthesis of Intermediate 2 of Compound 68

Intermediate 2 of Compound 68 was obtained in the same manner as that used for the production of Compound 1 by using Intermediate 1 of Compound 68 as the starting material.
Yield 93%; ESI MS m/z 273.1 (M+H)⁺; ¹H NMR (400 MHz, CD₃OD) δ 4.22 (1H, d, J=5.6 Hz), 3.22 (1H, d, J=5.2 Hz), 2.15 (1H, m), 2.01 (1H, m), 1.49 (9H, s), 1.02-0.93 (12H, m).

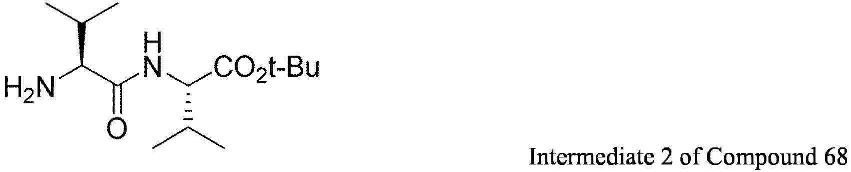

### Step 3. Synthesis of Intermediate 3 of Compound 68

Intermediate 3 of Compound 68 was obtained in the same manner as that used for the production of Compound 1 by using Boc-glutamic acid α-t-butyl ester and Intermediate 2 of Compound 68 as the starting materials.
Yield 90%; ESI MS m/z 558.5 (M+H)⁺; ¹H NMR (400 MHz, CD₃OD) δ 4.25-4.19 (2H, m), 3.99 (1H, dd, J=4.8, 9.6 Hz), 2.39-2.35 (2H, m), 2.16-2.05 (2H, m), 1.85 (1H, m), 1.49 (18H, s), 1.46 (9H, s), 1.01-0.96 (12H, m).

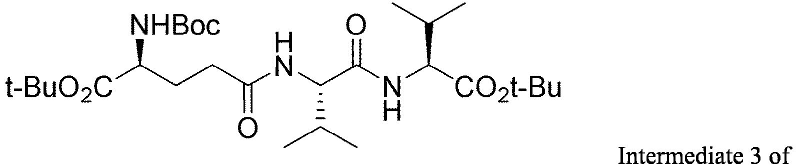

### Compound 68

### Step 4. Synthesis of Compound 68

Compound 68 was obtained in the same manner as that used for the production of Compound 11 by using Intermediate 3 of Compound 68 as the starting material.
Yield 99%; ESI MS m/z 346.1 (M+H)⁺; ¹H NMR (400 MHz, D₂O) δ 4.17 (1H, d, J=6.4 Hz), 4.04 (1H, d, J=7.6 Hz), 3.96 (1H, t, J=6.4 Hz), 2.51-2.44 (2H, m), 2.14-2.09 (2H, m), 1.97 (1H, m), 0.88 (3H, d, J=6.8 Hz), 0.87 (3H, d, J=6.4 Hz).

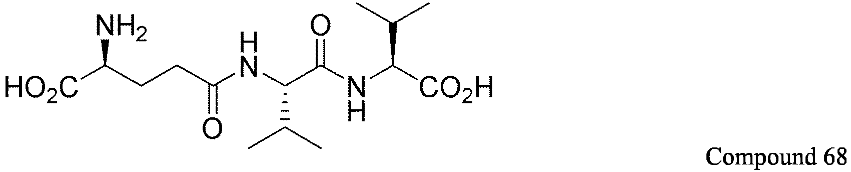

### Other compounds. Acquisition of Compounds 16 to 58 and 69 to 75

Compounds 16-58 and 69-75 mentioned below were obtained by purchasing or other means as appropriate.

That is, Compounds 16, 21, 22, 23, 25, 28, 30, 32, 33, 34, 54, and 56 were purchased from BACHEM. Compounds 38, 46, and 52 were purchased from Sigma. Compounds 44 and 45 were purchased from Peptide Institute. Compounds 17, 27, 31, and 48 were purchased from Kokusan Chemical. Compound 51 was purchased from Watanabe Chemical. Compounds 18, 19, 35, 37, 41, 42, 43, 47, 49, 50, 53, 55, 57, and 58 were purchased from Tokyo Kasei Kogyo. Compound 36 was purchased from FUJIFILM Wako Pure Chemicals. Compound 70 was purchased from Nacalai Tesque. Compounds 71 to 75 were obtained through contract synthesis at Watanabe Chemical.

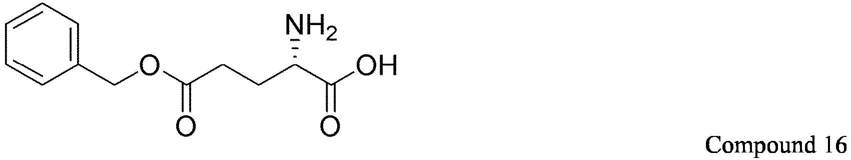

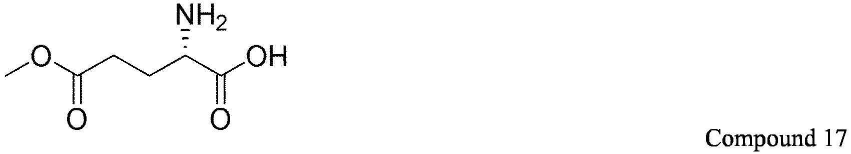

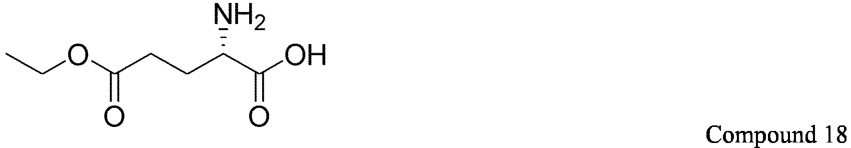

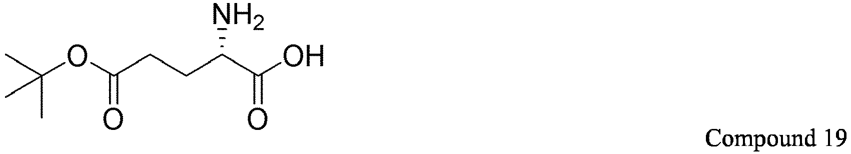

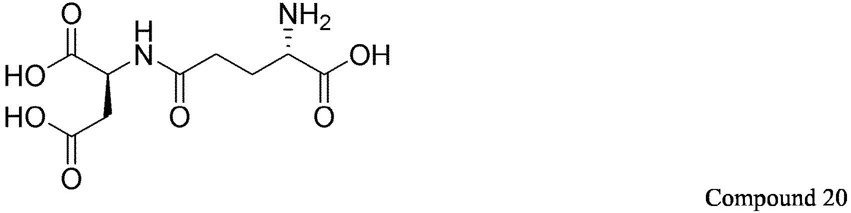

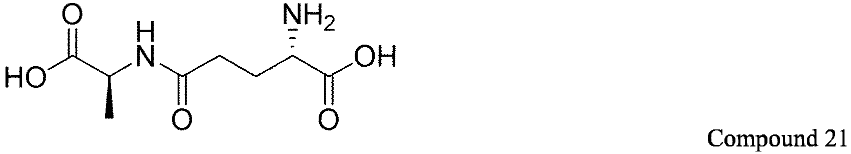

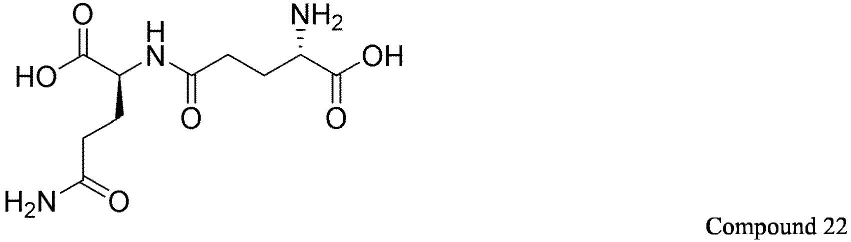

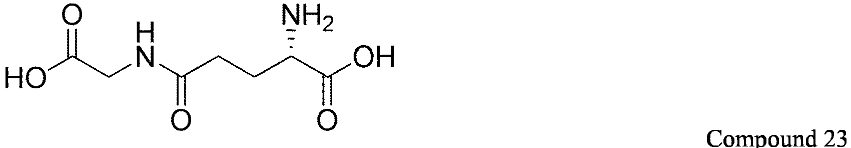

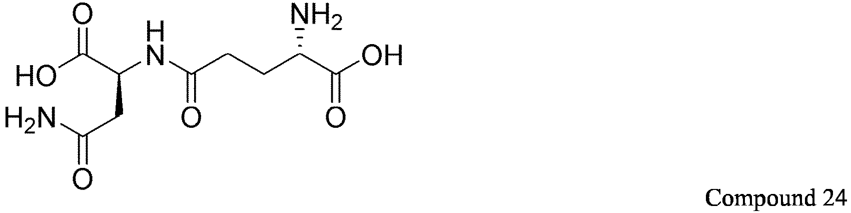

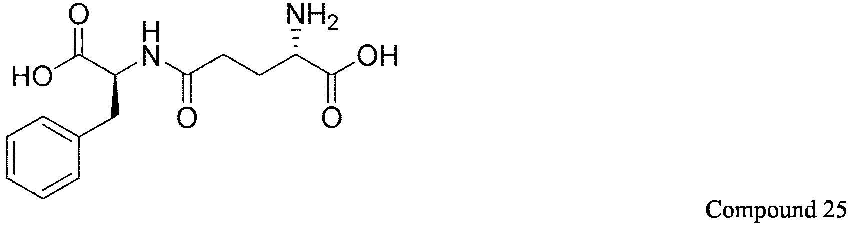

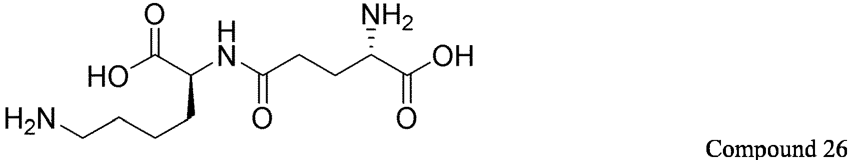

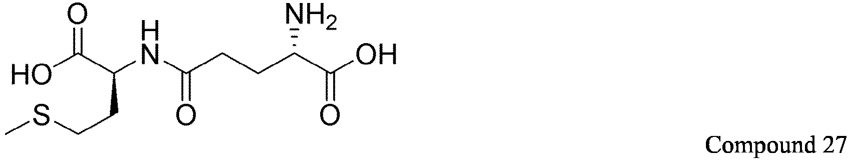

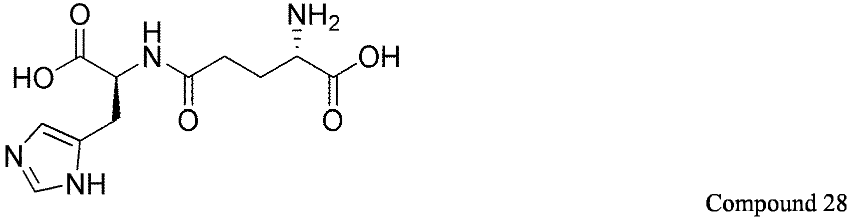

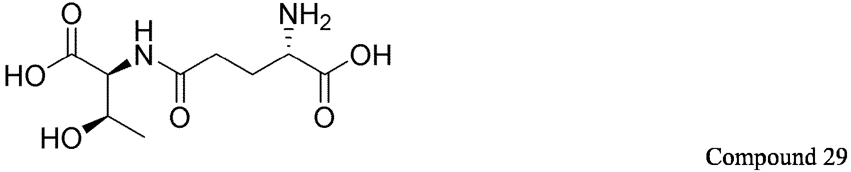

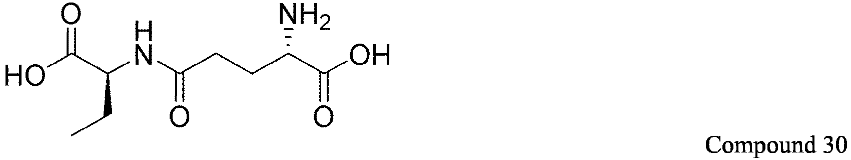

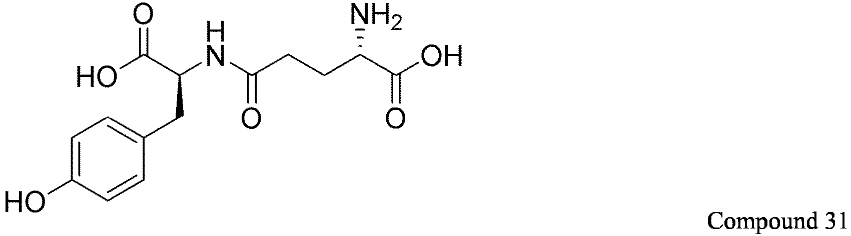

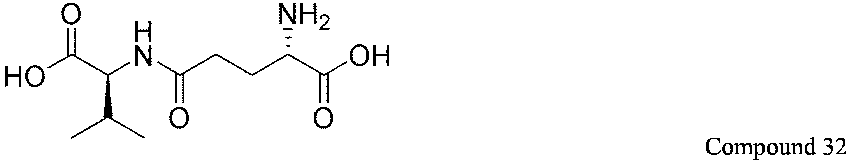

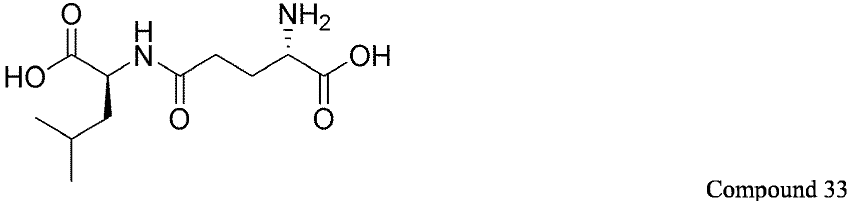

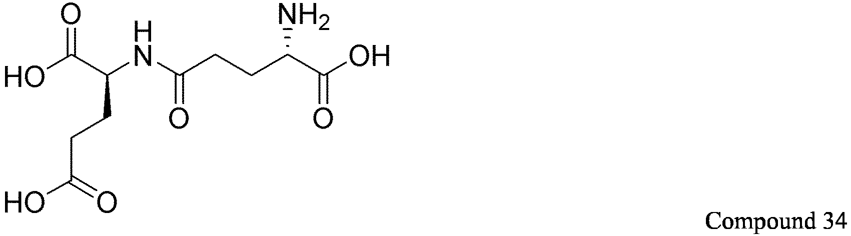

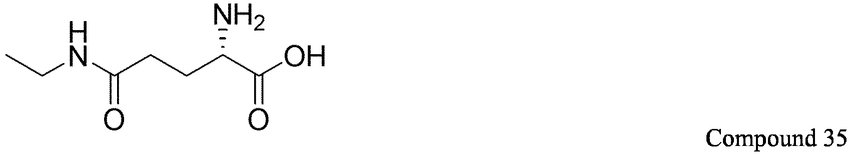

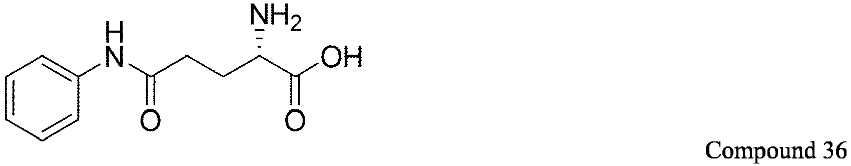

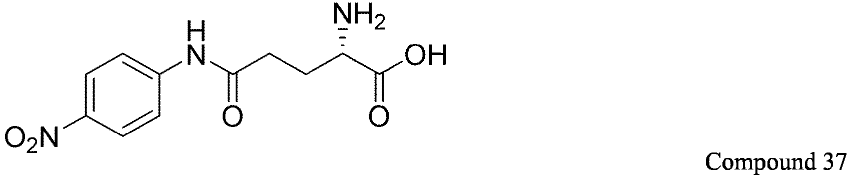

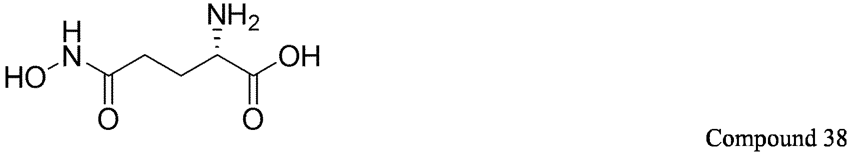

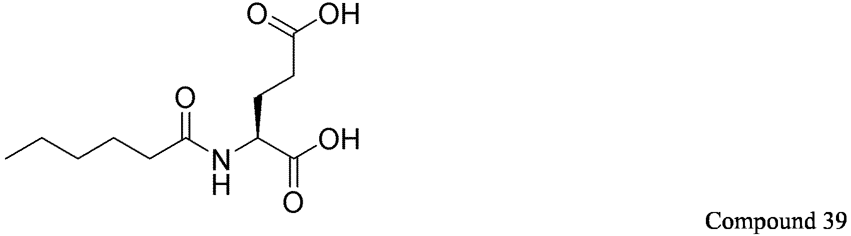

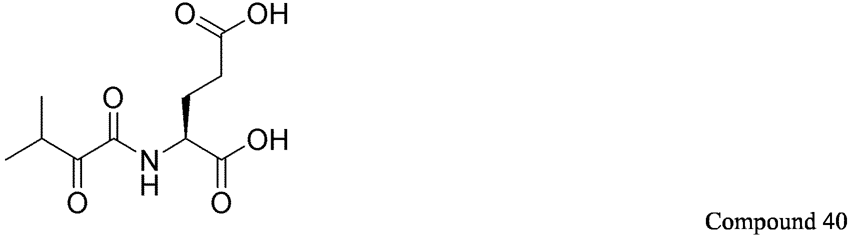

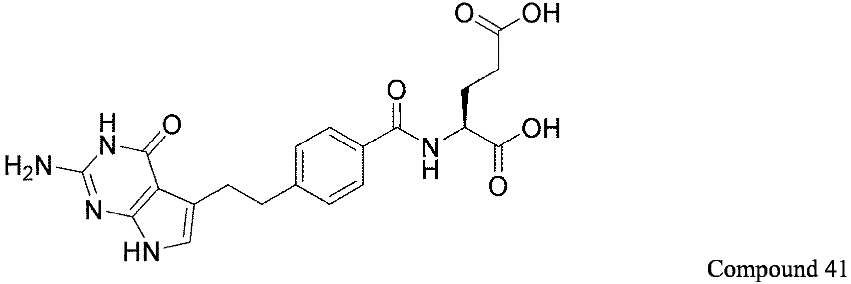

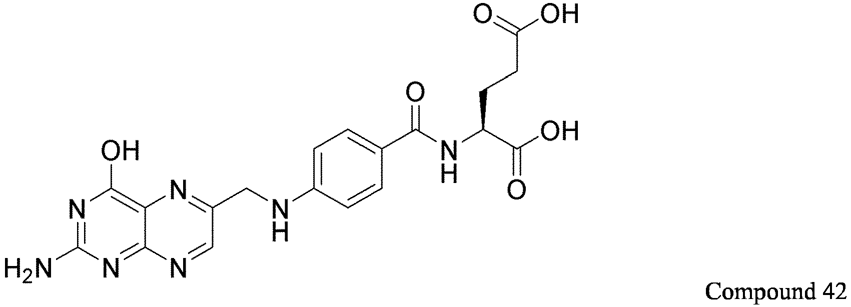

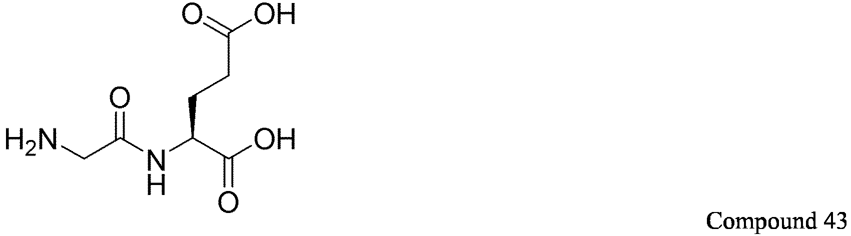

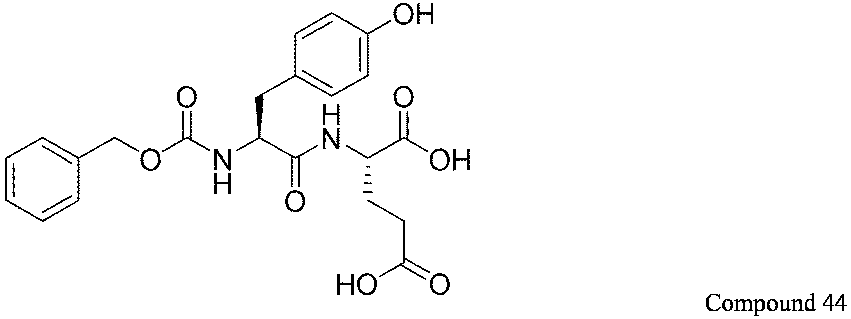

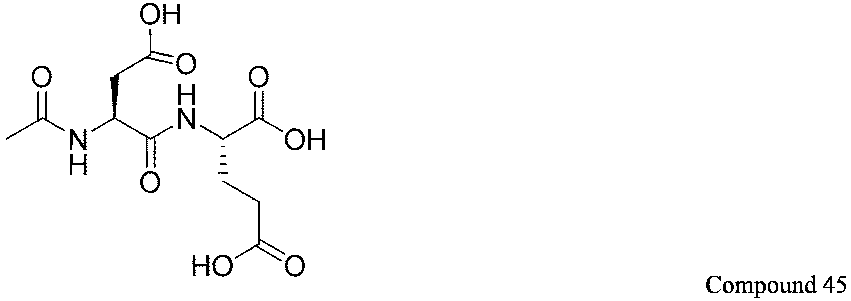

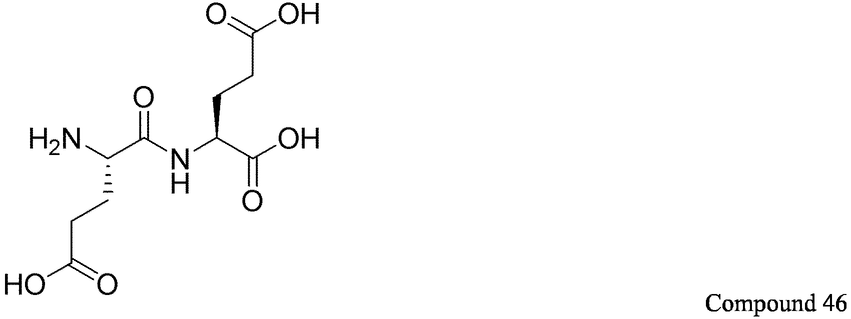

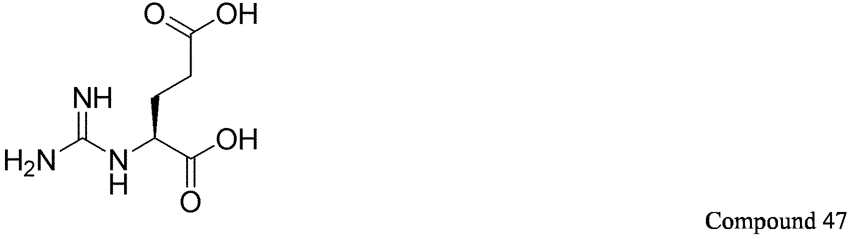

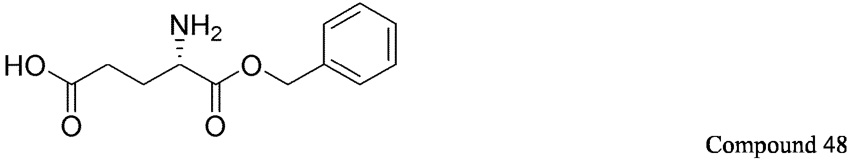

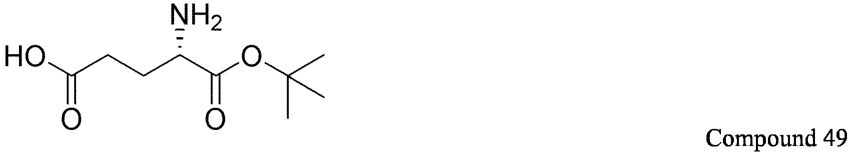

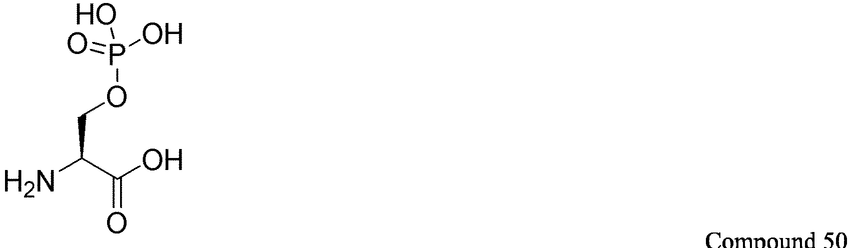

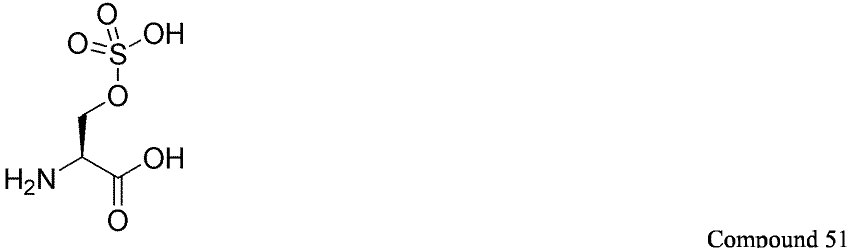

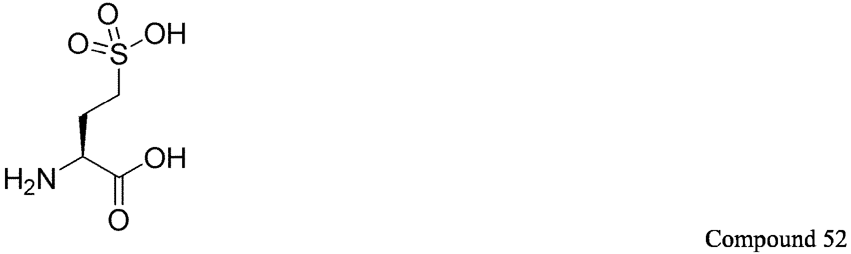

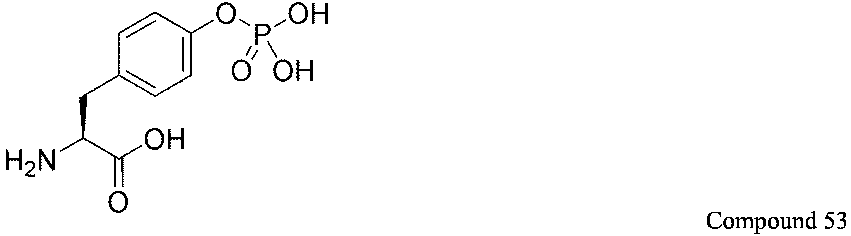

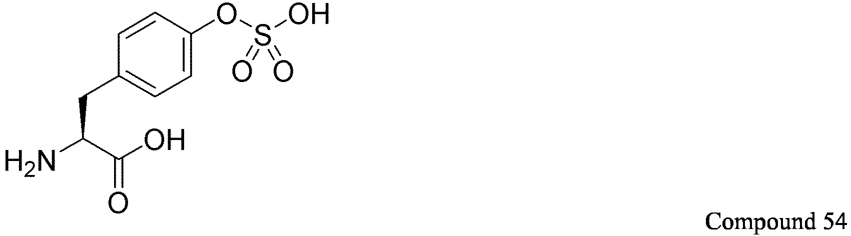

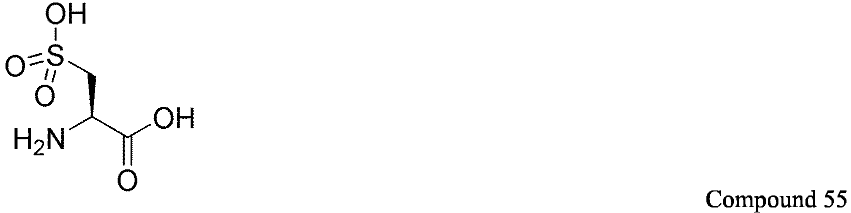

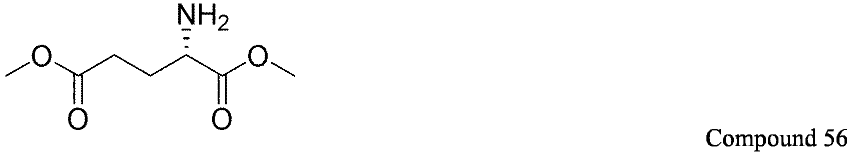

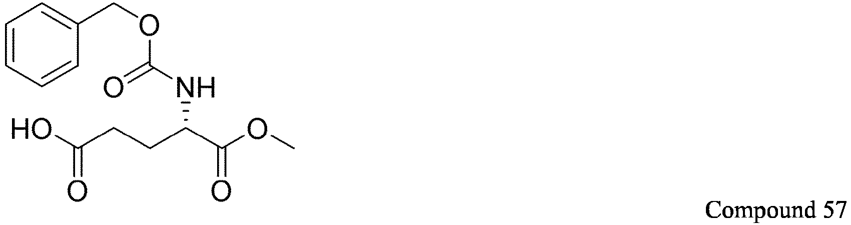

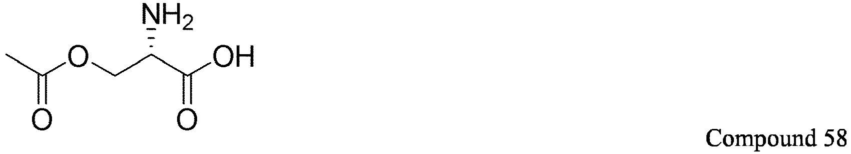

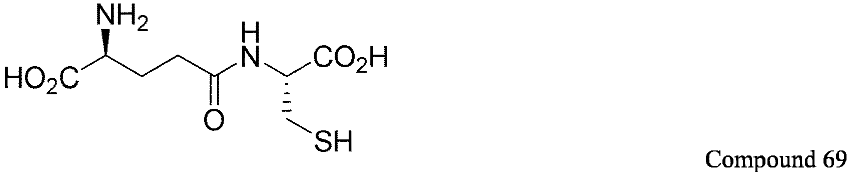

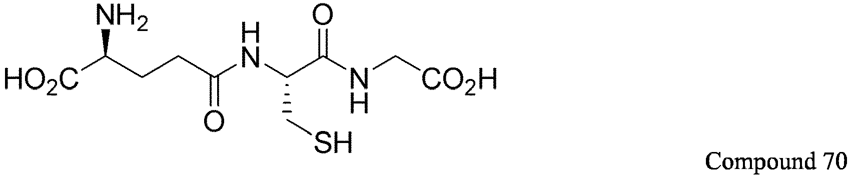

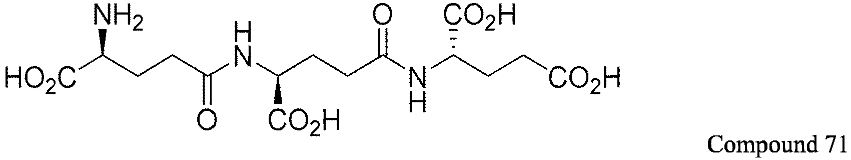

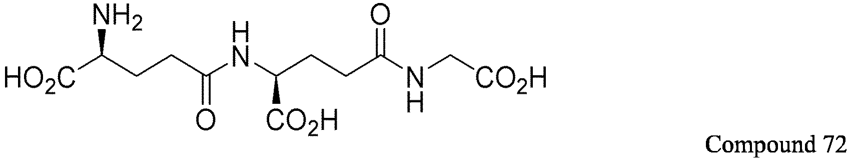

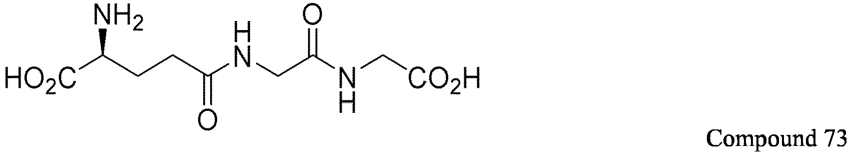

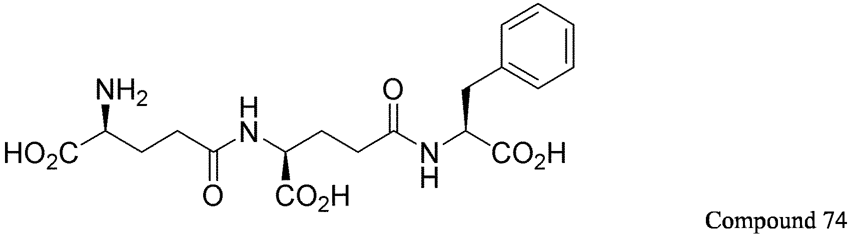

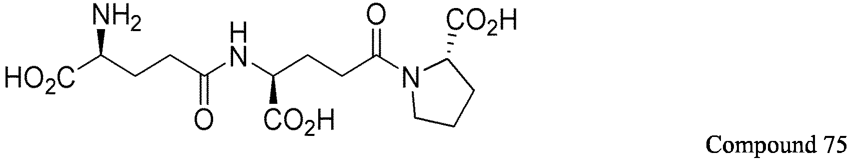

### Example 1. Evaluation of Arabidopsis thaliana Ca²⁺ signal-inducing activity of various compounds

In this example, Ca²⁺ signals produced by contact of Compounds 1-58 and 67-75 with the glutamate receptor of *Arabidopsis thaliana* were analyzed by a real-time imaging assay in which Ca²⁺ is visualized by fluorescence color development. That is, by using a recombinant *Arabidopsis thaliana* p35S::GCaMP3, which incorporated the gene encoding the calcium-sensitive fluorescent protein GCaMP3, as a material, changes in intracellular Ca²⁺ levels caused by treatments with Compounds 1-58 and 67-75 were detected as fluorescence luminance of GCaMP3, and thereby Ca²⁺ signal transduction in plant bodies was analyzed in real time.

The real-time imaging assay of the Ca²⁺ signals was performed as follows.

As the plant material, 2-week old recombinant *Arabidopsis thaliana* p35S::GCaMP3 grown on sterile agar medium [1 x MS salts, 1% (w/v) sucrose, 0.01% (w/v) myoinositol, 0.05% (w/v) MES and 0.4% (w/v) gellan gum; pH 5.8 adjusted with KOH] was used. A small cut of about 1 mm was made at the tip of the first leaf with scissors, and the plant was allowed to stand for 35 minutes. The plant was then placed under the field of view of the fluorescence microscope SMZ25 (Nikon Solutions), and 3 µL of a compound solution was applied to the leaf cut. The generated Ca²⁺ signals were examined in real time with ORCA-Flash4.0 V2 (Hamamatsu Photonics). As the compound solution, solutions prepared by dissolving Compounds 1-58 and 67-75, negative control, and positive control in a dilution medium [1 x MS salts, 1% (w/v) sucrose, 0.01% (w/v) myoinositol, and 0.05% (w/v) MES; pH 5.8 adjusted with KOH] at 100 mM, respectively, were used. However, for some compounds with low solubility, solutions prepared at 10 mM were used as the compound solution. Sorbitol, which does not produce Ca²⁺ signal, was used as the negative control. Glutamic acid was used as the positive control. Compounds that produced Ca²⁺ signals in one or more leaves were determined to be compounds that induce Ca²⁺ signals.

As a result, Ca²⁺ signals were observed in one or more leaves of all the plants treated with Compounds 1-58 and 67-75, that is, it was found that all the compounds induced Ca²⁺ signals. In particular, Compounds 1 and 34 caused strong Ca²⁺ signal induction.

### Example 2. Evaluation of pest damage resistance gene expression-inducing activity of various compounds

In this example, whether Compounds 1 and 34, which showed strong Ca²⁺ signal-inducing activity in Example 1, induce expression of a pest damage resistance gene in plants was analyzed by qPCR.

As the pest damage resistance gene, the AtOPR3 (12-oxophytodienoic acid reductase, involved in the jasmonic acid biosynthesis) gene, which is a typical pest damage resistance gene, was selected. As an internal standard, the AtACT8 gene was selected. The recombinant *Arabidopsis thaliana* p35S::GCaMP3 was used as a plant material, and cut was formed on the first leaf of the plant cultivated in the same manner as in Example 1 and treated with a compound. As the compound, Compounds 1 and 34 were used. Sixth leaves at 0 and 30 minutes after the compound treatment were collected and frozen in liquid nitrogen. The total RNA was extracted from the frozen leaf samples (50 to 200 mg) by using RNeasy Plant Mini Kit (Qiagen) according to the protocol thereof. The obtained total RNA was subjected to a residual genomic DNA-removing treatment with RNase free DNase I by using TURBO DNase Kit (Ambion) according to the protocol of the kit. By using this total RNA sample and SuperScriptIII First-Strand Synthesis System for RT-PCR (Invitrogen), single-stranded cDNA was prepared. The cDNA in an amount corresponding to 10 ng of the total RNA was used as the template, the primer concentration was 200 nM, and the reaction system was made to have a volume of 15 µL with 2xEvaGreen qPCR master mix. qPCR was performed by using Mx3000P QPCR System and MxPro qPCR software (Agilent Technologies) under the conditions of 95°C for 15 minutes, 40 cycles of 95°C for 20 seconds, 58°C for 15 seconds, and 65°C for 15 seconds, and 65°C for 15 seconds. For qPCR of the AtOPR3 gene, the primers of SEQ ID NOS: 1 and 2 were used. For qPCR of the AtACT8 gene, the primers of SEQ ID NOS: 3 and 4 were used. The relative expression amount of the AtOPR3 gene to that of the internal standard AtACT8 gene was calculated by the ΔΔCt method. The test was repeatedly performed 4 or 5 times, and the average of the results was calculated.

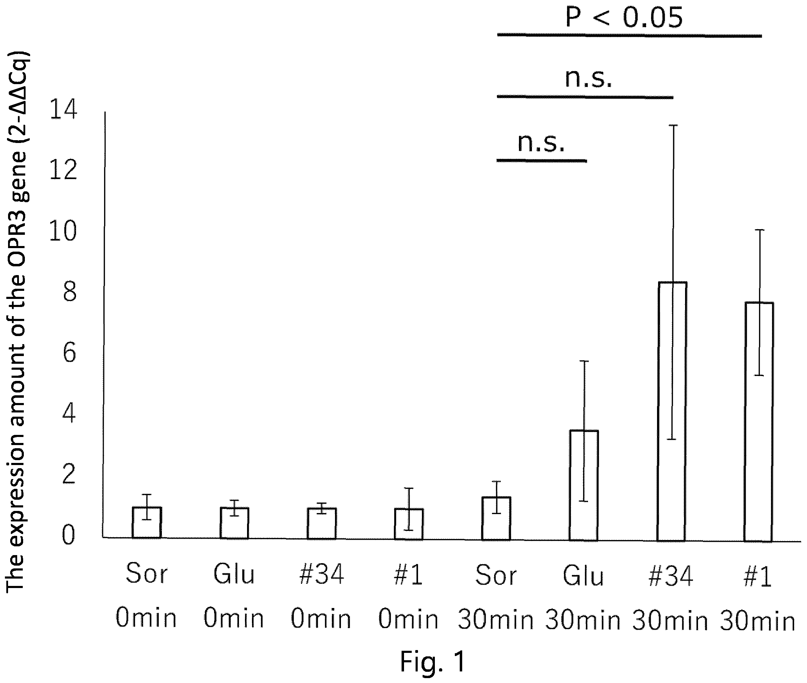

The results are shown in Fig. 1. Compounds 1 and 34 showed pest damage resistance gene expression-inducing activities equal to or greater than that of glutamic acid. In particular, Compound 1 showed a high pest damage resistance gene expression-inducing activity.

### Example 3. Pest damage-suppressing activity of various compounds

In this example, whether Compound 1, which showed Ca²⁺ signal-inducing activity and pest damage resistance gene expression-inducing activity in Examples 1 and 2, actually has a pest damage-suppressing activity was examined.

The recombinant *Arabidopsis thaliana* p35S::GCaMP3 was used as a plant material, and cut was formed on the first leaf of the plant grown in the same manner as in Example 1 and treated with a compound. Compound 1 was used as the compound. Sorbitol was used as the negative control, and glutamic acid as the positive control. The test was repeatedly conducted 10 times (using independent plants and pests) per plot. One hour after the treatment with the compound, the plant body and 2-week old larva of *Pieris rapae* were placed in the same container to perform a 4-hour feeding damage test. Weights of the plant body and larva were measured before and after the test to evaluate the weight of the feeding-damaged plant and the weight gain rate of the pest.

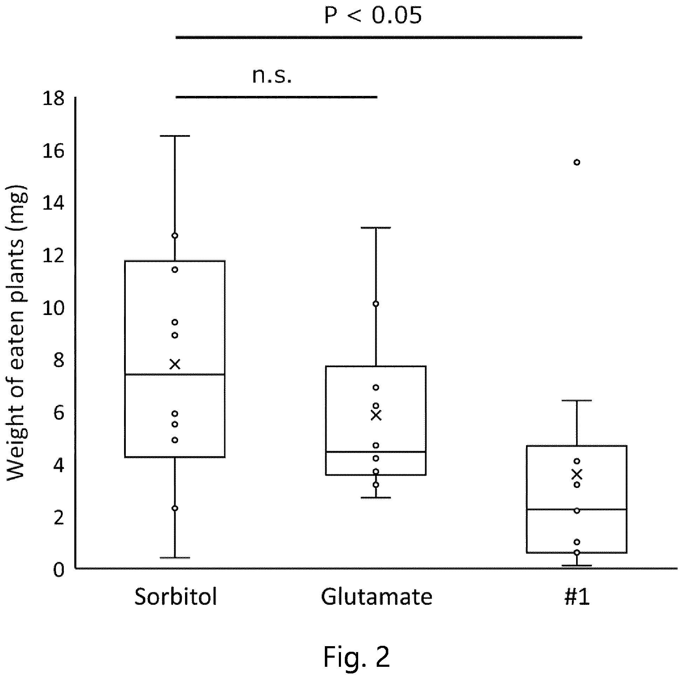

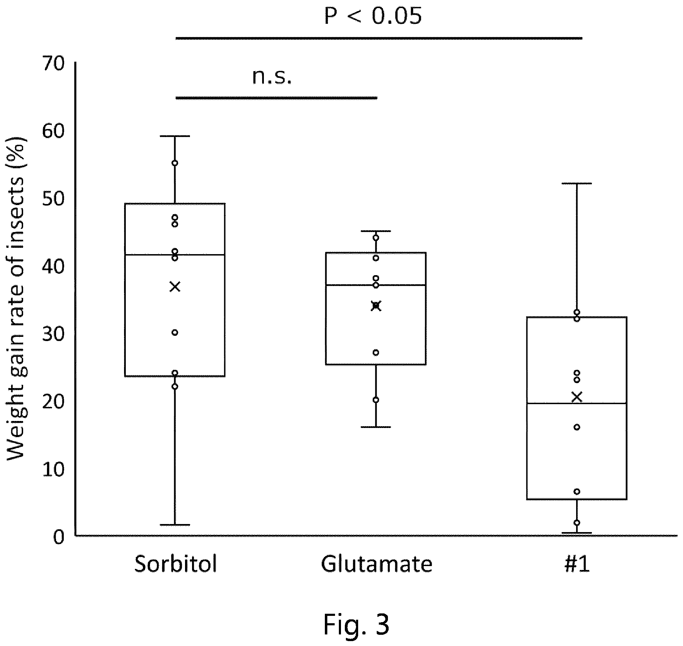

The results are shown in Figs. 2 and 3. In the glutamic acid-treated plot, the amount of the pest damage (weight of the feeding-damaged plant and weight gain rate of the pest) was similar to that observed in the sorbitol-treated plot, and thus no pest damage-suppressing activity was observed for glutamic acid. On the other hand, the amount of pest damage (weight of feeding-damaged plant and weight gain rate of the pest) was lower in the Compound 1-treated plot than in the sorbitol-treated plot, and thus a distinct pest damage-suppressing activity was observed for Compound 1.

### Example 4. Comparison of pest damage-suppressing activities of glutamic acid derivative and commercial product

In this example, the pest damage-suppressing activity of Compound 1, for which distinct pest damage-suppressing activity was observed in Example 3, and that of Jasmomate Ekizai (the active substance is prohydrojasmon, a typical injurious hormone-like substance), which is the only resistance inducer for pest damage on the market, were compared.

The recombinant *Arabidopsis thaliana* p35S::GCaMP3 was used as a plant material, and cut was formed on the first leaf of the plant cultivated in the same manner as those of Examples 1, 2, and 3 and treated with a compound. Compound 1 and Jasmomate were used as the compound. Compound 1 was used at 100 mM, and Jasmomate was used at a 500-fold dilution concentration (0.4 mM as prohydrojasmon concentration), which is the usual recommended treatment concentration thereof. Sorbitol was used as a negative control. The test was conducted for 15 replicates per plot (independent plants and insects). For Jasmomate, a spray treatment of the leaves was performed (spray plot, Jasmomate was sprayed twice at the concentration mentioned above with an atomizer). The test of the spray plot was conducted for 10 replicates (independent plants and insects). One hour after the compound treatment, the plant body and 2-week old larva of Pieris rapae were placed in the same container to perform a 4-hour feeding damage test. The larva was weighed before and after the test, and the weight gain rate of the pest was evaluated.

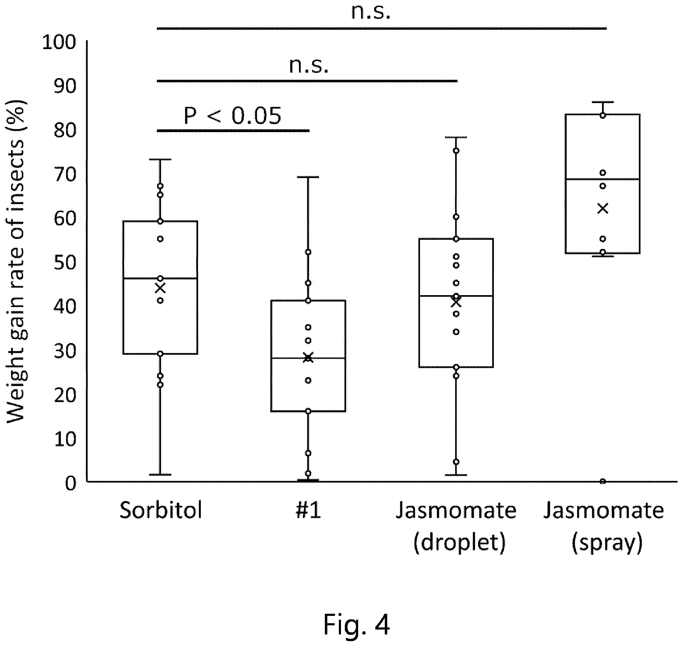

The results are shown in Fig. 4. In the Jasmomate-treated plot (droplet plot), the amount of pest damage (weight gain rate of the pest) was similar to that observed in the sorbitol-treated plot, and thus Jasmomate showed no pest damage-suppressing activity. On the other hand, the amount of pest damage (weight gain rate of the pest) was lower in the Compound 1-treated plot than in the sorbitol-treated plot, and thus a distinct pest damage-suppressing activity was observed for Compound 1.

### Example 5. Pest damage-suppressing activity of glutamic acid derivative in real crops

In this example, there was evaluated whether Compound 1, which was found to have a Ca²⁺ signal-inducing activity and pest damage resistance gene expression-inducing activity in Examples 1 and 2, and found to have a distinct pest damage-suppressing activity in Examples 3 and 4, has a pest damage-suppressing activity at a concentration in a range lower than 100 mM, at which the studies of the above examples up to Example 4 were conducted, in leaves of food crops. Glutamic acid was used as a positive control, and Jasmomate Ekizai (the active substance is prohydrojasmon, a typical injurious hormone-like substance), which is the only resistance inducer for pest damage on the market, was used as a control. The test was conducted for 10 replicates per plot (independent plants and pests).

The plant material was leaf discs collected from komatsuna *(Brassica rapa* var. *perviridis)* seedlings grown for 1 month after seeding on culture soil in pots, and the test organism was early second instar larva of *Spodoptera litura.* Compound 1 was used as a 1 mM solution in Silwet L-77 4,000-fold dilution solution prepared by adding Silwet L-77 to distilled water as a solvent. As a negative control, 100 mM or 1 mM sorbitol was used, and 100 mM or 1 mM glutamic acid was used as a positive control. Jasmomate Ekizai was used at a 500-fold dilution concentration (0.4 mM as prohydrojasmon concentration), which is the usual recommended treatment concentration thereof, and obtained by using distilled water as the solvent. One hour prior to the Compound 1 treatment, the first leaves of komatsuna seedlings were collected, and leaf discs were prepared by using a 5-cm diameter cork borer so that the main veins were included in the discs. One leaf disc was submerged in 50 ml of each test solution prepared in a 100-ml beaker, and immersed for about 10 seconds to perform the Compound 1 treatment. The treated leaves were placed on a paper towel, and allowed to air-dry for 20 to 30 minutes, then each leaf was stored in the same test container with 10 second instar larvae of *Spodoptera litura,* and the container was closed with a lid, and maintained at 25 ± 2°C, 16L8D. The test container was a plastic cup (φ 66 mm x 36 mm, clean cup manufactured by RISPAC) with a bottom lined with absorbent cotton topped with φ 55 mm filter paper, into which about 10 ml of distilled water was poured. One day after the release, the number of surviving *Spodoptera litura* larvae in each test container was counted, and a photograph was taken from a height of 5 cm above the container with the lid open so that the entire image could be photographed. Image analysis software (ImageJ) was used to determine the damage area (feeding damage area), and the average damage area ratio was calculated for each treatment plot.

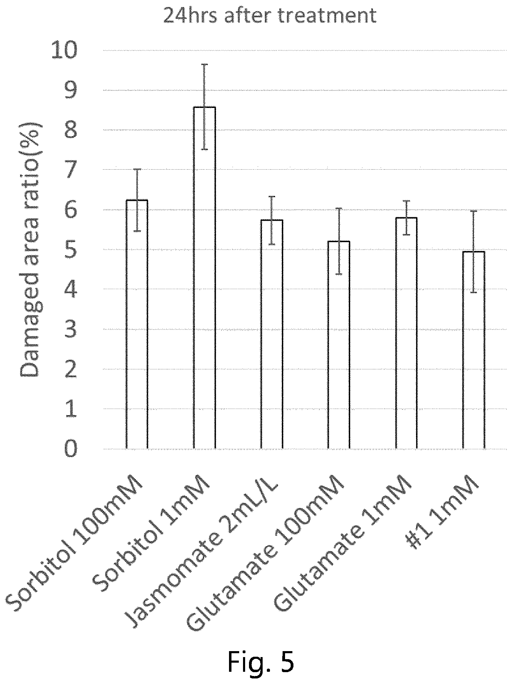

The results are shown in Fig. 5. The pest damage area ratio of the Jasumomate-treated plot was comparable to that of the 100 mM sorbitol-treated plot, i.e., Jasumomate showed no pest damage-suppressing activity. A greater pest damage-suppressing tendency was observed for the 100 mM glutamic acid treatment plot compared with the 100 mM sorbitol treatment plot. On the other hand, a pest damage-suppressing effect (specifically, feeding inhibitory effect) higher than those observed for the 100 mM glutamic acid treatment plot and Jasumomate treatment plot was observed in the Compound 1 treatment plot, despite that the treatment concentration used in the Compound 1 treatment plot was 1 mM.

### INDUSTRIAL APPLICABILITY

According to the present invention, results useful in the field of agri-horticulture, such as prevention of pest damage to plants, can be obtained.

### EXPLANATION OF SEQUENCE LISTING

SEQ ID NOS: 1-4, Primers

## Claims

1. A composition for inducing pest damage resistance in a plant or preventing pest damage to a plant, comprising the following ingredient (A):
(A) a compound represented by the following general formula (I): 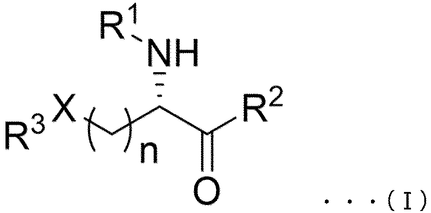 wherein:
n represents an integer of from 1 to 3 (i.e., 1, 2, or 3),
X represents an interatomic bond, oxygen atom (O), nitrogen atom bonded with hydrogen atom (NH), nitrogen atom bonded with methyl group (NMe), sulfur atom (S), or 4-phenoxy group,
R¹ represents hydrogen atom (H), amidino group, or an acyl group,
R² represents OR⁴ or NHR⁴,
R³ represents C(=O)-R⁵, sulfo group (SO₃H), phosphate group (PO₃H₂), or SO₂R⁶,
R⁴ represents hydrogen atom (H) or an optionally substituted alkyl group having 1 to 8 carbon atoms,
R⁵ represents an optionally substituted alkyl group having 1 to 8 carbon atoms, an amino acid, a dipeptide, OR⁷, or NR⁸R⁹,
R⁶ represents methyl group or an optionally substituted phenyl group,
R⁷ represents hydrogen atom (H), or an alkyl group having 1 to 8 carbon atoms, and
R⁸ and R⁹ independently represent hydrogen atom (H), an optionally substituted alkyl group having 1 to 8 carbon atoms, or an optionally substituted phenyl group.

2. The composition according to claim 1, wherein n is 2.

3. The composition according to claim 1, wherein X is an interatomic bond.

4. The composition according to claim 1, wherein the composition satisfies any of the following conditions:
(1) n is 2, X is an interatomic bond, R² is hydroxyl group, and R³ is C(=O)-R⁵;
(2) n is 2, X is an interatomic bond, R' is hydrogen atom, R² is hydroxyl group, R³ is C(=O)-R⁵, and R⁵ is other than hydroxyl group;
(3) n is 2, X is an interatomic bond, R¹ is other than hydrogen atom, R² is hydroxyl group, R³ is C(=O)-R⁵, and R⁵ is hydroxyl group; and
(4) n is 2, X is an interatomic bond, R¹ is hydrogen atom, R² is other than hydroxyl group, and R³ is C(=O)-OH.

5. The composition according to claim 1, wherein the ingredient (A) is selected from the compounds represented by the following formulas of Compound 1-58 and Compound 67-75:

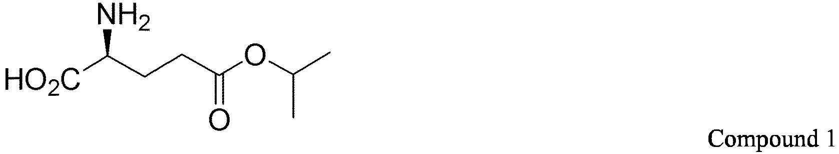

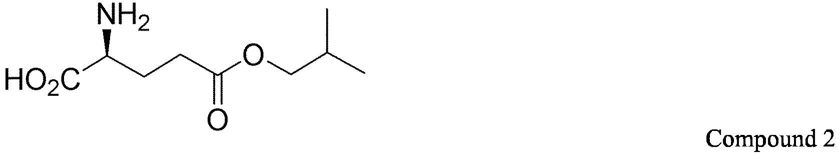

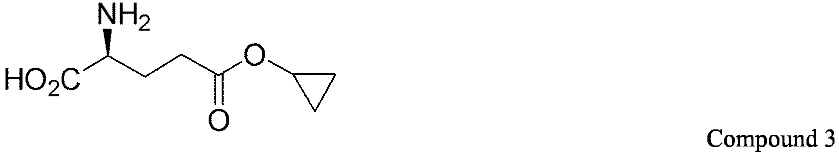

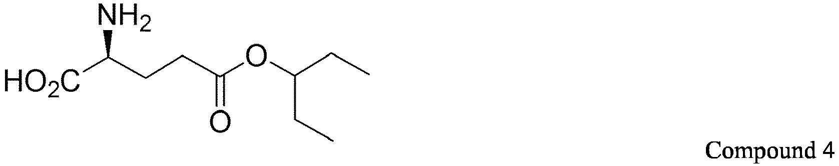

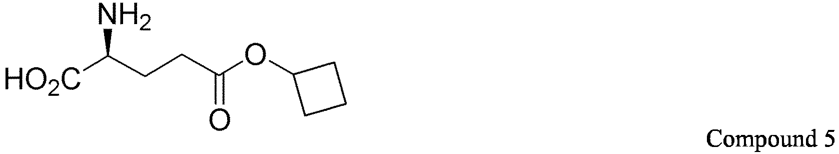

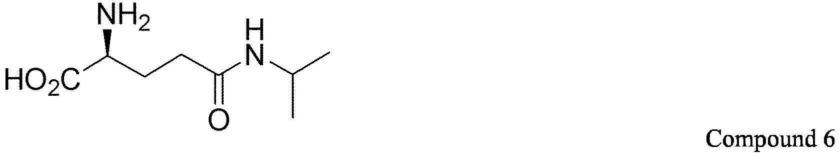

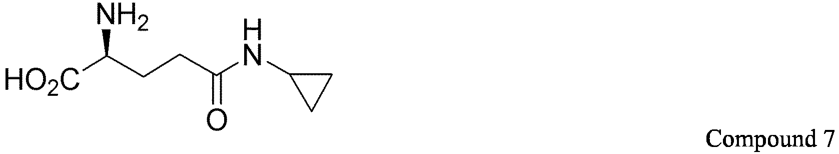

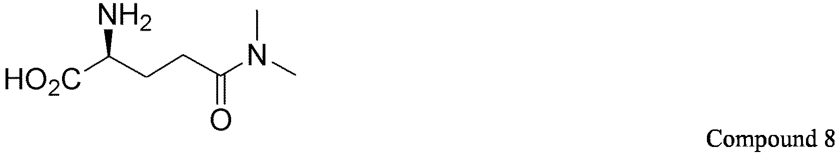

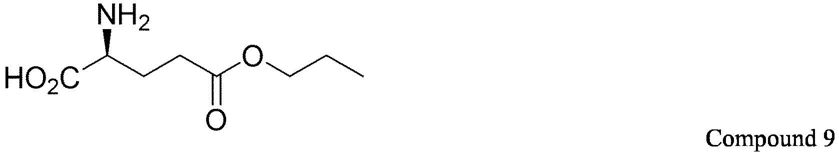

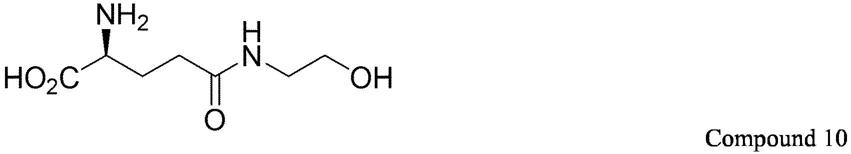

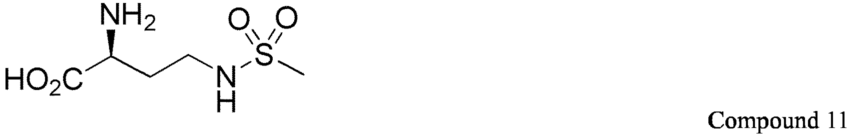

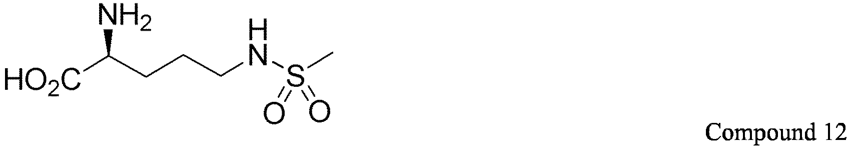

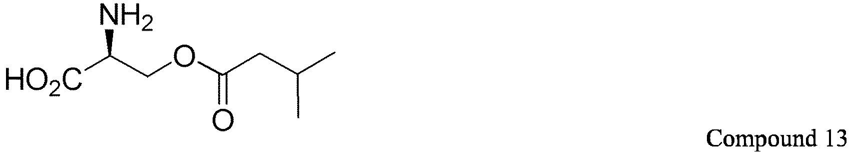

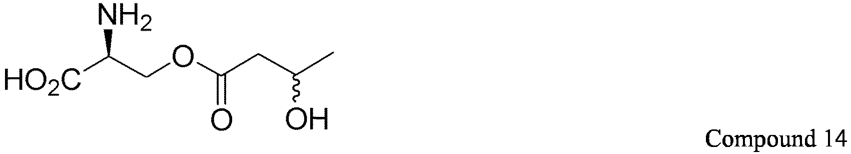

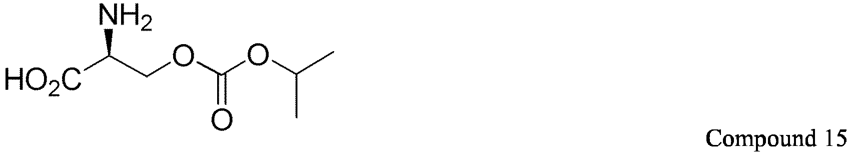

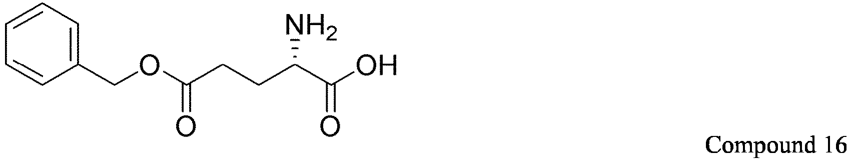

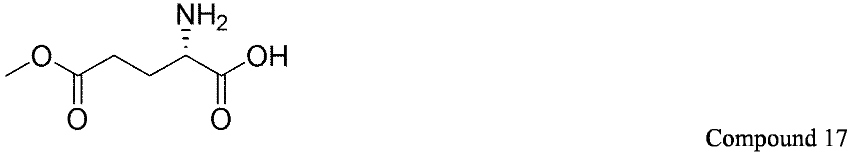

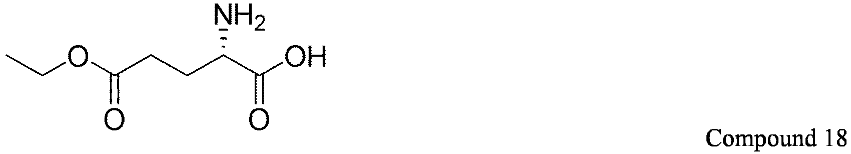

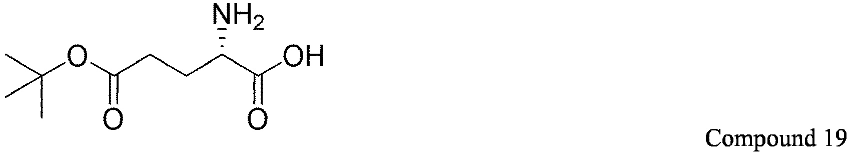

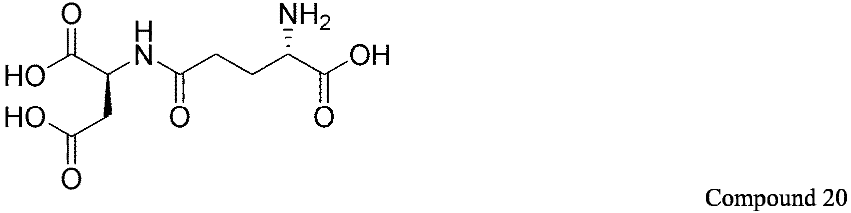

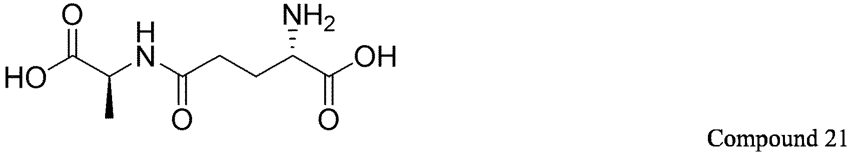

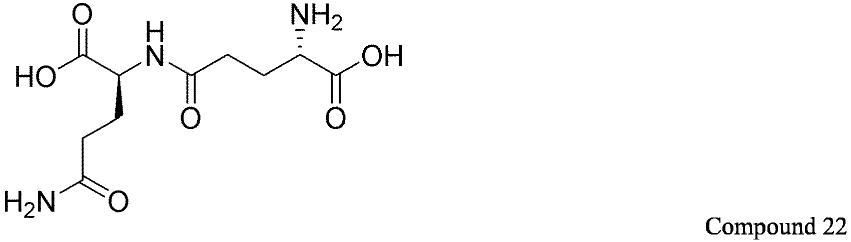

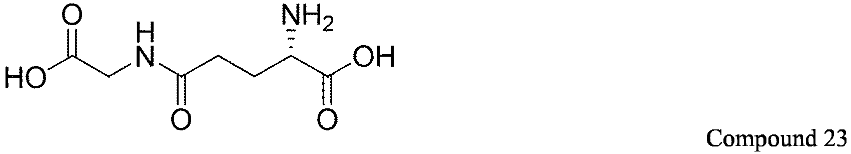

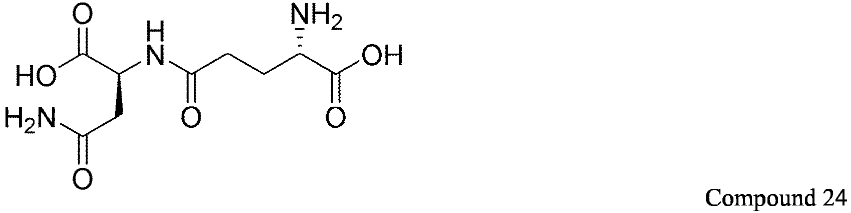

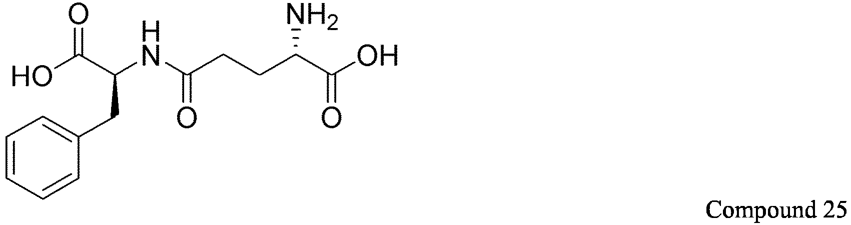

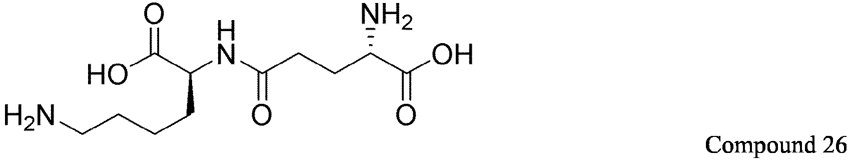

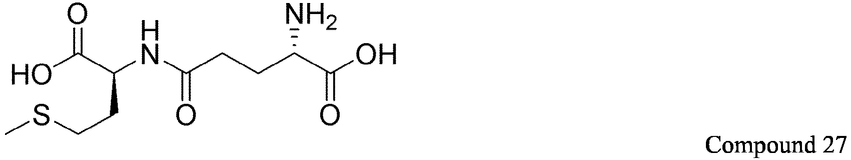

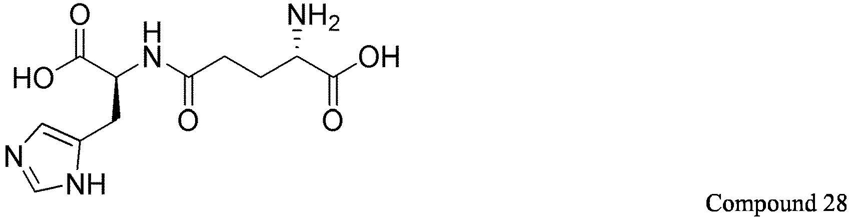

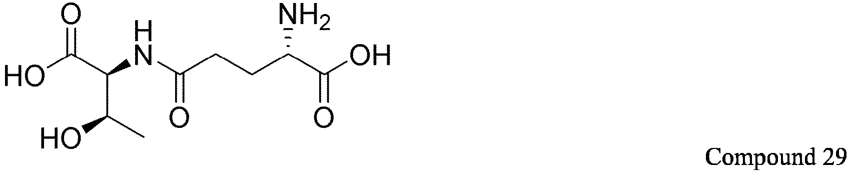

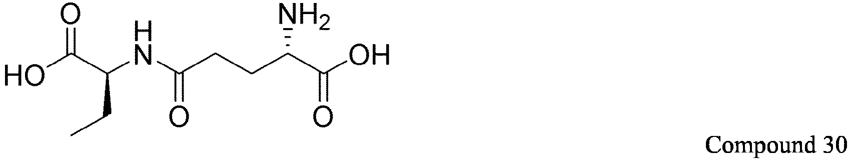

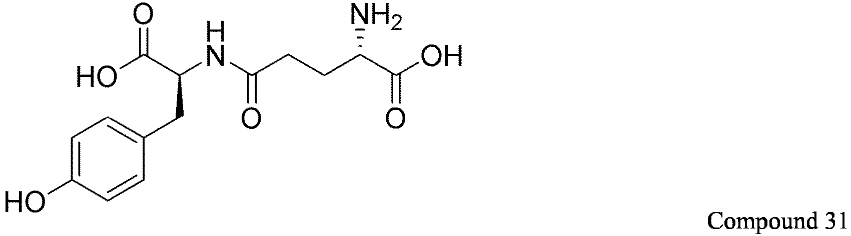

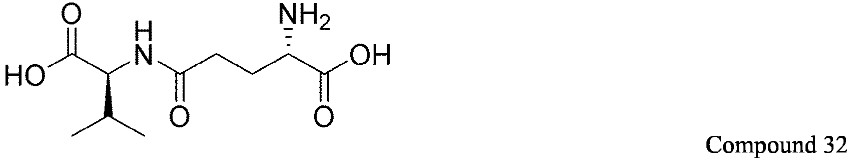

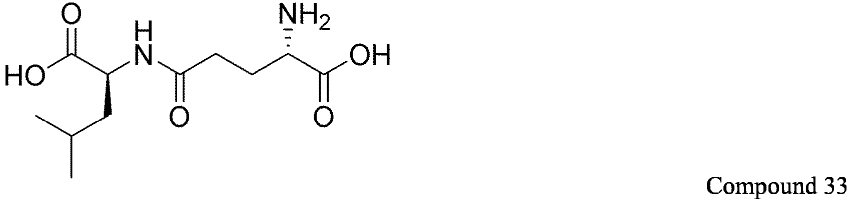

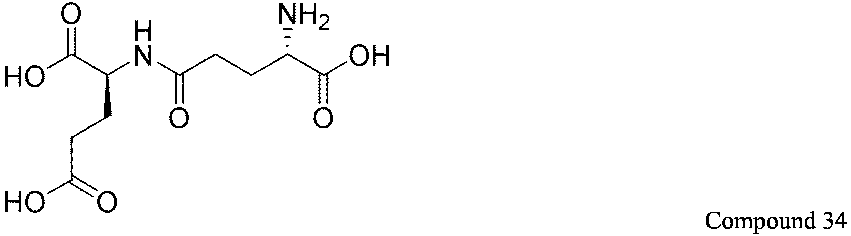

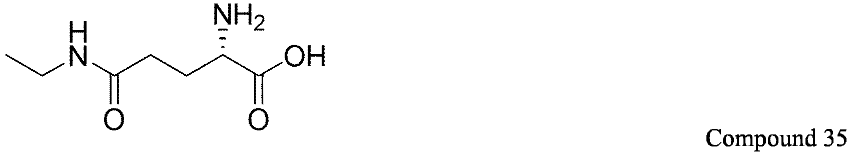

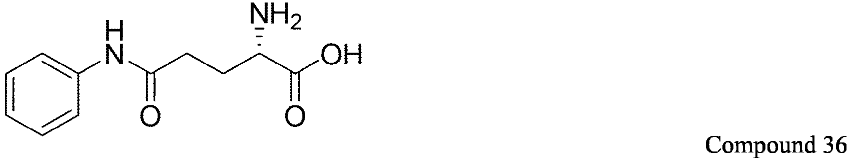

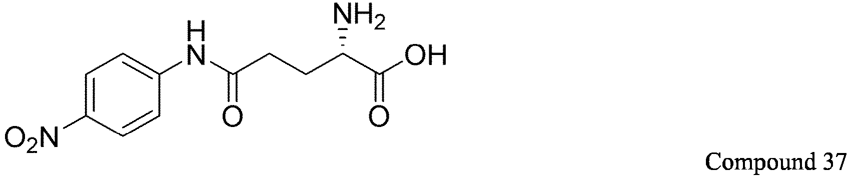

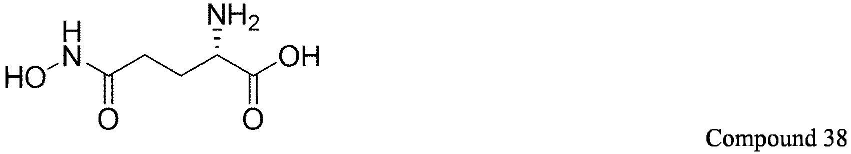

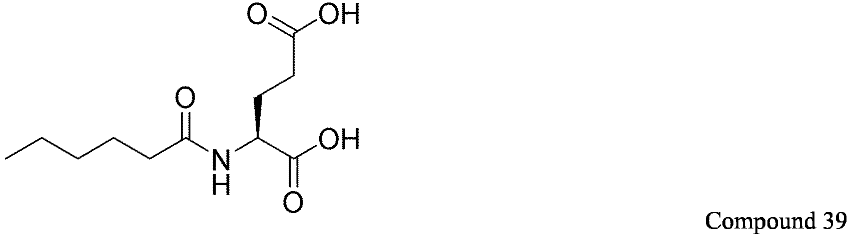

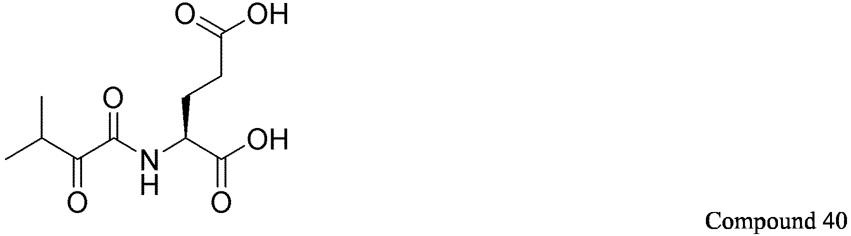

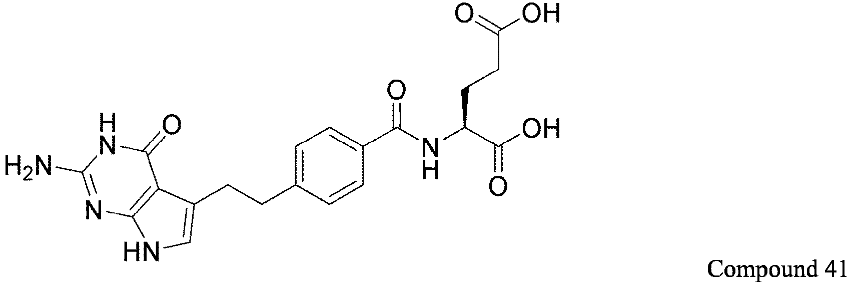

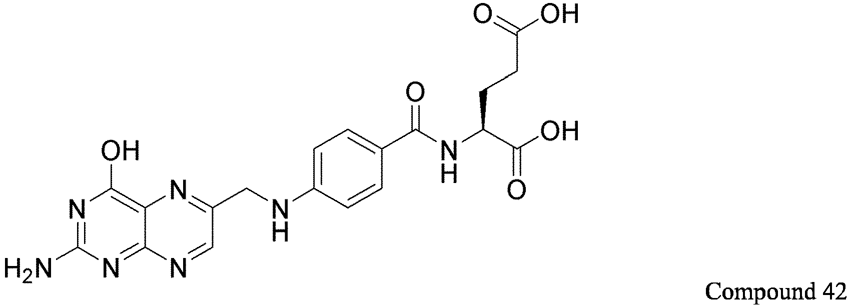

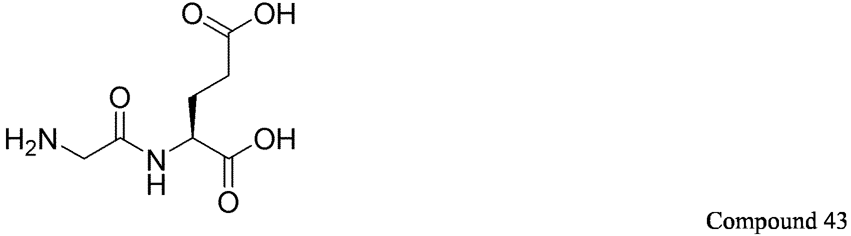

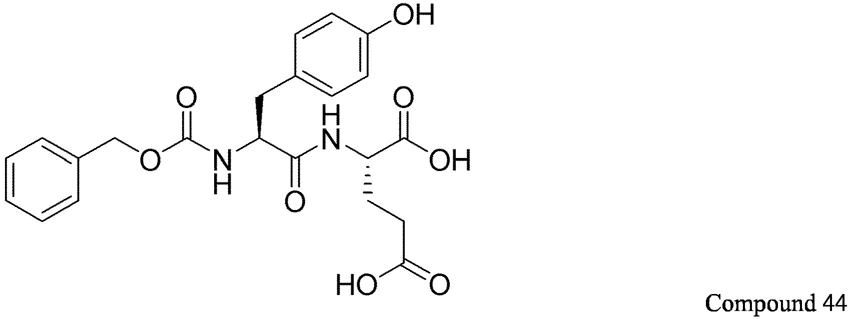

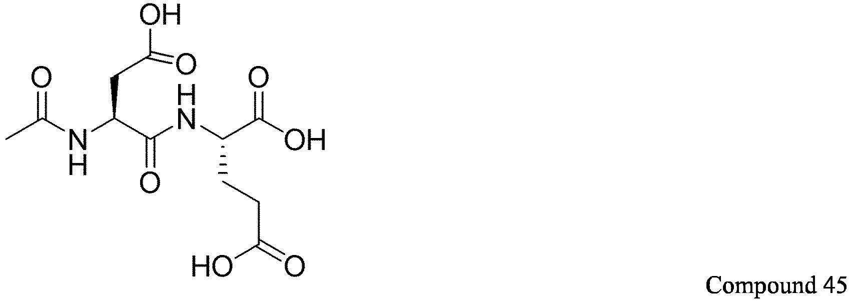

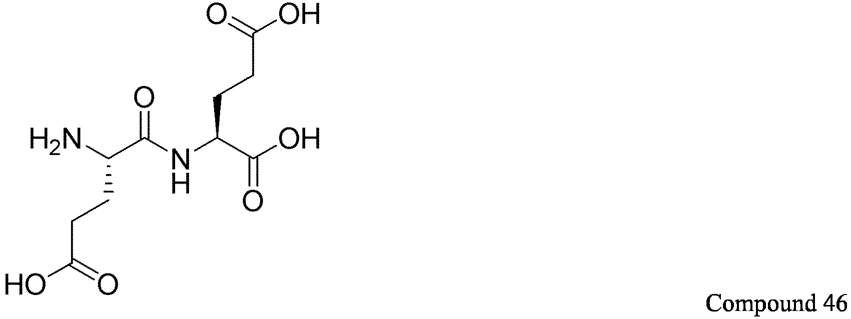

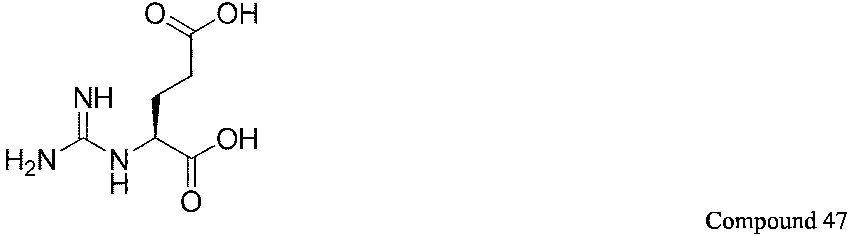

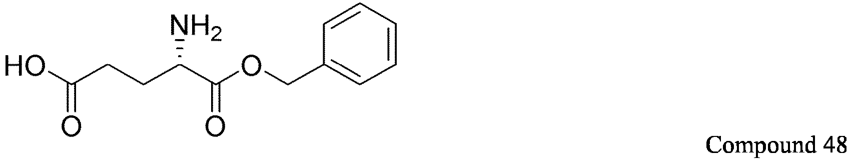

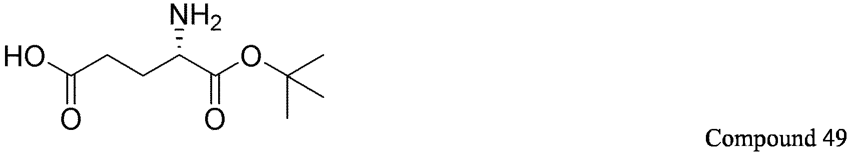

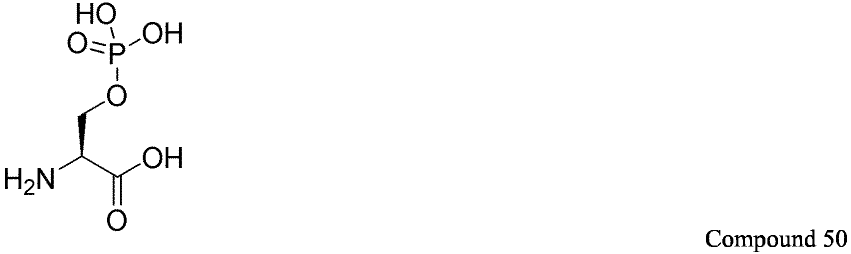

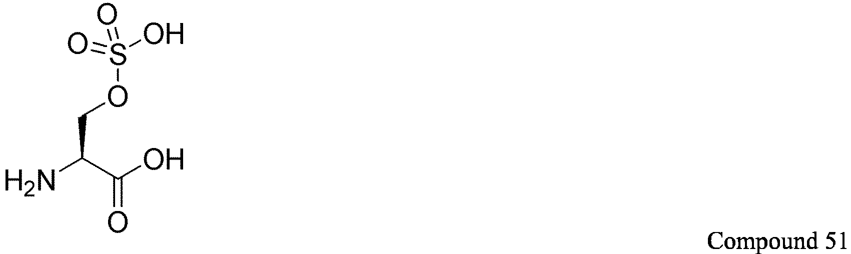

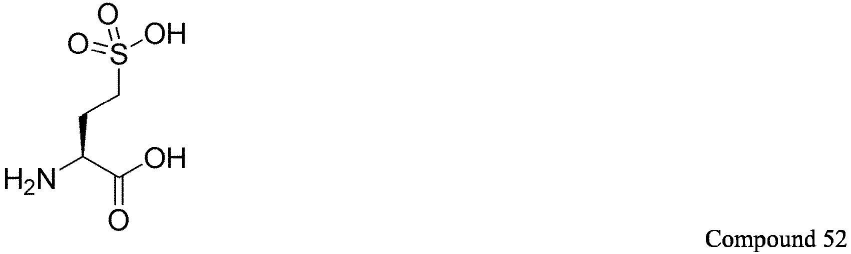

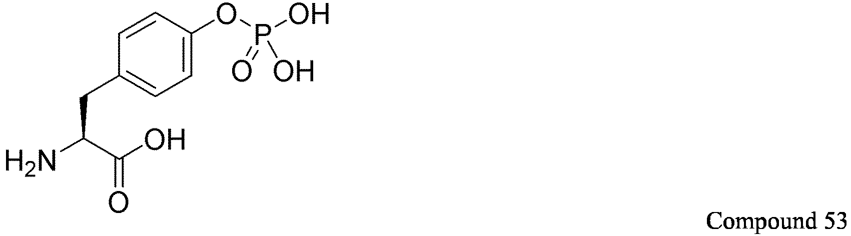

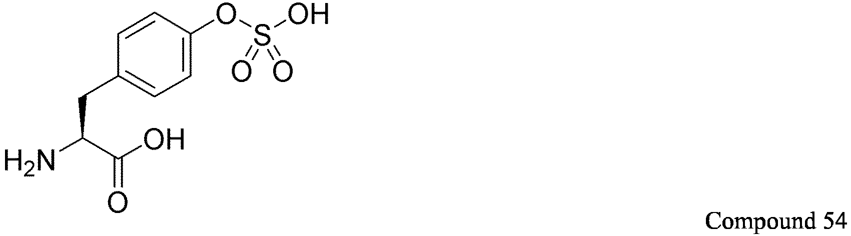

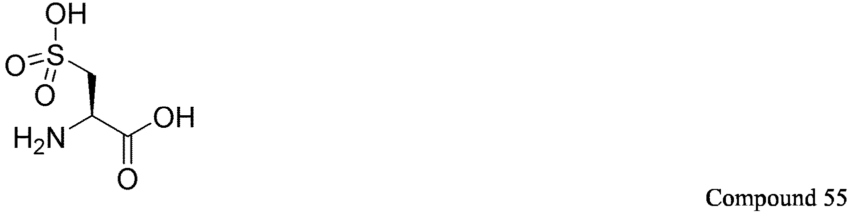

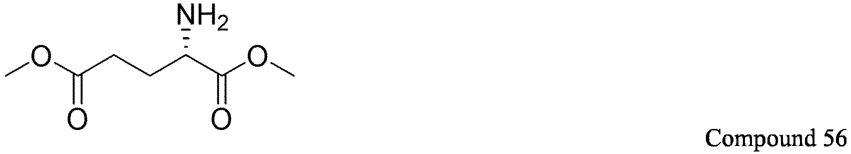

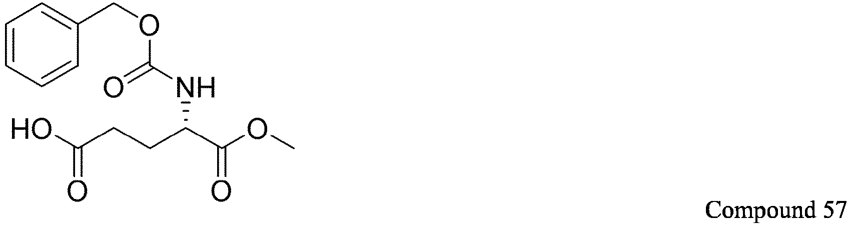

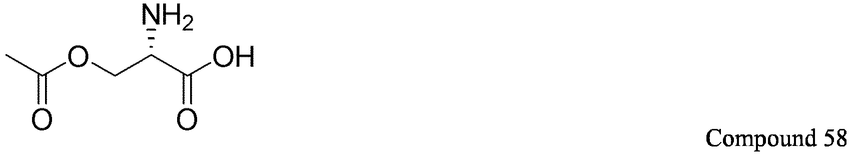

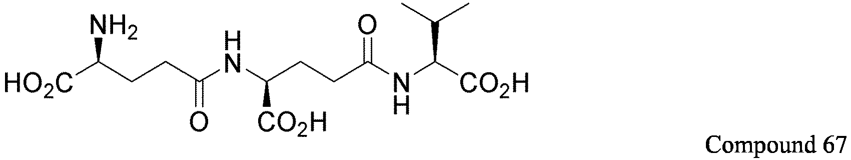

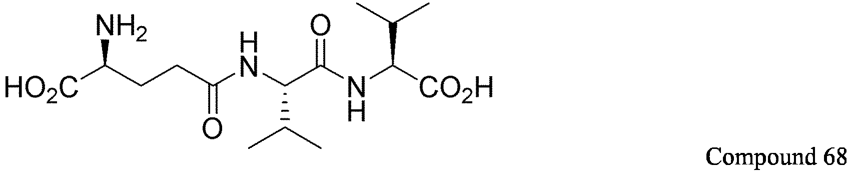

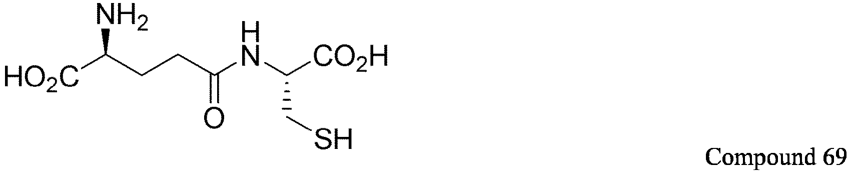

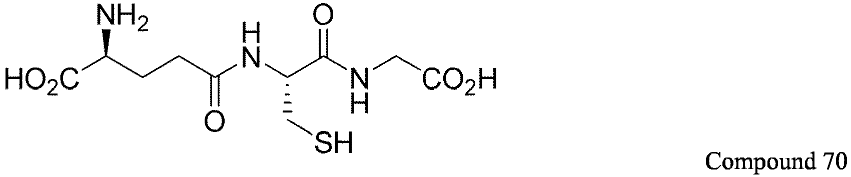

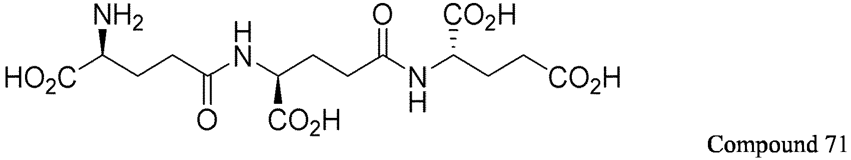

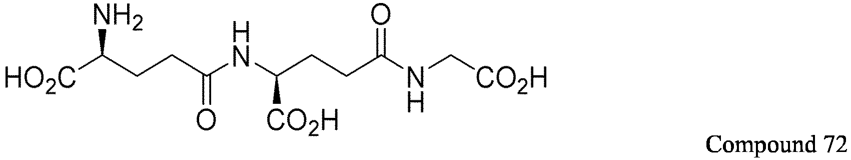

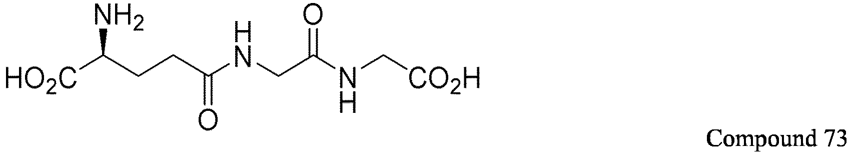

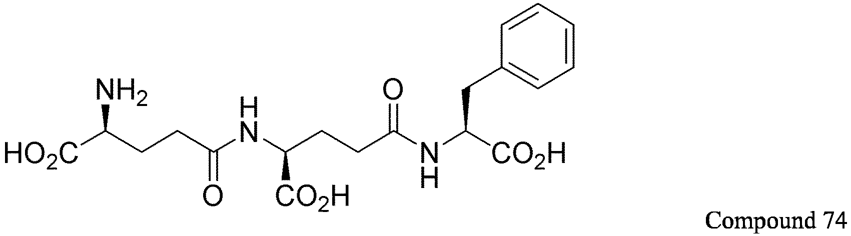

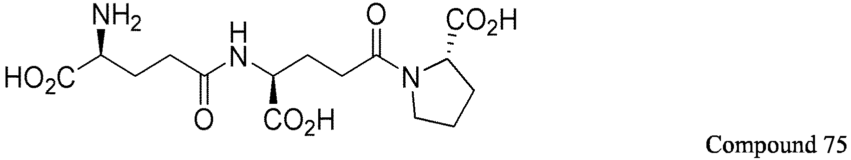

6. The composition according to claim 1, wherein the pest damage is pest damage caused by an organism classified in the order *Lepidoptera, Hemiptera, Coleoptera, Diptera, Orthoptera, Thysanoptera, Tylenchida, Collembola, Acarina,* or *Stylommatophora.*

7. The composition according to claim 1, wherein the pest damage is pest damage caused by an organism classified in the family *Plutellidae, Noctuidae, Pyralidae, Tortricidae, Lyonetiidae, Carposinidae, Gelechiidae, Crambidae, Arctiidae (Erebidae), Lymantriidae (Erebidae), Cicadellidae, Delphacidae, Psyllidae, Aphididae, Aleyrodidae, Coccoidea, Miridae, Tingidae, Pentatomidae, Lygaeidae, Scarabaeidae, Elateridae, Coccinellidae, Cerambycidae, Chrysomelidae, Curculionidae, Muscidae, Calliphoridae, Sarcophagidae, Anthomyiidae, Tephritidae, Opomyzoidea* (superfamily), *Chloropoidea* (superfamily), *Acrididae, Catantopidae, Pyrgomorphidae, Thripidae, Aeolothripidae, Merothripidae, Aphelenchoididae, Neotylenchidae, Onychiuridae, Isotomidae, Tetranychidae, Dermanyssidae, Acaridae, Sarcoptidae, Philomycidae,* or *Bradybaenidae.*

8. The composition according to any one of claims 1 to 7, wherein the plant is a *Poaceae* plant, *Solanaceae* plant, *Cucurbitaceae* plant, *Fabaceae* plant, *Brassicaceae* plant, *Rosaceae* plant, *Moraceae* plant, *Malvaceae* plant, *Apiaceae* plant, *Liliaceae* plant, *Asteraceae* plant, *Amaranthaceae* plant, *Ericaceae* plant, *Vitaceae* plant, *Rutaceae* plant, *Rubiaceae* plant, *Oleaceae* plant, *Lauraceae* plant, *Anacardiaceae* plant, *Sapindaceae* plant, *or Lamiaceae* plant.

9. A method for inducing pest damage resistance in a plant, comprising applying the following ingredient (A) to the plant:
(A) a compound represented by the following general formula (I): 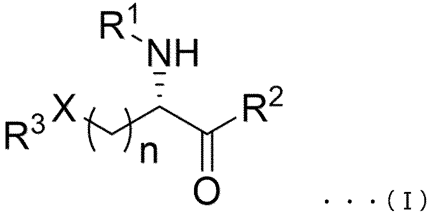 wherein:
n represents an integer of from 1 to 3 (i.e., 1, 2, or 3),
X represents an interatomic bond, oxygen atom (O), nitrogen atom bonded with hydrogen atom (NH), nitrogen atom bonded with methyl group (NMe), sulfur atom (S), or 4-phenoxy group,
R¹ represents hydrogen atom (H), amidino group, or an acyl group,
R² represents OR⁴ or NHR⁴,
R³ represents C(=O)-R⁵, sulfo group (SO₃H), phosphate group (PO₃H₂), or SO₂R⁶,
R⁴ represents hydrogen atom (H), or an optionally substituted alkyl group having 1 to 8 carbon atoms,
R⁵ represents an optionally substituted alkyl group having 1 to 8 carbon atoms, an amino acid, a dipeptide, OR⁷, or NR⁸R⁹,
R⁶ represents methyl group, or an optionally substituted phenyl group,
R⁷ represents hydrogen atom (H), or an alkyl group having 1 to 8 carbon atoms, and
R⁸ and R⁹ independently represent hydrogen atom (H), an optionally substituted alkyl group having 1 to 8 carbon atoms, or an optionally substituted phenyl group.

10. A method for preventing pest damage to a plant,
comprising applying the following ingredient (A) to the plant:
(A) a compound represented by the following general formula (I): 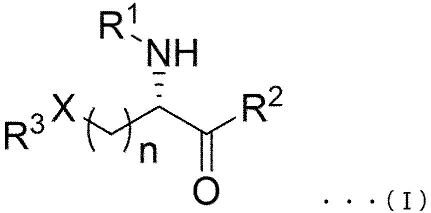 wherein:
n represents an integer of from 1 to 3 (i.e., 1, 2, or 3),
X represents an interatomic bond, oxygen atom (O), nitrogen atom bonded with hydrogen atom (NH), nitrogen atom bonded with methyl group (NMe), sulfur atom (S), or 4-phenoxy group,
R¹ represents hydrogen atom (H), amidino group, or an acyl group,
R² represents OR⁴ or NHR⁴,
R³ represents C(=O)-R⁵, sulfo group (SO₃H), phosphate group (PO₃H₂), or SO₂R⁶,
R⁴ represents hydrogen atom (H), or an optionally substituted alkyl group having 1 to 8 carbon atoms,
R⁵ represents an optionally substituted alkyl group having 1 to 8 carbon atoms, an amino acid, a dipeptide, OR⁷, or NR⁸R⁹,
R⁶ represents methyl group, or an optionally substituted phenyl group,
R⁷ represents hydrogen atom (H), or an alkyl group having 1 to 8 carbon atoms, and
R⁸ and R⁹ independently represent hydrogen atom (H), an optionally substituted alkyl group having 1 to 8 carbon atoms, or an optionally substituted phenyl group.

11. A method for producing a plant body,
comprising cultivating a plant with applying the following ingredient (A) to the plant:
(A) a compound represented by the following general formula (I): 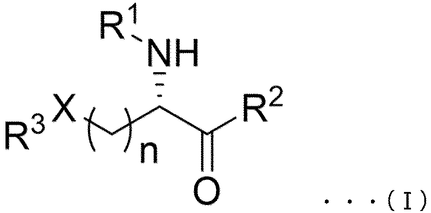 wherein:
n represents an integer of from 1 to 3 (i.e., 1, 2, or 3),
X represents an interatomic bond, oxygen atom (O), nitrogen atom bonded with hydrogen atom (NH), nitrogen atom bonded with methyl group (NMe), sulfur atom (S), or 4-phenoxy group,
R¹ represents hydrogen atom (H), amidino group, or an acyl group,
R² represents OR⁴ or NHR⁴,
R³ represents C(=O)-R⁵, sulfo group (SO₃H), phosphate group (PO₃H₂), or SO₂R⁶,
R⁴ represents hydrogen atom (H), or an optionally substituted alkyl group having 1 to 8 carbon atoms,
R⁵ represents an optionally substituted alkyl group having 1 to 8 carbon atoms, an amino acid, a dipeptide, OR⁷, or NR⁸R⁹,
R⁶ represents methyl group, or an optionally substituted phenyl group,
R⁷ represents hydrogen atom (H), or an alkyl group having 1 to 8 carbon atoms, and
R⁸ and R⁹ independently represent hydrogen atom (H), an optionally substituted alkyl group having 1 to 8 carbon atoms, or an optionally substituted phenyl group.

12. The method according to any one of claims 9 to 11, wherein n is 2.

13. The method according to any one of claims 9 to 11, wherein X is an interatomic bond.

14. The method according to any one of claims 9 to 11, wherein the method satisfies any of the following conditions:
(1) n is 2, X is an interatomic bond, R² is hydroxyl group, and R³ is C(=O)-R⁵;
(2) n is 2, X is an interatomic bond, R' is hydrogen atom, R² is hydroxyl group, R³ is C(=O)-R⁵, and R⁵ is other than hydroxyl group;
(3) n is 2, X is an interatomic bond, R¹ is other than hydrogen atom, R² is hydroxyl group, R³ is C(=O)-R⁵, and R⁵ is hydroxyl group; and
(4) n is 2, X is an interatomic bond, R' is hydrogen atom, R² is other than hydroxyl group, and R³ is C(=O)-OH.

15. The method according to any one of claims 9 to 11, wherein the ingredient (A) is selected from the compounds represented by the following formulas of Compound 1-58 and Compound 67-75:

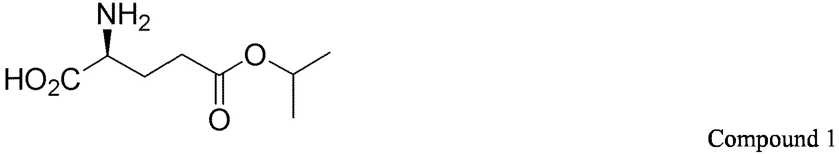

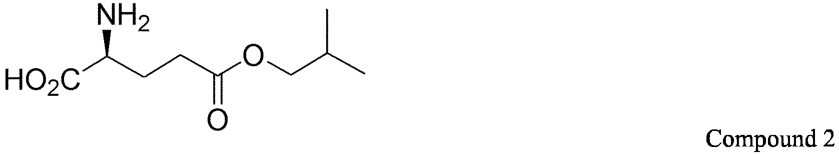

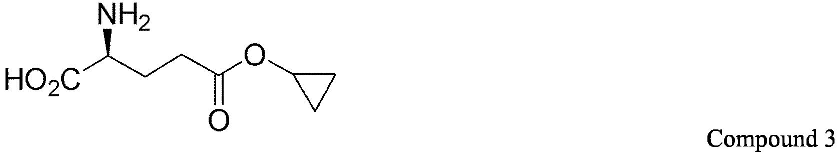

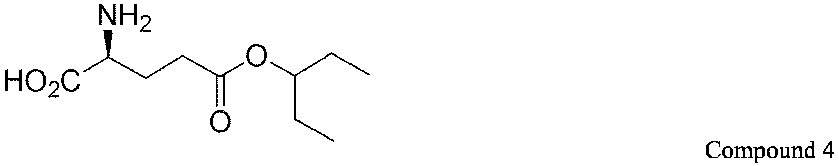

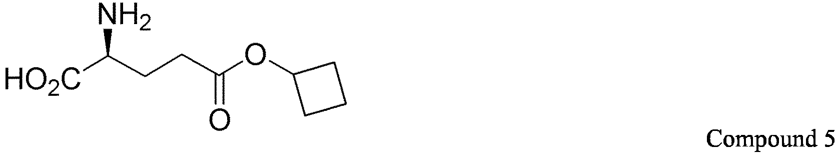

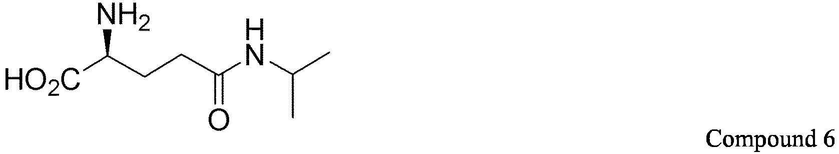

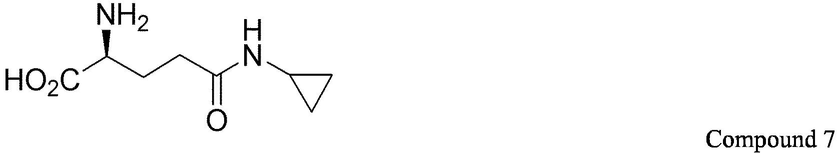

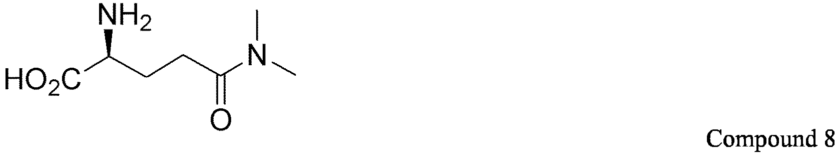

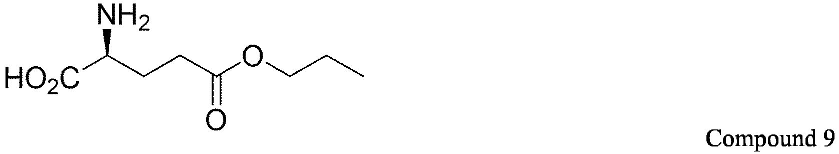

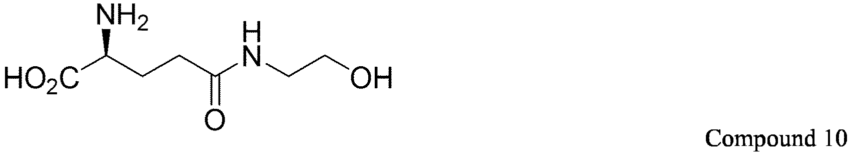

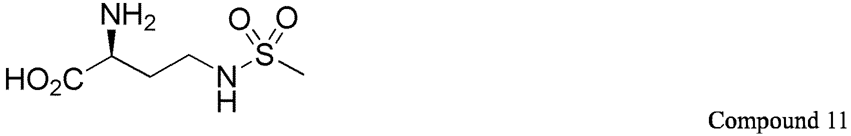

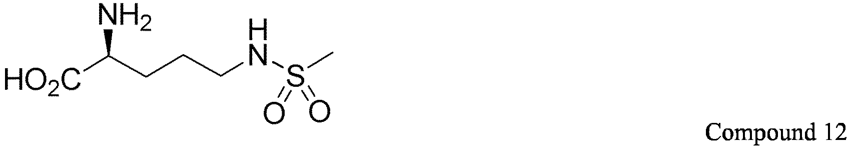

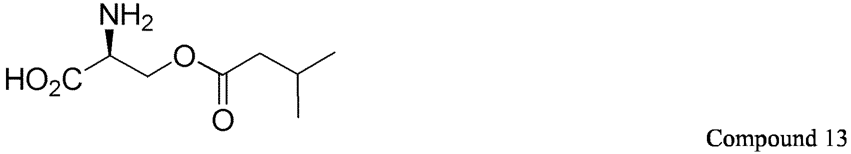

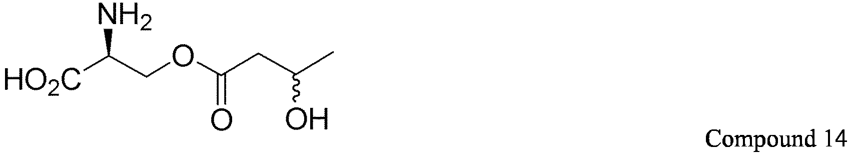

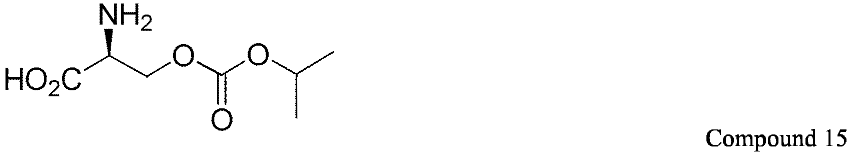

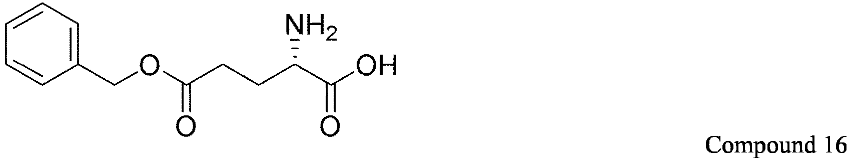

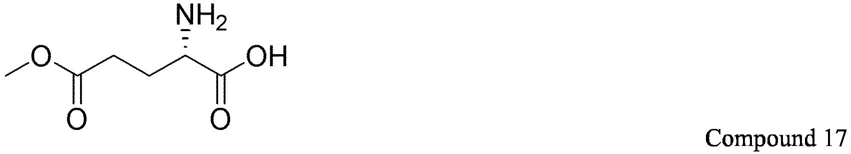

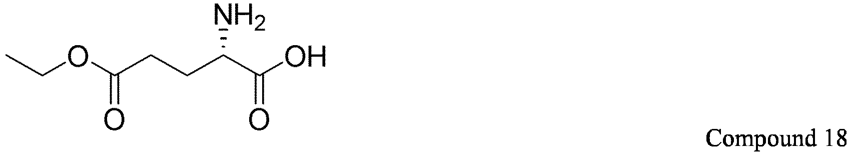

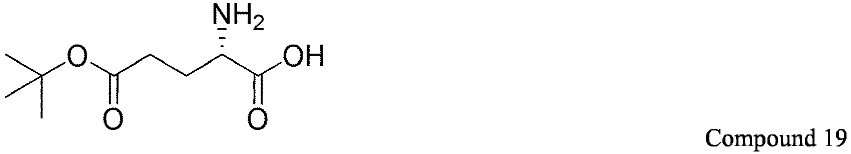

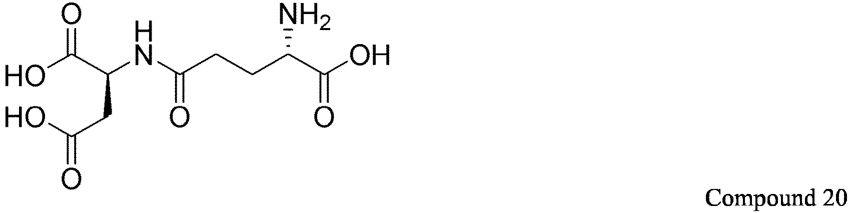

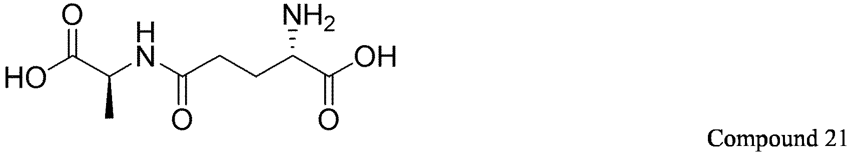

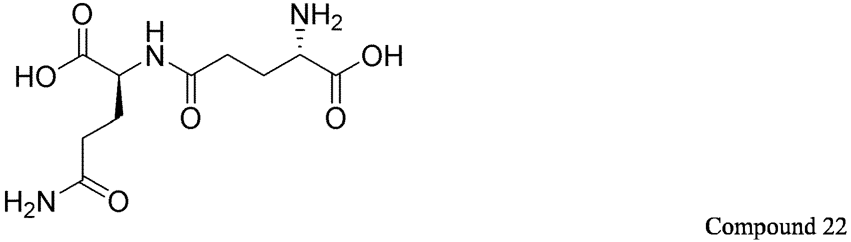

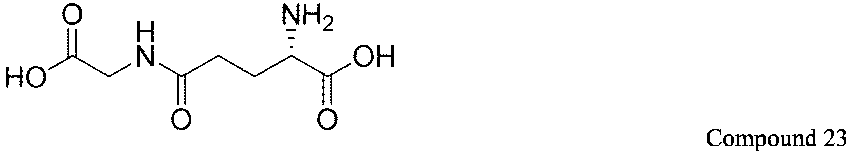

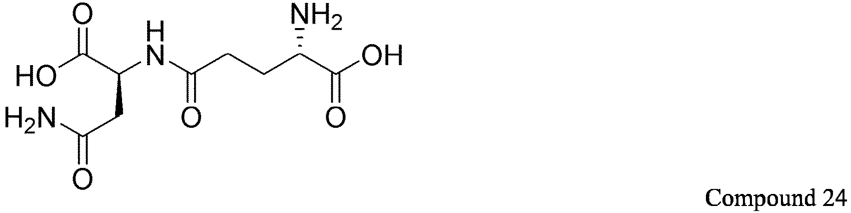

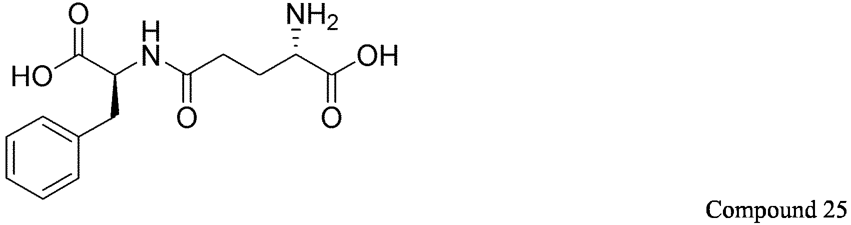

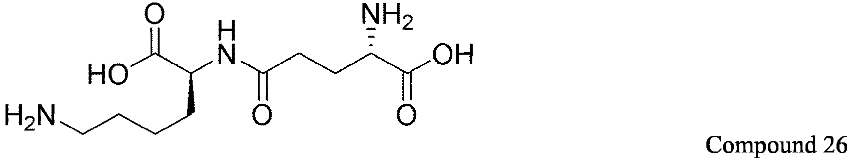

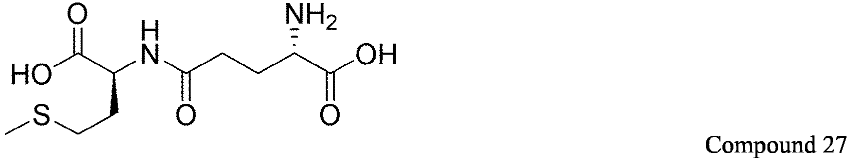

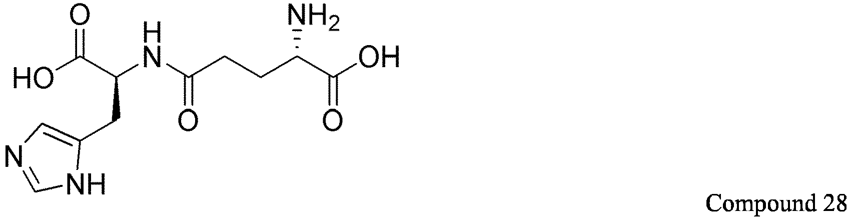

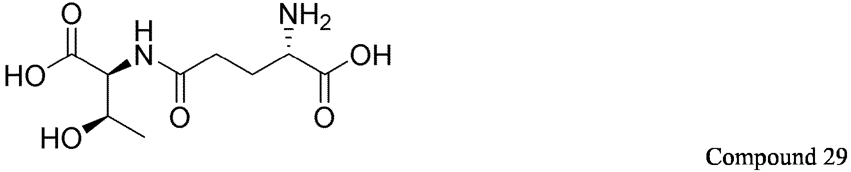

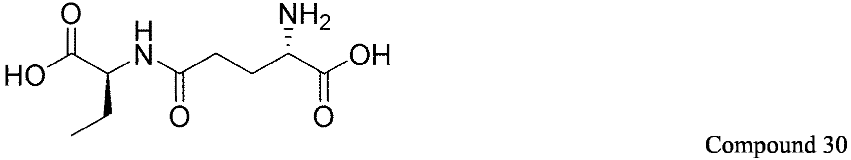

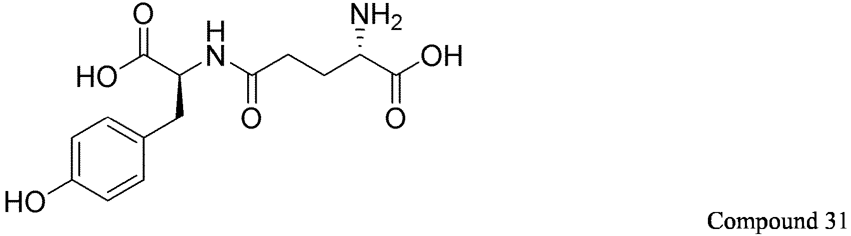

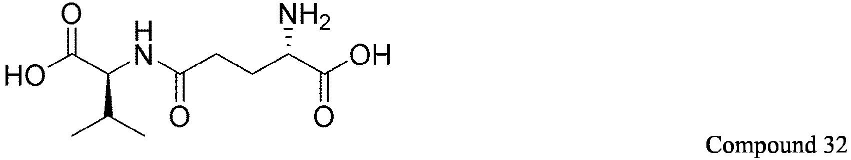

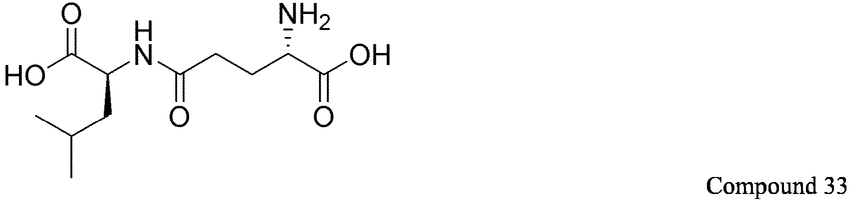

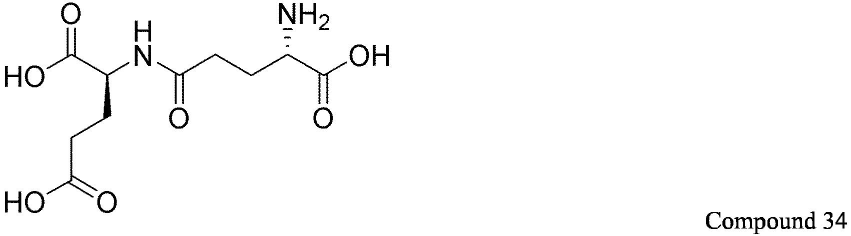

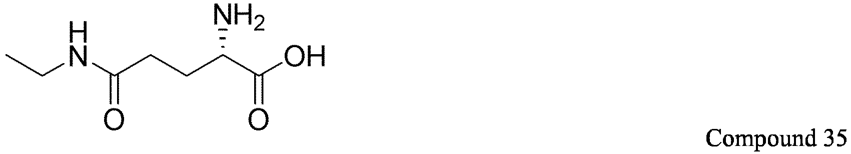

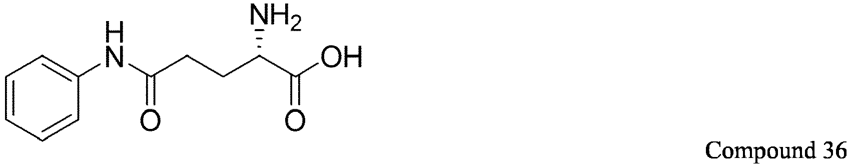

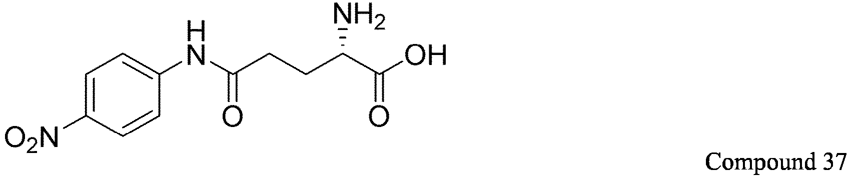

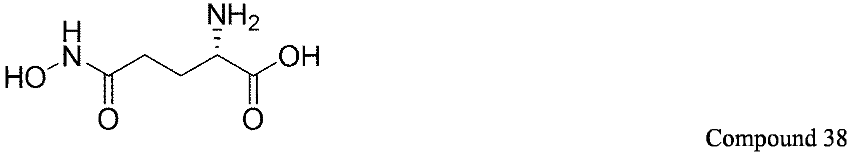

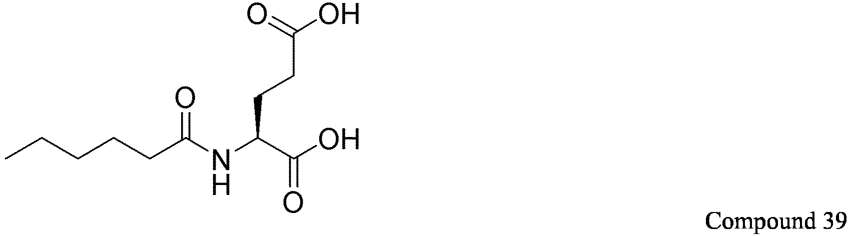

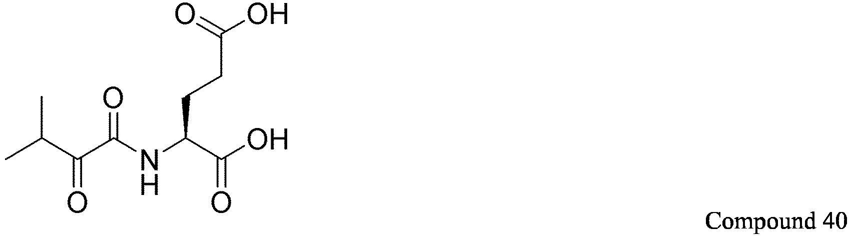

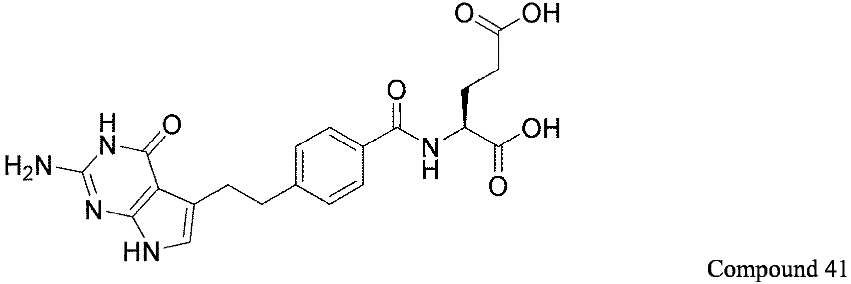

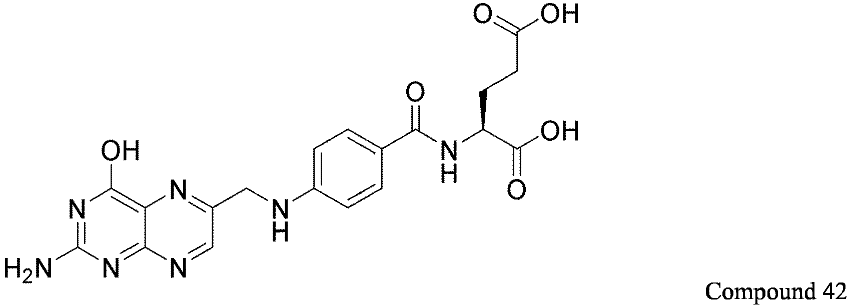

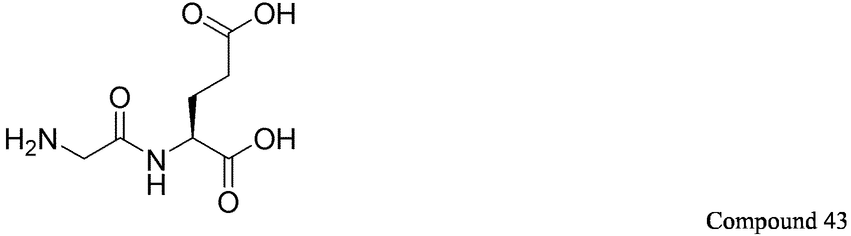

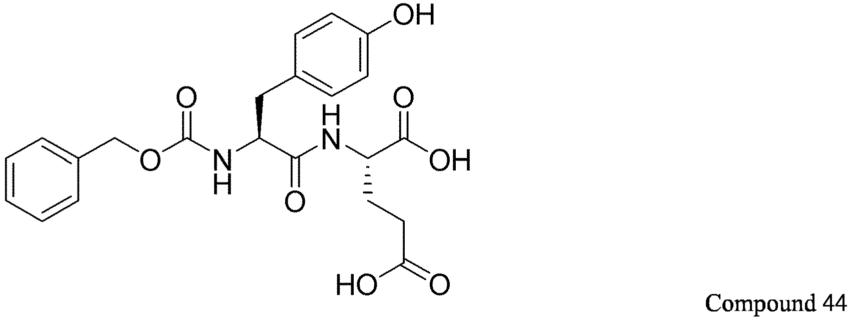

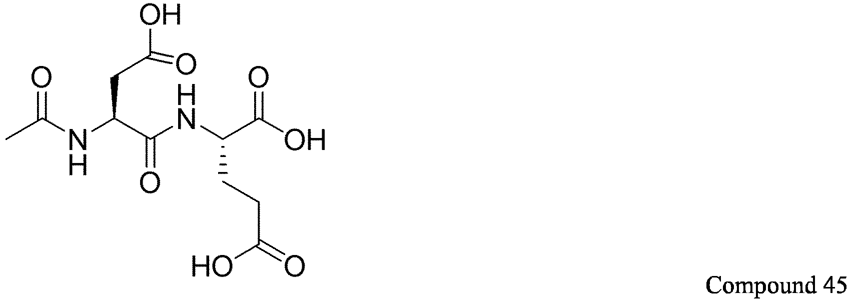

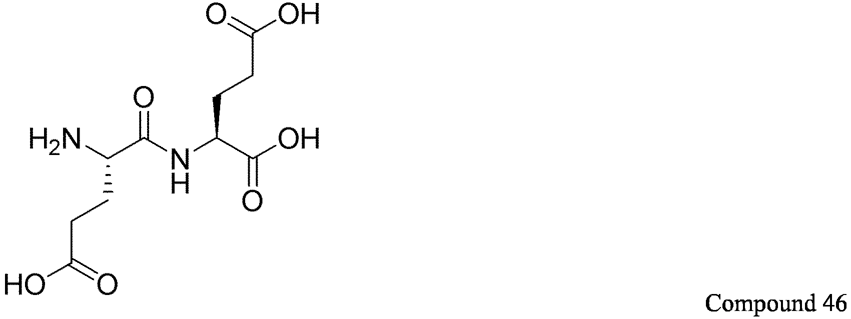

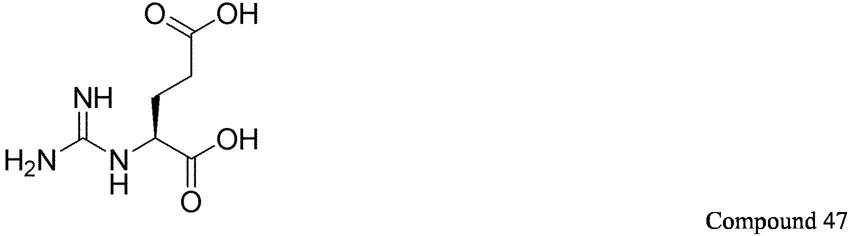

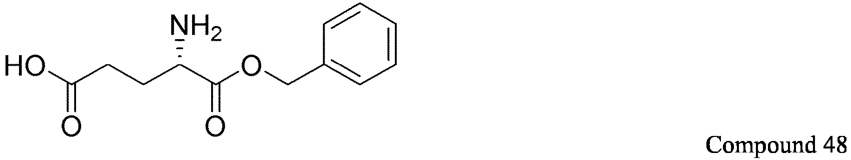

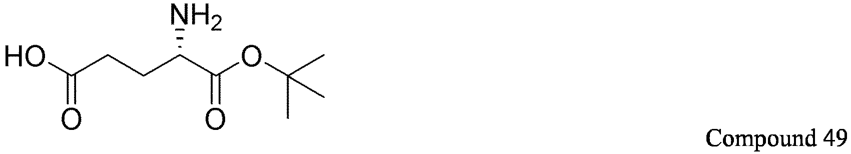

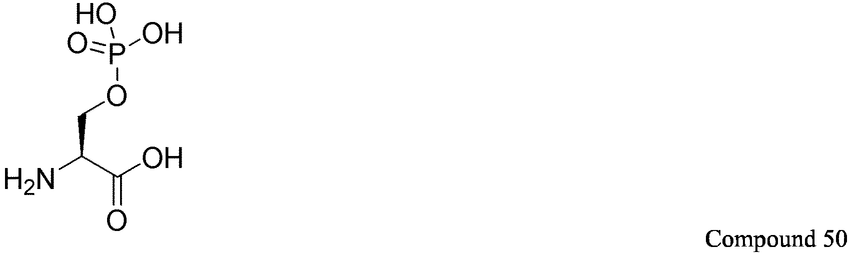

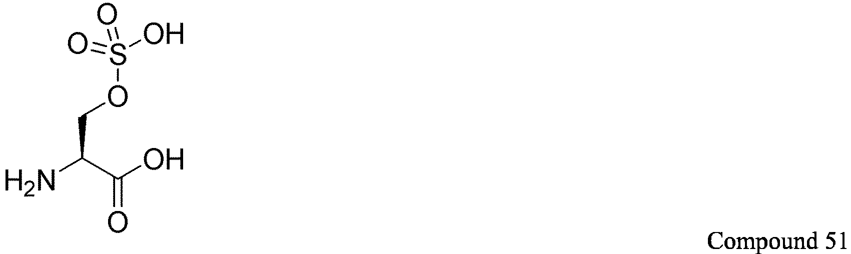

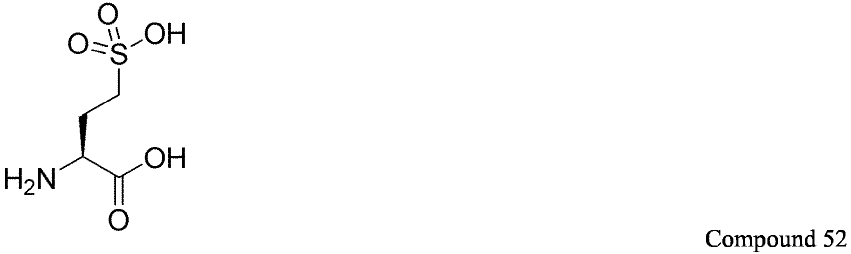

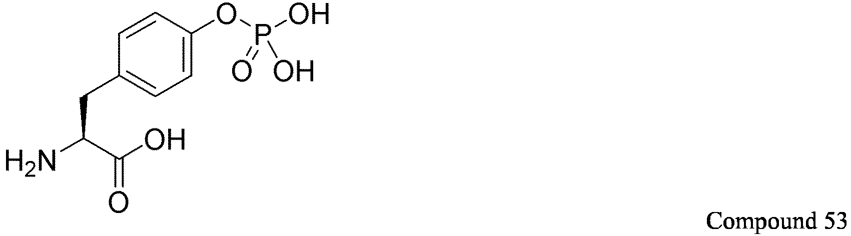

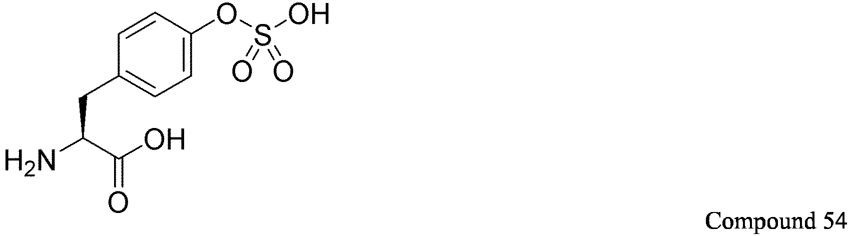

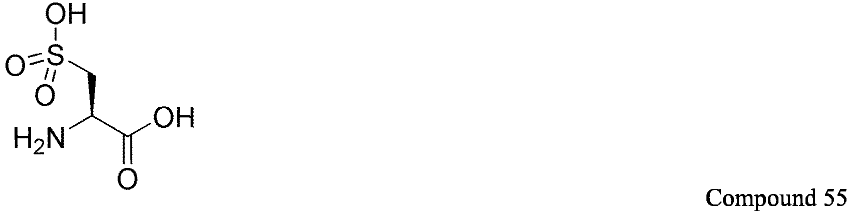

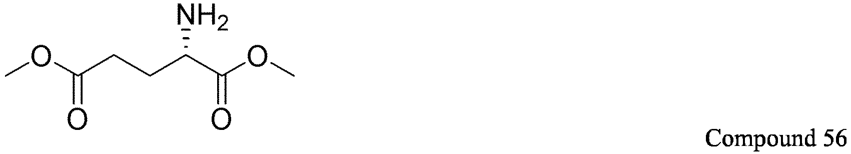

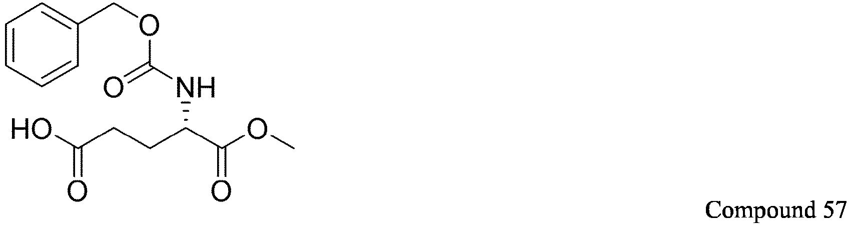

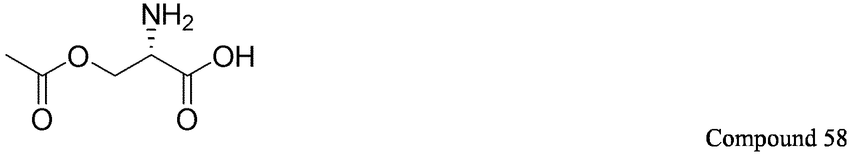

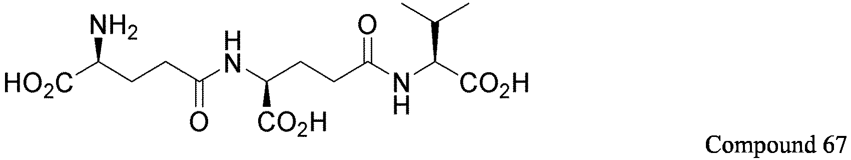

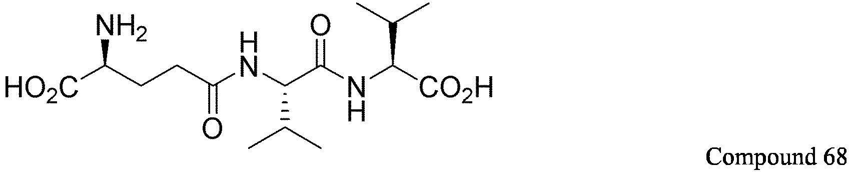

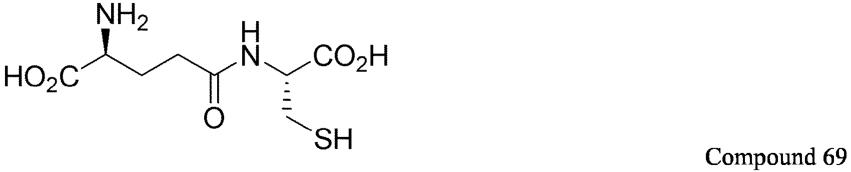

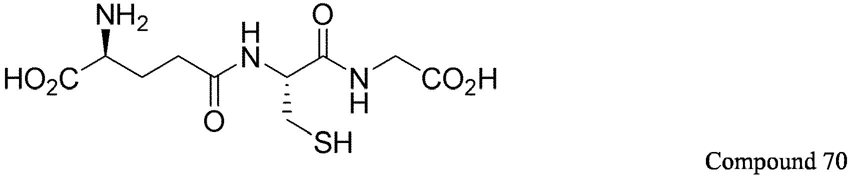

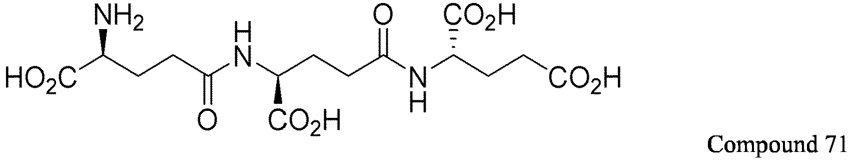

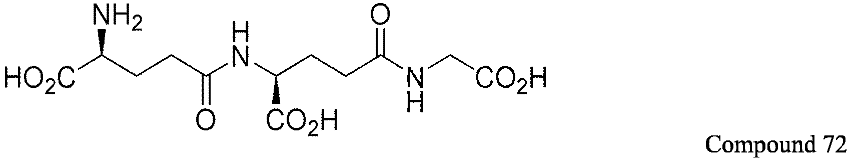

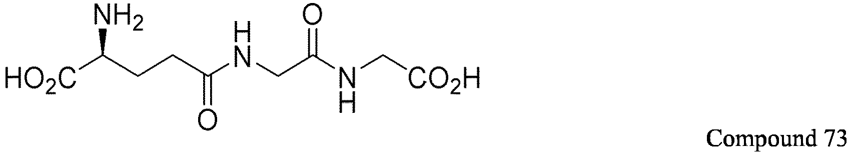

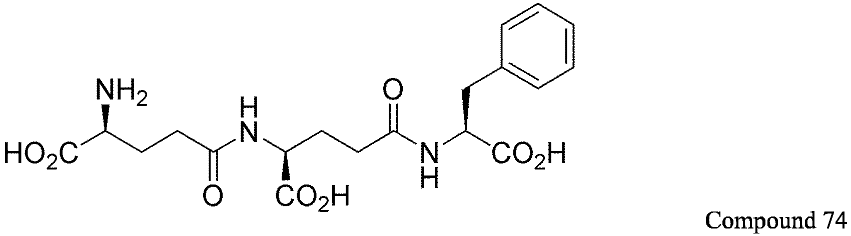

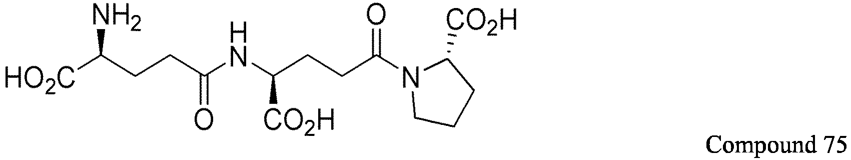

16. The method according to any one of claims 9 to 11, wherein the ingredient (A) is used in the form of a liquid containing the ingredient (A) at a concentration of 0.1 to 1,000 mM.

17. The method according to any one of claims 9 to 11, wherein the pest damage is pest damage caused by an organism classified in the order *Lepidoptera, Hemiptera, Coleoptera, Diptera, Orthoptera, Thysanoptera, Tylenchida, Collembola, Acarina,* or *Stylommatophora.*

18. The method according to any one of claims 9 to 11, wherein the pest damage is pest damage caused by an organism classified in the family *Plutellidae, Noctuidae, Pyralidae, Tortricidae, Lyonetiidae, Carposinidae, Gelechiidae, Crambidae, Arctiidae (Erebidae), Lymantriidae (Erebidae), Cicadellidae, Delphacidae, Psyllidae, Aphididae, Aleyrodidae, Coccoidea, Miridae, Tingidae, Pentatomoidea, Lygaeidae, Scarabaeidae, Elateridae, Coccinellidae, Cerambycidae, Chrysomelidae, Curculionidae, Muscidae, Calliphoridae, Sarcophagidae, Anthomyiidae, Tephritidae, Opomyzoidea* (superfamily), *Chloropoidea* (superfamily), *Acrididae, Catantopidae, Pyrgomorphidae, Thripidae, Aeolothripidae, Merothripidae, Aphelenchoididae, Neotylenchidae, Onychiuridae, Isotomidae, Tetranychidae, Dermanyssidae, Acaridae, Sarcoptidae, Philomycidae,* or *Bradybaenidae.*

19. The method according to any one of claims 9 to 11, wherein the plant is a *Poaceae* plant, *Solanaceae* plant, *Cucurbitaceae* plant, *Fabaceae* plant, *Brassicaceae* plant, *Rosaceae* plant, *Moraceae* plant, *Malvaceae* plant, *Apiaceae* plant, *Liliaceae* plant, *Asteraceae* plant, *Amaranthaceae* plant, *Ericaceae* plant, *Vitaceae* plant, *Rutaceae* plant, *Rubiaceae* plant, *Oleaceae* plant, *Lauraceae* plant, *Anacardiaceae* plant, *Sapindaceae* plant, *or Lamiaceae* plant.
